# EUROPEAN PATENT SPECIFICATION

(11) **EP 1 501 465 B1**
(45) Date of publication and mention of the grant of the patent: **21.10.2009**
(21) Application number: 03728653.1
(22) Date of filing: 02.05.2003
(51) Int. Cl.: A61K 6/00, C12N 9/24

(54) **VECTORS HAVING BOTH ISOFORMS OF BETA-HEXOSAMINIDASE**
VEKTOREN MIT BEIDEN ISOFORMEN VON BETA-HEXOSAMINIDASE
VECTEURS POSSEDANT LES DEUX ISOFORMES DE BETA-HEXOSAMINIDASE

(30) Priority: 02.05.2002 US 377503 P
(43) Date of publication of application: 02.02.2005
(73) Proprietor: UNIVERSITY OF ROCHESTER, Rochester, NY 14642 (US)
(72) Inventor: KYRKANIDES, Stephanos, Penfield, NY 14526 (US)
(74) Representative: Helbing, Jörg
(86) International application number: PCT/US2003/013672
(87) International publication number: WO 2003/092612

(56) References cited:
- WO-A-02/44355
- US-A- 5 217 865
- TEIXEIRA C A ET AL: "Retrovirus-mediated transfer and expression of beta-hexosaminidase alpha-chain cDNA in human fibroblasts from GM2-gangliosidosis B1 variant" HUMAN GENE THERAPY, vol. 12, no. 14, 20 September 2001 (2001-09-20), pages 1771-1783, XP002367837 ISSN: 1043-0342
- WILDNER ET AL: "GENERATION OF A CONDITIONALLY neo(r)-CONTAINING RETROVIRAL PRODUCER CELL LINE: EFFECTS OF neo(r) ON RETROVIRAL TITER AND TRANSGENE EXPRESSION" GENE THERAPY, MACMILLAN PRESS LTD., BASINGSTOKE, GB, vol. 5, no. 5, 1998, pages 684-691, XP002100126 ISSN: 0969-7128
- GUIDOTTI J E ET AL: "Adenoviral gene therapy of the Tay-Sachs disease in hexosaminidase A-deficient knock-out mice" HUMAN MOLECULAR GENETICS, OXFORD UNIVERSITY PRESS, SURREY, GB, vol. 8, no. 5, 1999, pages 831-838, XP002975923 ISSN: 0964-6906
- GUIDOTTI J -E ET AL: "Retrovirus-mediated enzymatic correction of Tay-Sachs defect in transduced and non-transduced cells" HUMAN MOLECULAR GENETICS, vol. 7, no. 5, May 1998 (1998-05), pages 831-838, XP002367838 ISSN: 0964-6906
- LACORAZZA H D ET AL: "Expression of human beta-hexosaminidase alpha-subunit gene (the gene defect of Tay-Sachs disease) in mouse brains upon engraftment of transduced progenitor cells" NATURE MEDICINE, NATURE PUBLISHING GROUP, NEW YORK, NY, US, vol. 2, no. 4, April 1996 (1996-04), pages 424-429, XP009030194 ISSN: 1078-8956
- POESCHLA E M ET AL: "Efficient transduction of non-dividing human cells by feline immunodeficiency virus lentiviral vectors" NATURE MEDICINE, NATURE PUBLISHING GROUP, NEW YORK, NY, US, vol. 4, no. 3, March 1998 (1998-03), pages 354-357, XP002107905 ISSN: 1078-8956
- HAVENGA M J E ET AL: "Second gene expression in bicistronic constructs using short synthetic intercistrons and viral IRES sequences" GENE, ELSEVIER, AMSTERDAM, NL, vol. 222, no. 2, November 1998 (1998-11), pages 319-327, XP004150061 ISSN: 0378-1119
- KYRKANIDES S ET AL: "beta-hexosaminidase lentiviral vectors: transfer into the CNS via systemic administration" MOLECULAR BRAIN RESEARCH, ELSEVIER SCIENCE BV, AMSTERDAM, NL, vol. 133, no. 2, 18 February 2005 (2005-02-18), pages 286-298, XP004750329 ISSN: 0169-328X
- ARFI ET AL: "Bicistronic lentiviral vector corrects beta-hexosaminidase deficiency in transduced and cross-corrected human Sandhoff fibroblasts" NEUROBIOLOGY OF DISEASE, BLACKWELL SCIENCE, OXFORD, GB, vol. 20, no. 2, November 2005 (2005-11), pages 583-593, XP005118692 ISSN: 0969-9961
- KYRKANIDES STEPHANOS ET AL: "Transcriptional and posttranslational regulation of Cre recombinase by RU486 as the basis for an enhanced inducible expression system." MOLECULAR THERAPY : THE JOURNAL OF THE AMERICAN SOCIETY OF GENE THERAPY. NOV 2003, vol. 8, no. 5, November 2003 (2003-11), pages 790-795, XP002367839 ISSN: 1525-0016
- BOURGOIN C ET AL: "Widespread distribution of beta-hexosaminidase activity in the brain of a Sandhoff mouse model after coinjection of adenoviral vector and mannitol." GENE THERAPY, vol. 10, no. 21, October 2003 (2003-10), pages 1841-1849, XP002367840 ISSN: 0969-7128
- MYEROWITZ R. ET AL: 'Human beta-hexosaminidase alpha chain: coding sequence and homology with the beta chain' PNAS vol. 82, 1985, pages 7830 - 7834, XP002980641
- KORNELUK R.G. ET AL: 'Isolation of cDNA clones coding for the alpha-subunit of human beta-hexosaminidase. Extensive homology between the alpha- and beta-subunits and studies on tay-sachs disease' J. BIOL. CHEM. vol. 261, no. 18, 1986, pages 8407 - 8413, XP002980642
- NEOTE K. ET AL: 'Characterization of the human HEXB gene encoding lysosomal beta-hexosaminidase' GENOMICS vol. 3, no. 4, November 1988, pages 279 - 286, XP002977726
- DATABASE GENBANK [Online] 24 August 1997 MARTIN D.R. ET AL: 'Mutation analysis of feline GM2 gangliosidosis, variant O', XP002980643 Retrieved from EMBL Database accession no. AF014805
- PROIA R.L.: 'Mutation analysis of feline GM2 gangliosidosis, variant O extensive homology of intron placement in the alpha- and beta-chain genes' PNAS vol. 85, no. 6, 1988, pages 1883 - 1887, XP002110042
- KOLODNY E.H.: 'Molecular genetics of the beta-hexosaminidase isoenzymes: an introduction' ADVANCES IN GENETICS vol. 44, 2001, pages 101 - 126, XP002980682
- DATABASE GENBANK [Online] 13 November 2001 STRAUSBERG R.L. ET AL: 'Generation and initial analysis of more than 15,000 full-length human and mouse cDNA sequences', XP002980644 Database accession no. BC017378
- BEUTLER E.: 'Chapter 9: Subunit structure of hexosaminidase isozymes' ADVANCES IN GENETICS 2001, pages 93 - 100, XP002980683
- PROIA R.L.: 'Chapter 11: cloning the beta-hexosaminidase genes' ADVANCES IN GENETICS vol. 44, 2001, pages 127 - 135, XP002980684
- CAPECCHI M.R. ET AL: 'Targeted gene replacement' SCIENTIFIC AMERICAN vol. 270, no. 3, March 1994, pages 34 - 41, XP002970759

## Description

### I. BACKGROUND OF THE INVENTION

Lysosomal storage disorders are disorders that typically arise from the aberrant or non-existent proteins involved in degradation function within the lysosomes. This causes a decrease in the lysosomal activity, which in turn causes an accumulation of unwanted materials in the cell. These unwanted materials can cause severe cellular toxicity and can impair, for example, neuronal function. These diseases severely impair the quality of life of those who have them, and can even result in death. Two diseases, Tay-Sachs and Sandoffs, are related to the functional impairment of the lysosomal protein β-hexosaminidase. β-hexosaminidase is a hetero or homo dimer made up of two subunits arising from two separate genes, HexA and HexB. Mutation of the HexA gene, causing functional problems with the HEX-α (HexA/HexB) polypeptide, results in Tay-Sachs disease, whereas mutation of the HexB gene, causing functional problems in the HEX-α (HexA/HexB) and HEX-β (HexB/HexB) polypeptides, results in Sandoff's disease. Clinically, it is not uncommon for patients to display only mild features at infancy, but due to increasing lysosomal storage over time, progress to severe forms of the disease by adolescence.

Current treatments include bone marrow transplantation, which has been employed in some cases of individuals during childhood but with modest outcomes. A significant problem with the bone marrow transplantation approach is that it may address the lack of specific metabolic activity in peripheral tissues, but due to the presence of the blood-brain-barrier it fails to avert disease progression in the central nervous system. Hence patients often continue to clinically deteriorate due to central nervous system involvement with subsequent development of neurodegeneration, blindness, mental retardation, paralysis and dementia.

Enzyme replacement strategies targeting peripheral and central nervous system tissues utilizing gene therapy is a logical approach for treating inherited metabolic disorders. In a study by Akli *et al*. (1996), the authors report successful restoration of β-hexosaminidase in fibroblasts derived from patients with *HexA* deficiency via adenoviral-mediated gene transfer *in vitro*. Likewise a *HexA* transgene and a *HexB* transgene was successfully introduced into neural progenitor cells utilizing retroviral vectors (Lacorazza et al., "Expression of human beta-hexosaminidase alpha-subunit gene (the gene defect of Tay-Sachs disease) in mouse brains upon engraftment of transduced progenitor cells" Nat Med 2(4):424-9 (1996 Apr).
WO 02/44355 discloses a protozoan expression system for preparing lysosomal storage disease enzymes.
C.A. Teixeira et al., Human Gene Ther. 12:1771-1783 (2001) discloses the retroviros-medicated transfer and expression of β-hexoaminidase α-chain cDNA in human firbroblasters.
O. Wildner et al., Gene Ther. 5:684-691 (1998) discloses the use of a conditional retroviral vector using the Cre system and an IRES for bicistronic expression from retroviral vectors.
J.E. Guidotti et al., Human Mol. Gen. 8(5):831-838 (1999) discloses adenoviral vectors comprising sequences coding for HEX-α and vectors coding for HEX-β.
Co-administration of both vectors to HEX A-/-mice results in enzymatic correction of β-hexoaminidase.
M.J.E. Havevenga et al., Gene 222:319-327 (1998) discloses that the seond gene expression in bicistronic constructs can be controlled by short synthetic intercistrons and viral IRES sequence.

Disclosed herein are vectors which solve the problems associated with enzyme replacement therapies directed to β-hexoaminidase deficiencies.

### II. SUMMARY OF THE INVENTION

In accordance with the purposes of this invention, as embodied and broadly described herein, this invention, in one aspect, relates to a bicistronic lentiviral vector comprising a nucleic acid sequence having at least 70% identity to the sequence set forth in SEQ ID NO:2 and encoding the subunit α of the β-hexosaminidase (HEX-α), a nucleic acid sequence having at least 70% identity to the sequence set forth in SEQ ID NO:4 and encoding the subunit B of the β-hexosaminidase (HEX-β), a promoter, and an integrated ribosomal entry site (IRES), wherein the sequence encoding the HEX-β is orientated 5' to the IRES sequence and the IRES sequence is located 5' to the sequence encoding HEX-α, and wherein said bicistronic lentiviral vector produces a functional HEX-α/HEX-β heterodimer. Said vector is suitable for perinatal gene delivery, including delivery of HEX-α and HEX-β, and can be used for preparing a medicament for the treatment of inherited lysosomal disorders such as Tay-Sachs and Sandoffs disease.

Additional advantages of.the invention will be set forth in part in the description which follows.

### III. BRIEF DESCRIPTION OF THE DRAWINGS

The accompanying drawings, which are incorporated in and constitute a part of this specification, illustrate several embodiments of the invention and together with the description, serve to explain the principles of the invention.

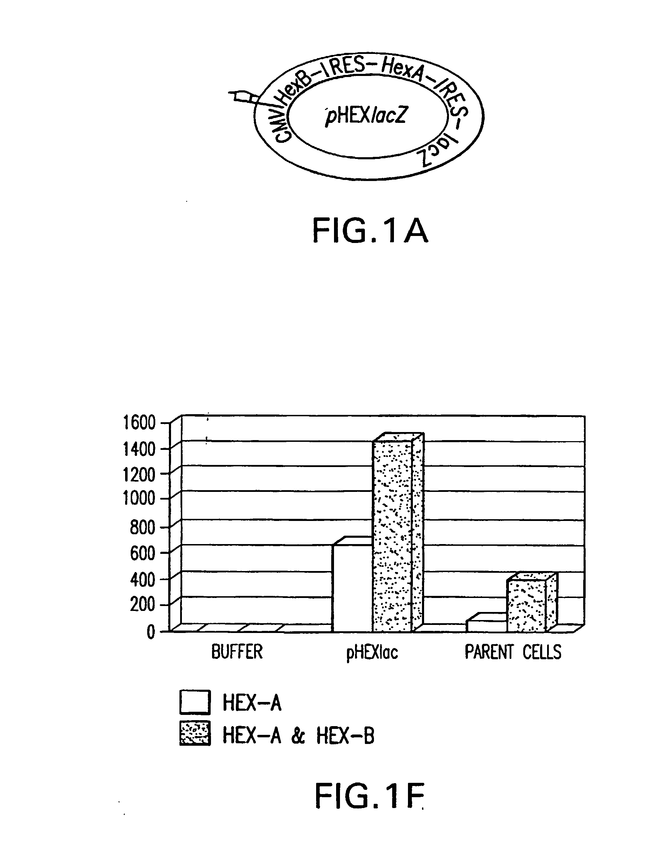

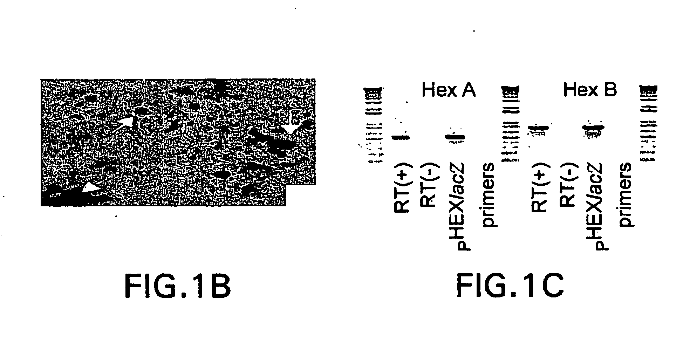

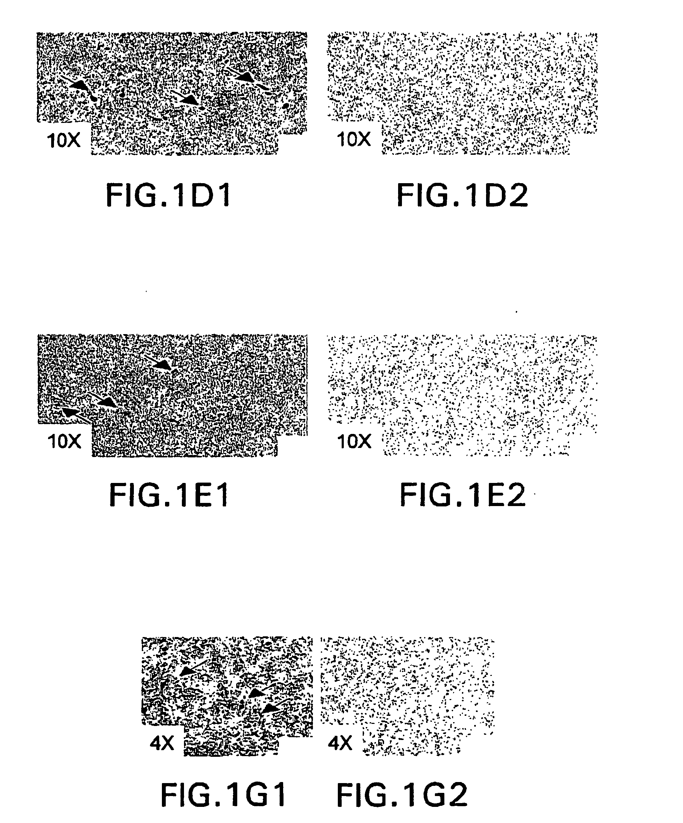

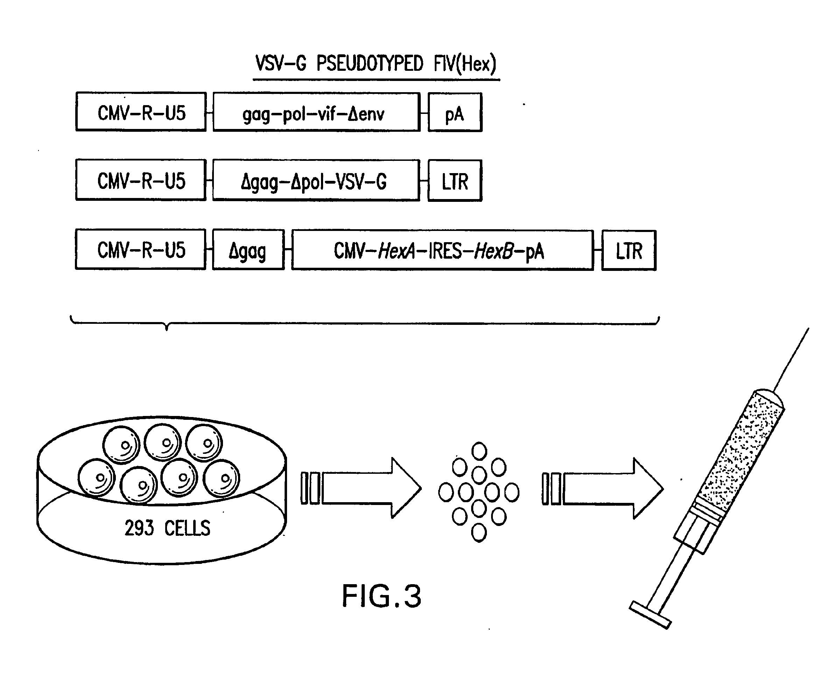

Figure 1 shows that HEX*lacZ* encodes for both isoforms of human β-hexosaminidase, HexA & HexB. Figure 1(A) shows *p*HEX*lacZ* vector. BHK^{HexlacZ} are developed by stable *HexlacZ* transduction. Figure 3(B) shows cells stain positively by X-gal histochemistry. Figure 3(C) shows HexA & HexB mRNA is detected by RT-PCR in total RNA extracts. Figure 3(D₁) shows human HEXA & figure 3(E₁) shows human HEXB proteins are detected in BHK^{HexlacZ} by imunocytochemistry. Figure 3(F₁) shows HEXA & HEXA+HEXB activity is measured by 4MUGS & 4MUG fluorometry, respectively. Figure (G) β-hexosaminidase detection by Fast Garnet histochemistry. (D₂,E_{2,} G₂ are controls for D₁,E₁,G₁, respectively).

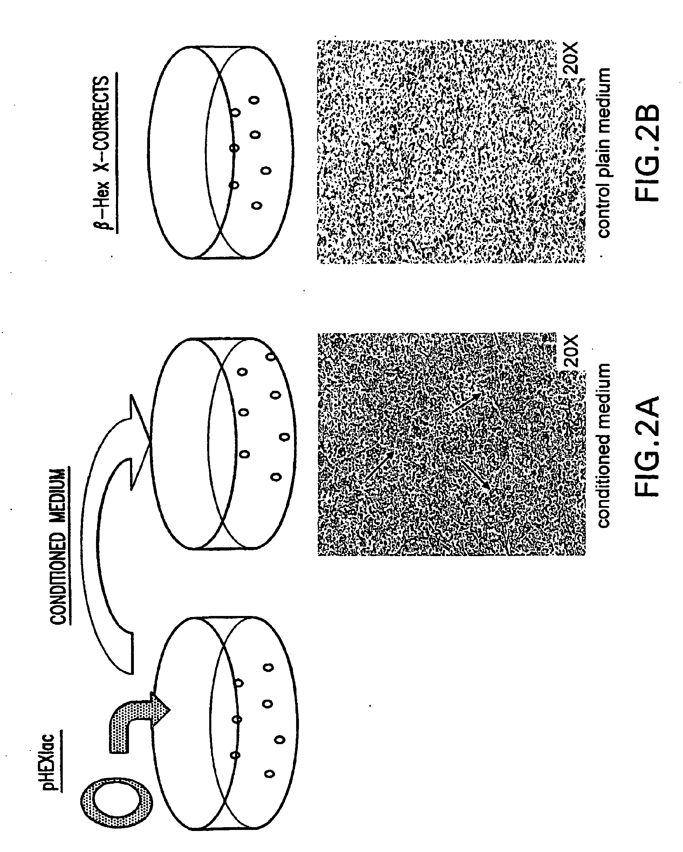

Figure 2 shows that the β-Hex therapeutic gene cross-corrects. An important property of the β-Hex transgene is the products hHEXA & hHEXB have the ability to cross-correct, specifically, to be released extracellularly and then to be absorbed via paracrine pathways by other cells whereby they contribute to β-hexosaminidase activity. For this purpose, BHK^{HexlacZ} cells were cultured and the supernatant was collected (conditioned medium), filtered (.45mm) and applied on normal mouse kidney fibroblasts in culture. Forty-eight hours later, the cells were washed thoroughly with phosphate buffered saline, and briefly treated with a trypsin solution to remove extracellular proteins from the cell surfaces. Following trypsin inactivation with Tris/EDTA buffer, the cells were fixed with 4% paraformaldehyde solution and processed by Fast Garnet histochemistry for β-hexosaminidase activity. Fast Garnet histochemistry of murine fibroblasts exposed to (A) conditioned medium collected from BHK^{HexlacZ} cells compared to cells exposed to medium from normal parent BHK-21 cells (B). These results demonstrate that hHEXA & hHEXB, products of the β-Hex transgene, are released into the extracellular medium and can be absorbed by other cells via paracrine pathways resulting in induction of the cellular β-hexosaminidase.

Figure 3 shows a representation of a lentiviral system containing the HexA and HexB genes. The 3-vector FIV(Hex) system The FIV(Hex) lentiviral system is comprised of 3 vectors: Packaging vector providing the packaging instructions in trans,-VSV-G envelop vector providing the envelop instructions in trans, - FIV(Hex) vector containing the therapeutic bicistronic gene.

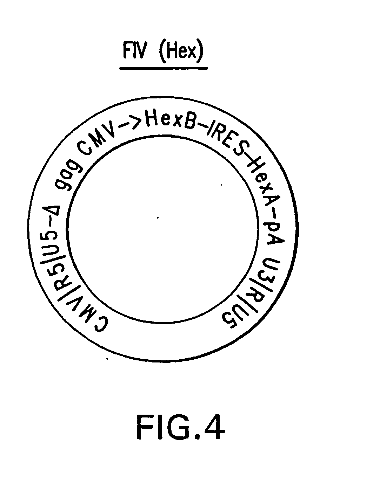

Figure 4 shows a representation of a Fiv(Hex) vector. Backbone FIV vector constructed by Proeschla et al. (1998)

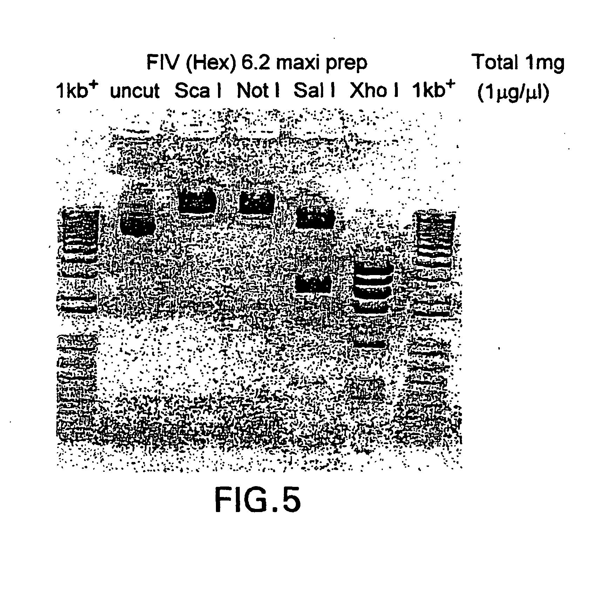

Figure 5 shows restriction fragment pattern of Feline immunodeficiency viral vector comprising a β-Hex construct. A maxi prep of FIV(Hex) clone 6.2 in 500 TB with 3X solution run through 2 columns. Yield of DNA was 1.095 mg. Final concentration is 1microg/microl. Restriction enzyme digest with ScaI, notl, Sall, and Xhol. The bands are as expected.

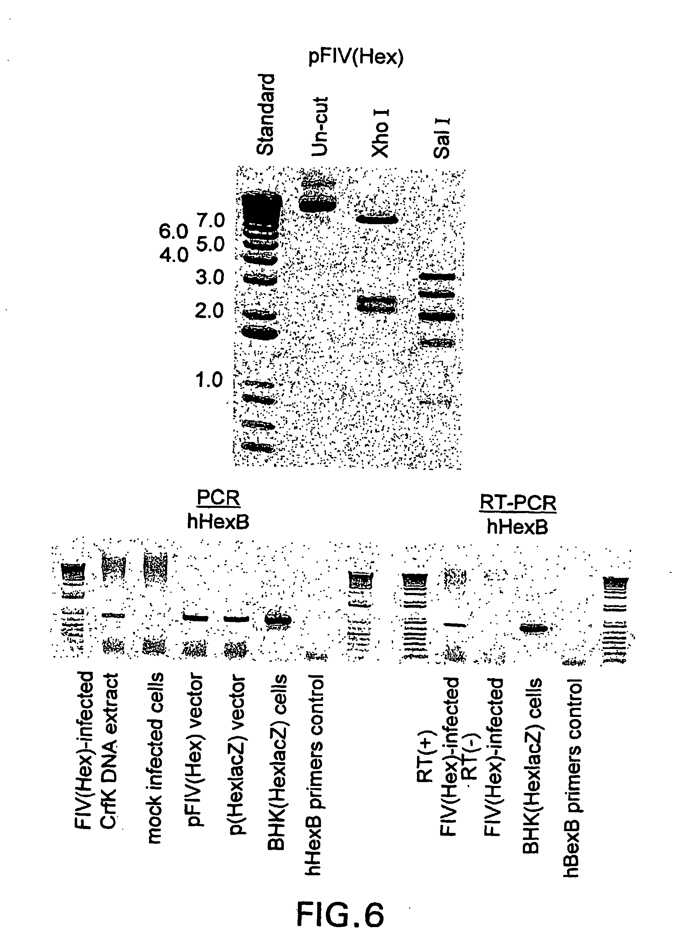

Figure 6 shows fibroblast infection by FIV(Hex) in vitro.

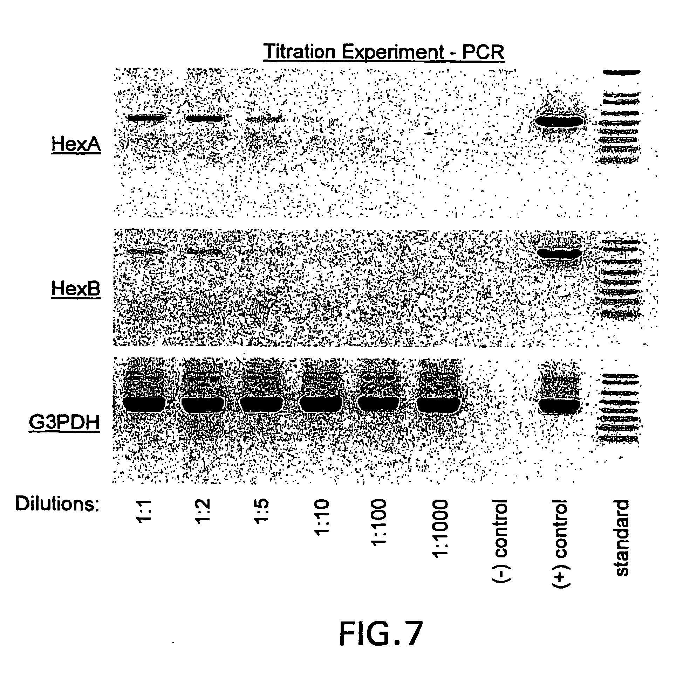

Figure 7 shows an FIV(Hex) titration experiment.

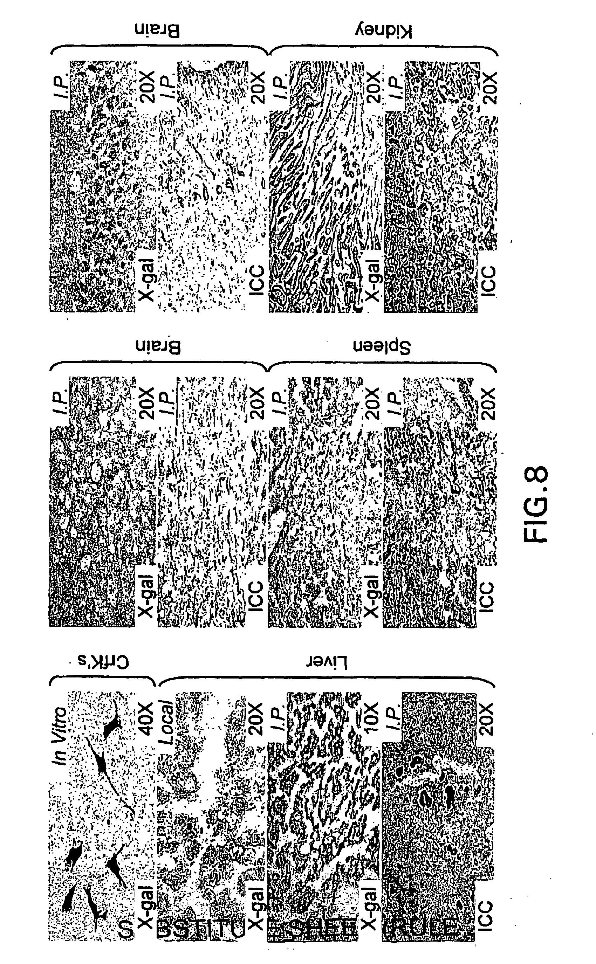

Figure 8 shows FIV(lacZ) administration to adult mice. FIV(lacZ) infection of murine fibroblasts (CrfK's) *in vitro*, as well as of liver cells following direct transdermal intra-hepatic injection. Liver, brain and spleen sections stained for β-galactosidase following intraperitoneal injection to 3 month old mice. lacZ expression was detected by X-gal staining (blue stain) and immunocytochemistry (ICC; black stain) on fixed tissue sections harvested 1 month post-treatment.

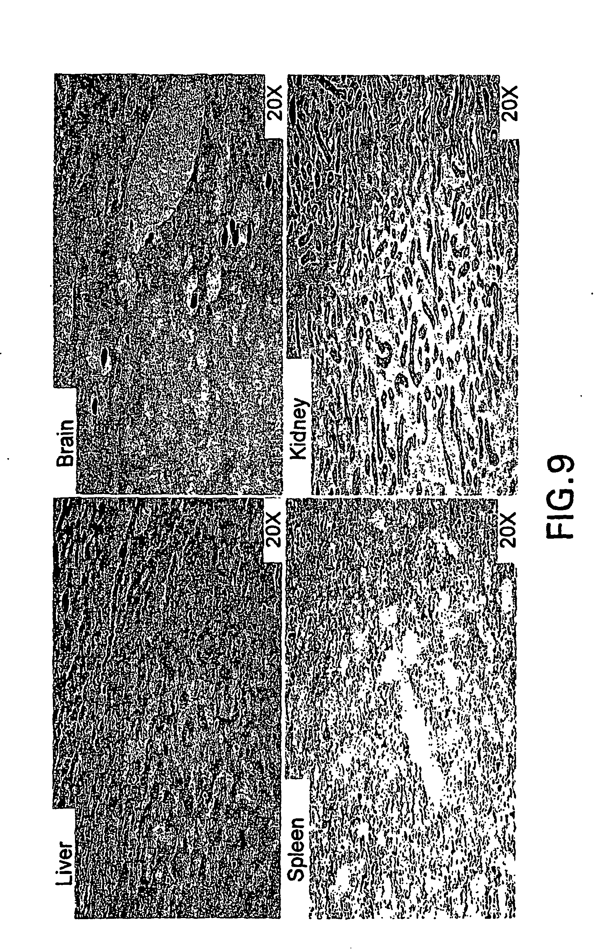

Figure 9 shows FIV(lacZ) administration to P4 mice. Liver, brain, spleen and kidney sections stained for β-galactosidase following intraperitoneal injection to mice of perinatal age (4 days old). lacZ expression was detected by X-gal staining (blue stain) on fixed tissue sections harvested 3 months post-treatment.

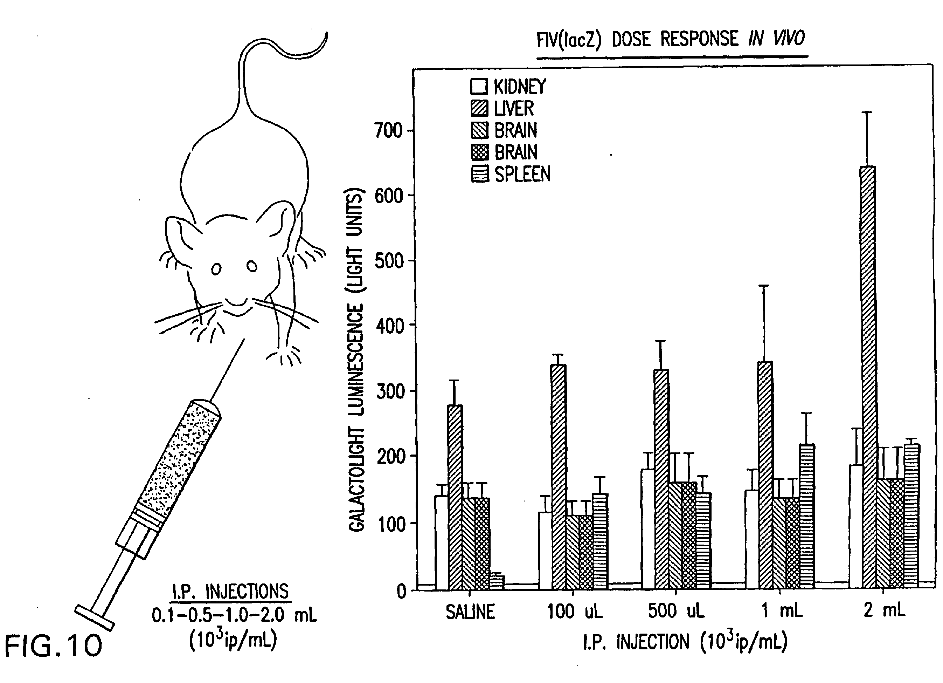

Figure 10 shows dose response of IP injections. Young adult mice (6 weeks old) were injected intra-peritoneally with different doses of FIV(lacZ) {0.1 mL, 0.5 mL, 1.0 mL and 2.0mL of 10³ infectious particles per mL} viral solution. One month following treatment the animals were sacrificed and lacZ reporter gene expression was measured. It was found that increasing doses of FIV result in increasing levels of gene therapy efficacy. In the clinical, human disease arena, this would optimally translate into intravenous administration of 10⁵-10⁶ infectious FIV particles to ensure similar efficacy levels of gene therapy.

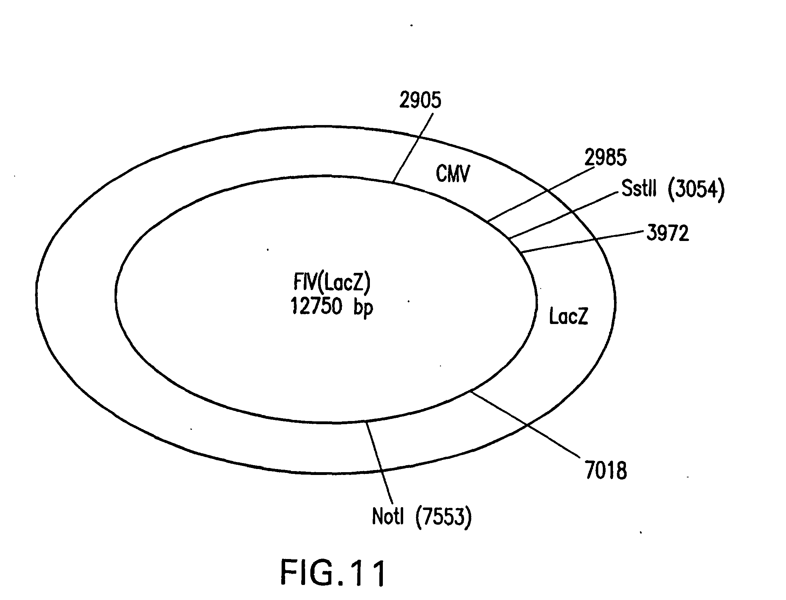

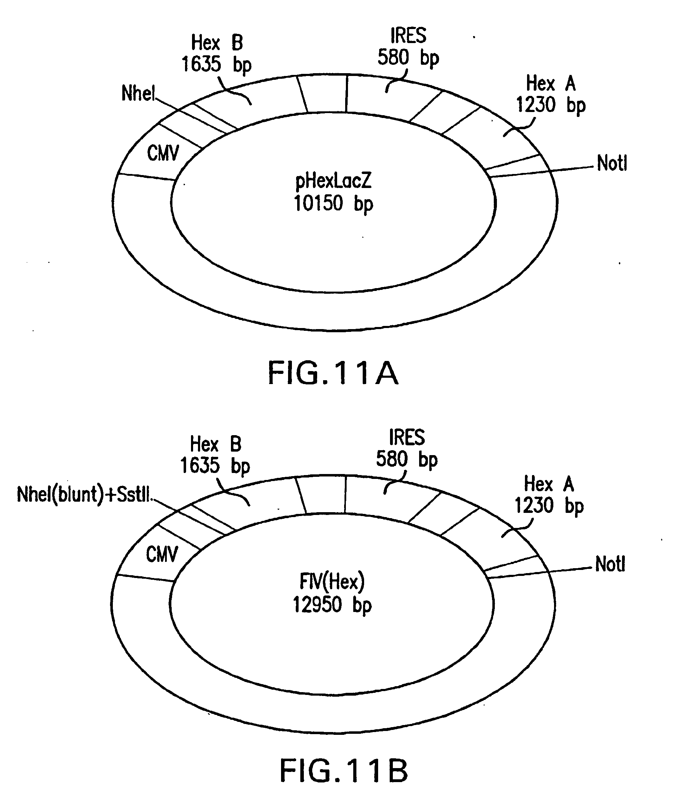

Figure 11 shows diagrams of the vectors used to make the constructs discussed in Examples 1 and 2. FIV(Hex) is constructed by ligating the backbone part of FIV(LacZ), and the fragment of HexB-IRES-HexA from pHexLacZ. FIV(LacZ) is 12750 bp, after cut with SstII and Notl (generate 4500 bp and 8250 bp bands). Purify the 8250 bp band which contains the FIV backbone with CMV promoter. pHexlacZ is a construct of 10150 bp. Cut with NheI and NotI, there are 4700 bp and 5450 bp fragments. The 4700 bp band contains the structure of HexB-IRES-HexA, which doesn't have CMV.

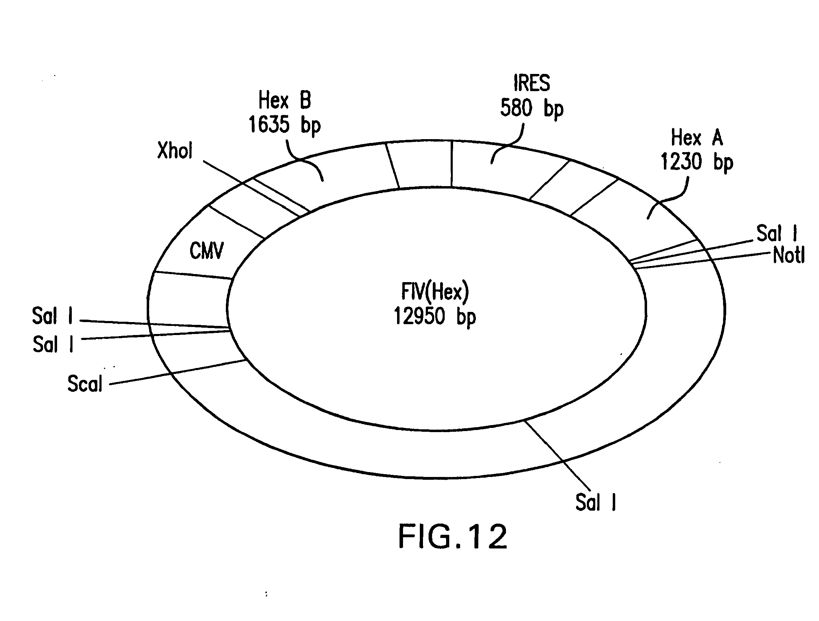

Figure 12 shows how the structure of FIV(Hex) was confirmed. The constructs were digested with different restriction enzymes: (Result see Figure 5). ScaI: cut once in the FIV backbone (generated one band 13 Kb). Notl: the site of ligation, and it is the only site (generated one band 13 Kb). Sal I: one site in HexB-IRFS-HexA and 3 sites in FIV backbone (generated one band t 8.5 Kb, one wide band with 2184 bp and 2400 bp, one band 34 bp which is invisible). Xho I: there is one site in HexB-IRFS-HexA and six sites in the FIV backbone (FIV(LacZ): at 502, 1410, 1453, 7559, 7883 and 9949 bp). These generated 6 bands (908 bp, 43 bp(invisible), 1.7Kb, 324 bp, 2066 bp, 3.3 Kb, and 2.8 Kb).

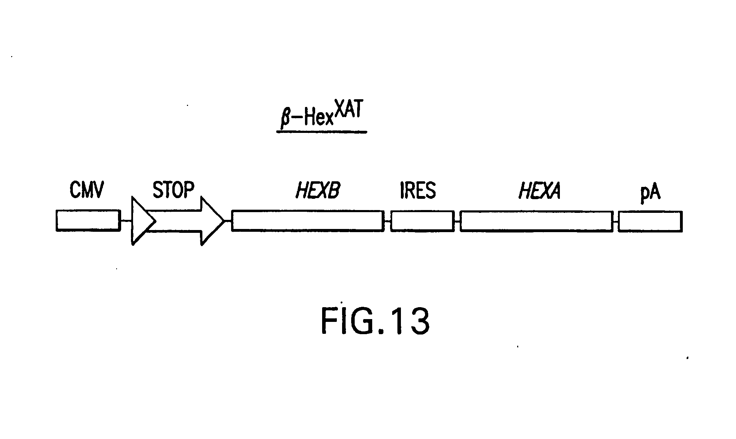

Figure 13 shows a transcription termination cassette (STOP) flanked by 2 loxP sites was inserted between the promoter CMV and the therapeutic gene HexB-IRES-HexA. This results in inhibition of gene expression, until the STOP cassette is exsionally removed via the action of cre recombinase. The termination stop can consist of for example, a neomycin gene, whose termination signal acts as a termination signal for the rest of the transcript. Any reporter gene could be inserted and used in this way.

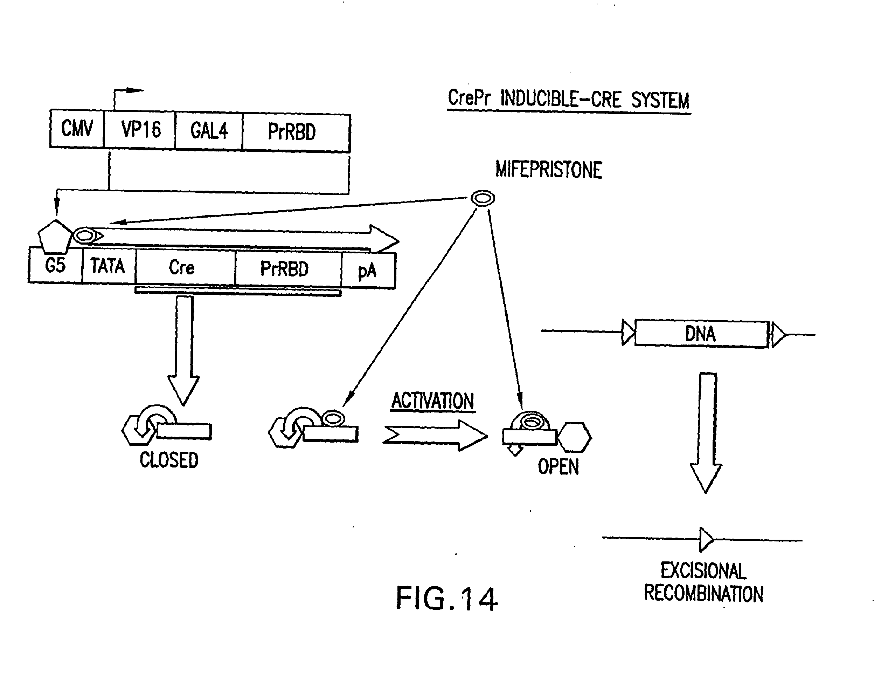

Figure 14 shows a dually regulated inducible cre-recombinase system which was constructed. The activity of this construct is regulated exogenously by RU486. Furthermore, a stable cell line for this system was developed, whereby addition of RU486 in the culture media results in activation of cre-recombinase and subsequently excisional recombination of DNA, such as a transcription termination cassette flanked by 2 loxP sites.

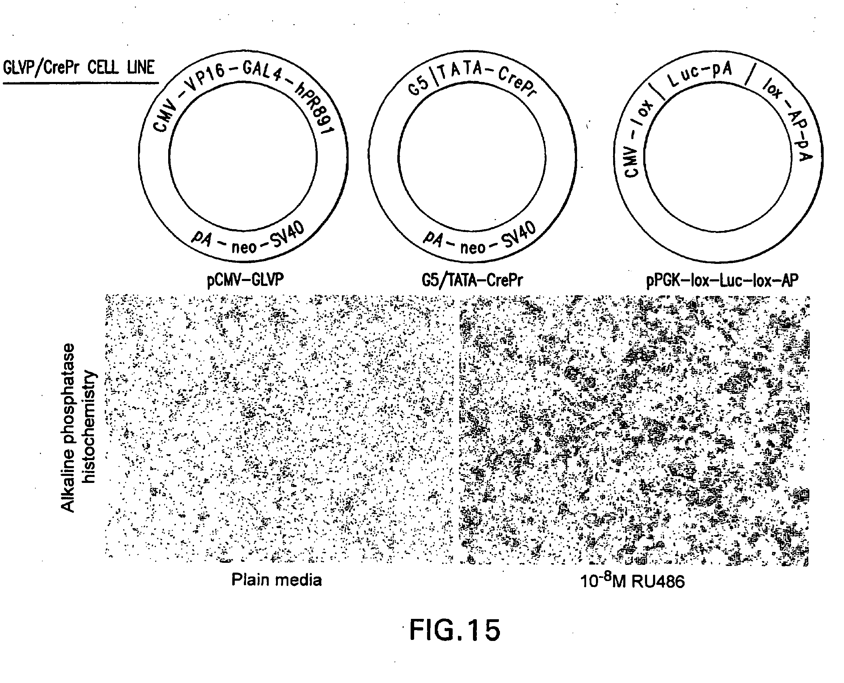

Figure 15 shows an example of the function of stable cell line, named GLVP/CrePr cell line, described in figure 14. In this case, the dual reporter vector CMV-lox-Luc-lox-AP was transiently transfected into the cell line. Alkaline phospatase (AP) activity was evaluated in vitro after the addition of RU486 to the culture media by an AP histochemical staining method.

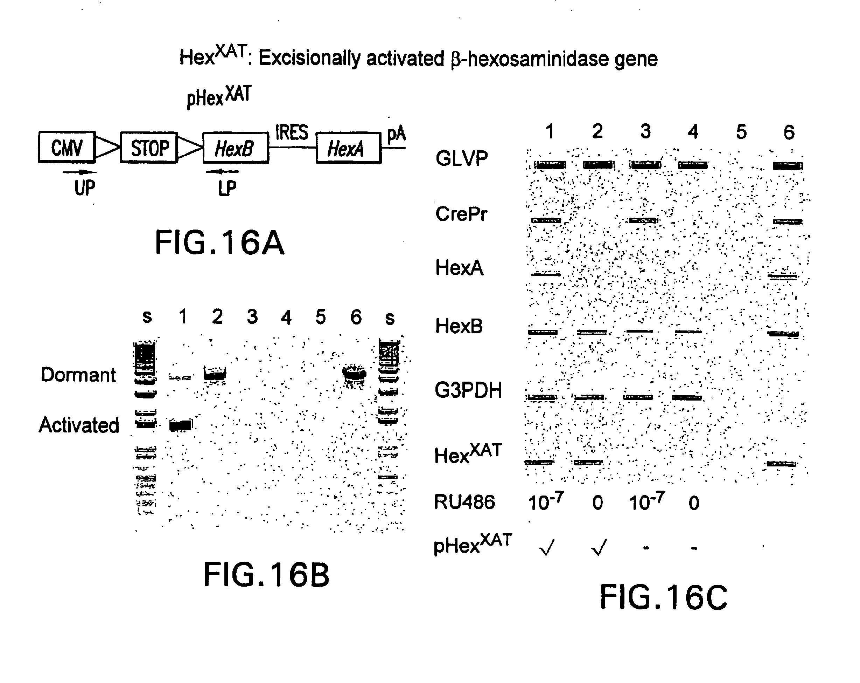

Figure 16A shows the excisionally activated β-hexosaminidase gene Hex^{XAT} was constructed by placing a floxed transcription termination cassette (STOP) upstream to the first open reading frame: CMV-loxP-STOP-loxP-HexB-IRES-HexA. Figure 16B shows Hex^{XAT} was transiently transfected into our inducible cre cell line. Activation of cre-recombinase resulted in loxP directed DNA recombination and excision of the STOP cassette. Figure 16C Cre-mediated activation of Hex^{XAT} resulted in HexA and HexB upregulation (column 1). RU486 stimulation of GL VP/CrePr results in site-directed recombination and subsequent activation of a dormant transcriptional unit. A. shows the p Hex^{XAT}, a bicistronic transgene comprised of a "floxed" transcription-termination cassette (STOP), and both isoforms of the human β-hexosaminidase, was transiently tmasfected into the GLVP/CrePr cell line. B. RU-486 administration resulted in loxP-directed excisional recombination, C. resulting in transcriptional activation and synthesis of HexA and HexB mRNA.

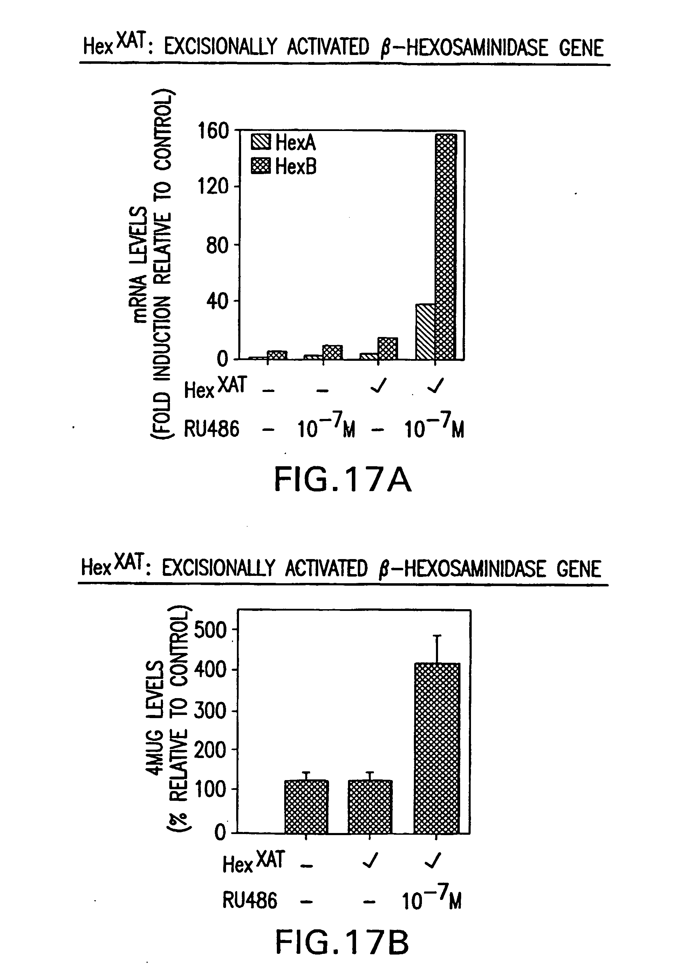

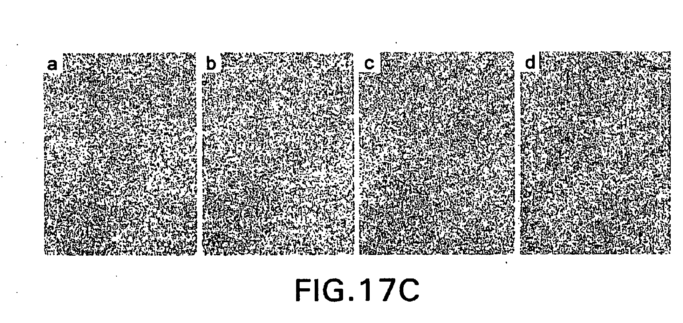

Figure 17 shows the semi-quantitative analysis for HexA and HexB showed induction of gene transcription following Hex^{XAT} activation at (A) the mRNA level, (B) enzyme activity level in vitro, as well as (C) histochemical level in situ. RU486 significantly induces β-hexosaminidase expression in the GLVP/CrePr cell line. β-hexosaminidase activity was found significantly upregulated in p Hex^{XAT} -transfected GLVP/CrePr cells 4 days after RU486 administration at the (A) HexA & HexB mRNA, (B) enzyme activity in vitro, as well as (C) in fixed monolayers in situ, as assessed by RTPCR, 4-MUG fluorescence and X-Hex histochemistry, respectively.

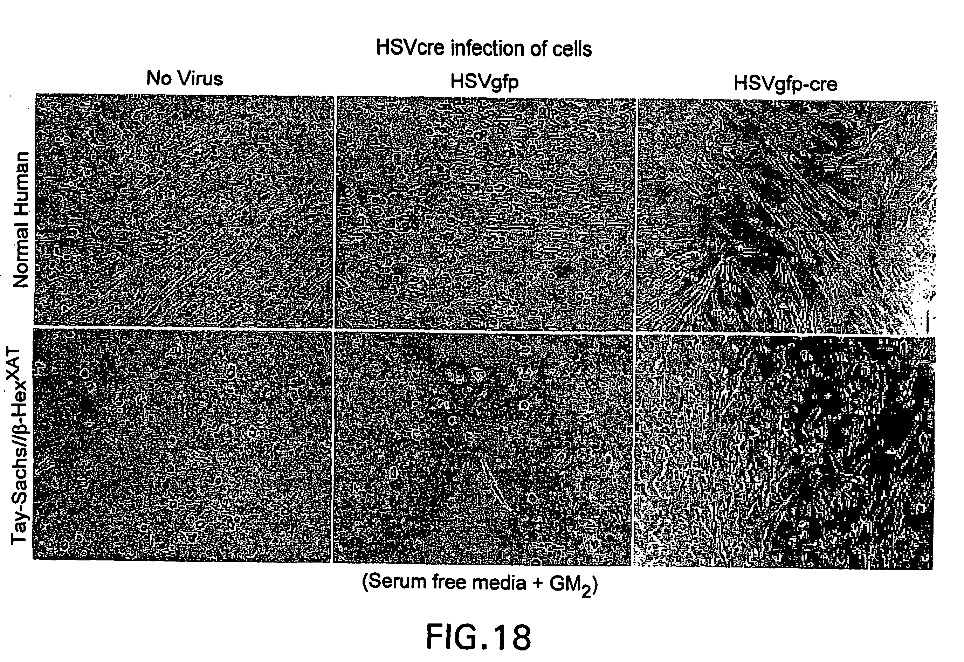

Figure 18 shows Hex^{XAT} was stably expressed in fibroblasts derived from a patient with Tay-Sachs disease (TSD). Gene activation was mediated by infection of the cellos with a HSV aplicon viral vector capable of transducing cells with the cre recombinase. This figure demonstrates that activation of the Hex gene results in protection of the TSD cells from death following GM₂ substrate challenge.

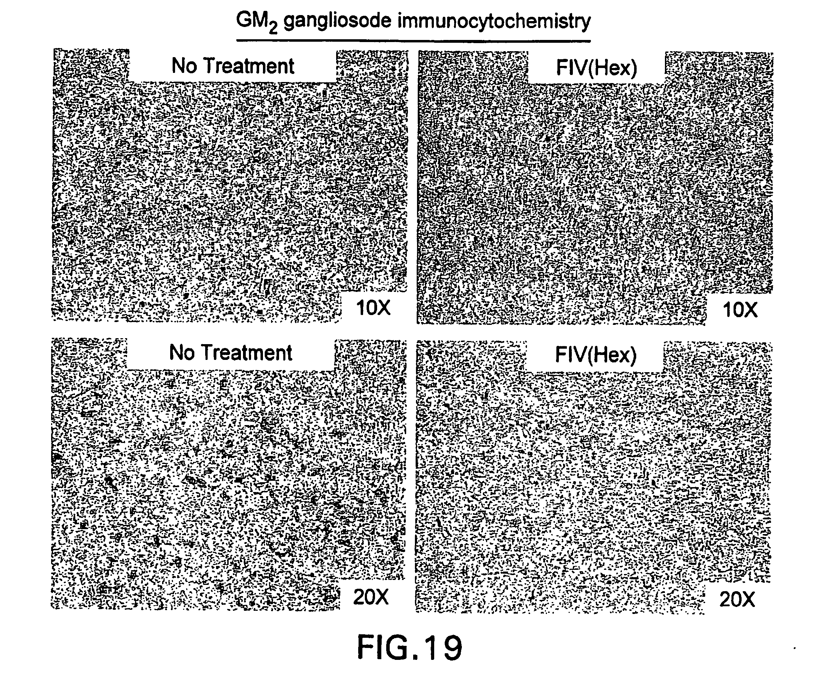

Figure 19 shows that the virus produced in Figure 3 above can resolve GM2 storage in TSD cells cultured in vitro.

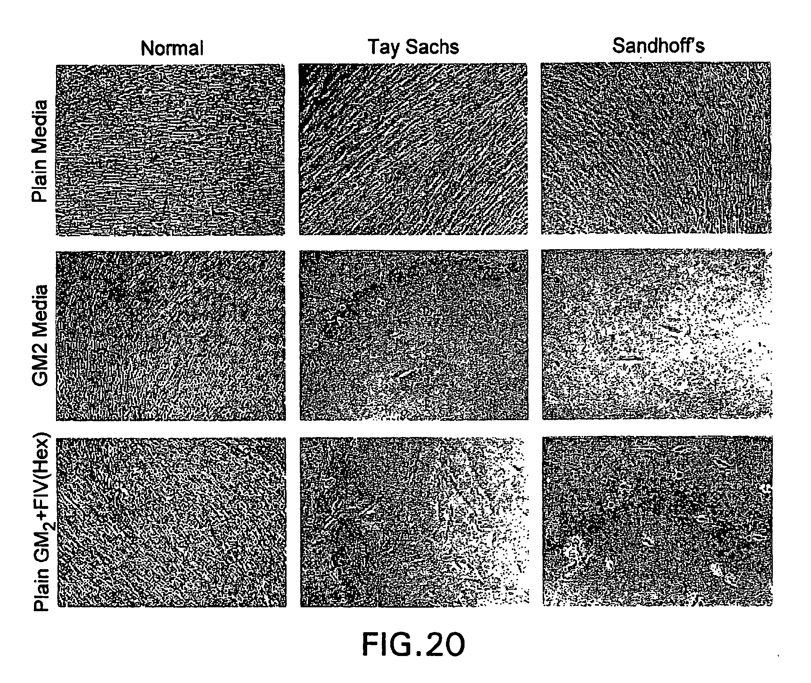

Figure 20 shows the Hex gene was cloned in the FIV backbone as shown in Fig.3 producing the virus FIV(Hex), which was then used to infect TSD cells challenged with GM₂ substrate. This figures shows that delivery of our Hex gene with FIV(Hex) in TSD cells in vitro confers protection to cell death following GM₂ administration.

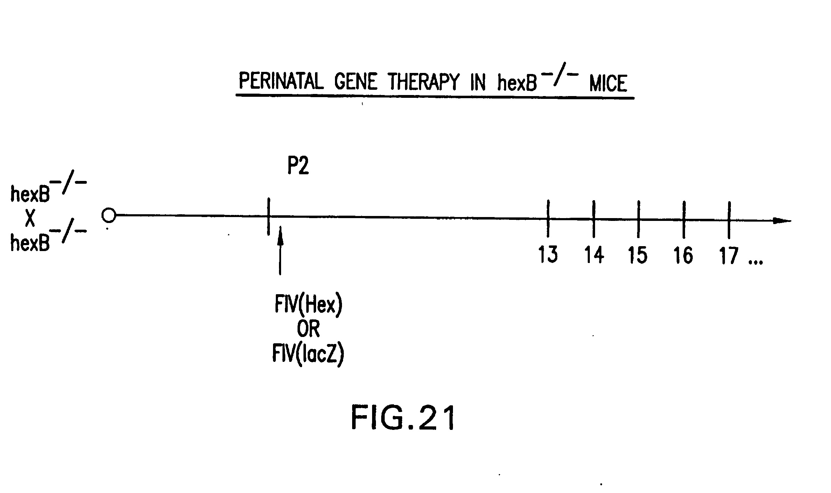

Figure 21 shows HexB^{-/-} knock out pups (2 days) were injected 100uL of FIV(Hex) virus intraperitoneally. The animals were monitored weekly while they assumed growth until sacrificed (16-18 weeks of age).

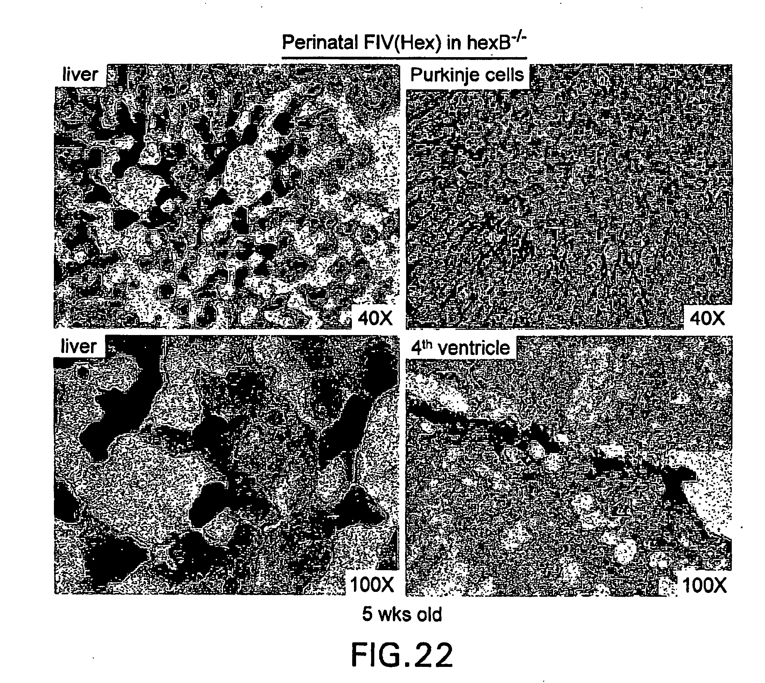

Figure 22 shows expression of HEXB protein in adult mice that were injected with the FIV(Hex) virus as infants 2 days after birth. HEXB protein expression was detected by immunocytochemistry in the liver and brain of these mice.

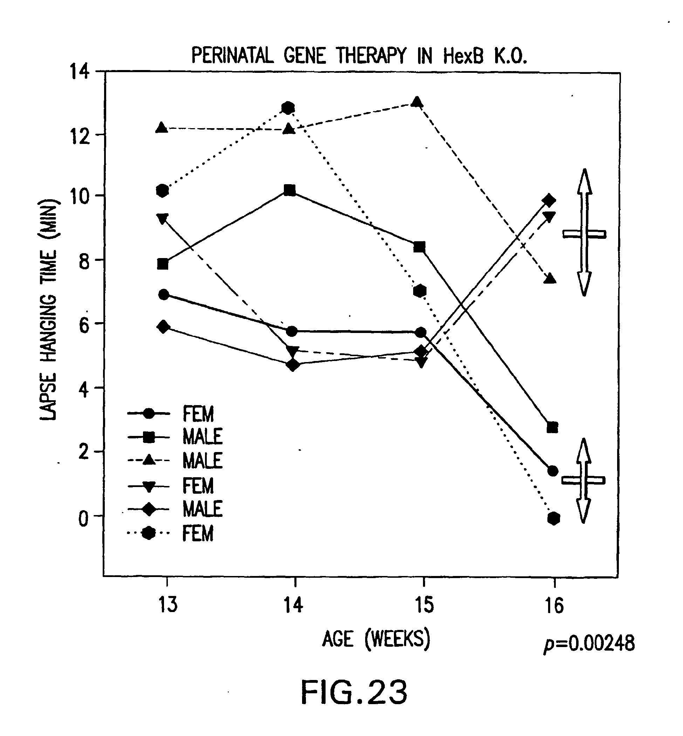

Figure 23 shows locomotive performance in relation to age (in weeks) of 6 mice that were treated 2 days after birth: 3 mice were injected with FIV(Hex) and 3 with FIV(lacZ) and served as controls. At 16 weeks of age, the "classic" stage that the hexB knockout mice display the disease, there was significant disease difference between the two groups.

### IV. DETAILED DESCRIPTION

The present invention may be understood more readily by reference to the following detailed description of preferred embodiments of the invention and the Examples included therein and to the Figures and their previous and following description.

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It also understood that for every value disclosed, "about" that value is also disclosed. For example, if the value "10" is disclosed, then "about 10" is also disclosed, even if not specifically recited out as "about 10."

In this specification and in the claims which follow, reference will be made to a number of terms which shall be defined to have the following meanings:

"Optional" or "optionally" means that the subsequently described event or circumstance may or may not occur, and that the description includes instances where said event or circumstance occurs and instances where it does not.

"Primers" are a subset of probes which are capable of supporting some type of enzymatic manipulation and which can hybridize with a target nucleic acid such that the enzymatic manipulation can occur. A primer can be made from any combination of nucleotides or nucleotide derivatives or analogs available in the art which do not interfere with the enzymatic manipulation.

"Probes" are molecules capable of interacting with a target nucleic acid, typically in a sequence specific manner, for example through hybridization. The hybridization of nucleic acids is well understood in the art and discussed herein. Typically a probe can be made from any combination of nucleotides or nucleotide derivatives or analogs available in the art.

### B. Compositions and methods

### 1. Lysosomal disorders

Lysosomal storage disorders are a group of closely related metabolic diseases resulting from deficiency in enzymes essential for the degradation of gangliosides, mucopolysaccharides, as well as other complex macromolecules. With the dysfunction of a lysosomal enzyme, catabolism of correlate substrates remains incomplete, leading to accumulation of insoluble complex macromolecules within the lysosomes. For example, β-hexosaminidase defects result in lysosomal storage of GM₂ gangliosides leading to the development of Tay-Sachs or Sandhoffs disease. Similarly, mucopolysaccharidoses (MPS) are a group of closely related metabolic disorders that result from deficiencies in lysosomal enzymes involved in glycosaminoglycan metabolism, leading to lysosomal mucopolysaccharide storage. Affected patients, depending on the specific disorder and clinical severity, may present with neurodegeneration, mental retardation, paralysis, dementia and blindness, dysostosis multiplex, craniofacial malformations and facial dysfiguration. Below, some of the most common conditions of this family of diseases are summarized.

| Representative examples of common lysosomal storage disorders | | |
|---|---|---|
| Disease | Enzyme Deficiency | Storage Metabolite |
| Glycogenosis-Type 2 | α-1,4-Glucosidase | Glycogen |
| Gangliosidoses | | |
| GM₁ Gangliosidosis | GM₁ ganglioside β-galactosidase | GM₁, ganglioside |
| *Tay-Sachs disease* | *Hexosaminidase* -α *subunit* | *GM₂ ganglioside* |
| *Sandhoff disease* | *Hexosaminidase* -β *subunit* | *GM₂ ganglioside* |
| Sulfatidoses | | |
| Krabbe disease | Galactosylceramidase | galactocerebroside |
| Fabry disease | α-Galactosidase A | ceramide trihexoside |
| Gaucher disease | Glucocerebrosidase | glucocerebroside |
| Niemann-Pick-types A & B | Sphingomyelinase | sphingomyelin |
| Mucopolysacchari doses | α-L-Iduronidase | dermatan/heparan sulfate |
| Hurler's syndrome | L-Iduronosulfate sulfatase | |
| Hunter's syndrome | | |
| Mucolipidoses | | |
| Mucolipidosis - II | Mannose-6-phosphate kinases | mucopolysaccharide/ glycolipid |
| Pseudo-Hurler's | | |
| Fucosidosis | α-Fucosidase | Glycoproteins |
| Mannosidosis | a-Mannosidase | oligosaccharides |
| Wolman Disease | Acid Lipase | triglycerides |

### 2. Histopathology & Pathophysiology - A progressive disorder

In storage diseases, the affected cells become distended and display vacuolated cytoplasms, which appear as swollen lysosomes under the electronic microscope. For example, in the central nervous system, the neurons of the brain, trigeminal and spinal root ganglia in patients suffering from GM₂ gangliodisoses display swollen vacuolated perikarya stored with excessive amounts of lysosomal storage. As a result, these organelles become large in size and numbers, interfering with normal cell functions. The formation of meganeurites, axon hillock enlargements accompanied by secondary neuritic sprouting, present as cardinal histopathological feature of gangliosidoses and mucopolysaccharidoses (Purpura and Suzuki, 1976; Walkley *et al*., 1988). Purpura and Suzuki proposed that meganeurites, and the synapses they develop, contribute to the onset and progression of neuronal dysfunction in storage diseases, by altering electrical properties of neurons and modifying integrative operations of somatodendritic synaptic inputs. In addition, Walkley *et al*. (1991) suggested that this neuroaxonal dystrophy commonly involved GABAergic neurons, and proposed that the resulting defect in neurotransmission in inhibitory circuits may be an important factor underlying brain dysfunction in lysosomal storage diseases. Consequently, the clinical phenotype often includes neurodegeneration, mental retardation, paralysis, dementia and blindness. In addition, some storage disorders also affect peripheral tissues, such as cartilage and bone, resulting in abnormal growth & development of long bones, vertebrae, ribs and jaws, ultimately leading to anomalies of the skeleton, the cranium and dysfiguration of the face (Mucopolysaccharidoses, and Sandhoff's disease to some degree).

One cardinal characteristic of storage disorders is their progressively worsening (progressive) nature. The deficiency of metabolic enzymes results in accumulation of insoluble metabolites in the lysosomes, which becomes excessive and deleterious over time due to the additive effects of accumulating insoluble metabolite storage. For example, patients suffering from mucopolysaccharidoses (Hurler's or Hunter's) display only a mild degree of the disease's phenotype at infancy, but, due to increasing storage over time, progress to severe forms by adolescence, often leading to death (Gorlin *et al*., 1990). This provides a window of opportunity in mammalian development during which the pathophysiological process of the disease can be attenuated by restoring lysosomal enzymatic activity early enough in life to prevent the development of a "full-blown" disease and, perhaps, to reverse its progression.

### 3. Tay-Sachs & Sandhoffs disorders

The lysosomal enzyme β-hexosaminidase is comprised of 2 subunits (peptides), HEX-α and HEX-β, encoded by two distinct genes, HexA and HexB, respectively. β-hexosaminidase exists in 3 isoforms (proteins), HEXA (α/β heterodimer), HEXB (β/β homodimer) and HEXS (α/α homodimer). Mutation of the HexA gene, causing functional problems with the HEX-α polypeptide in humans results in Tay Sachs disease, whereas mutation of the HexB gene, causing functional problems in the β-Hex polypeptide, in Sandhoff's disease. In Tay Sachs disease, HexA mutation results in loss of HEXA isoform (α/β heterodimer), whereas in Sandhoff's disease, HexB mutation results in loss of both HEXA (α/β heterodimer) and HexB (β/β homodimer) isoforms, leading to a more severe clinical phenotype. Affected patients, depending on the clinical severity, may present with neurodegeneration, mental and motor deteriotation, dysarthria, impaired thermal sensitivity, blindness, as well as facial dysfiguration (doll-like and coarse facies). Histopathologically, the cells of the brain (neurons and glia), spleen and cartilage appear swollen with vaculolated/clear perikarya suggestive of lysosomal storage. Biochemical analysis reveals a complete lack of β-hexosaminidase activity accompanied by lysosomal accumulation of GM₂ gangliosides. As a result, the lysosomes become large in size and numbers, significantly crippling normal cellular function. Clinically, it is not uncommon for patients to display only mild features at infancy, but due to increasing storage over time, progress to severe forms of the disease by adolescence (Gorlin *et al*., 1990). Similarly, other affected mammals, such as affected mice pups, display only mild anomalies at birth, but quickly develop their distinct abnormal features (1 month of age).

### 4. Blood brain barrier formation

The blood-brain barrier (BBB) is a structure unique to the central nervous system and is the result of tight junctions between the brain endothelial cells (Goldstein et al., 1986). Previous work (Risau et al., 1986) on the development of mouse BBB using large protein molecules (horse radish peroxidase) suggested BBB formation during the late days of embryonic life (E17 in mouse). Furthermore, BBB in the adult is not absolute; whereby certain areas of the brain do not develop BBB and thus allow for free exchange of molecules through them. These areas include the median eminence (hypothalamus), pituitary, choroids plexus, pineal gland, subfornical organ, organum vasculosum lamina terminalis and area posterma (Risau & Wolburg, 1990). This allows for the intrusion of FIV(Hex) virions into the brain matter through an incomplete BBB as well as through areas lacking BBB during the first few days after birth as discussed in the examples herein. Disclosed herein a diffuse expression of lacZ throughout the brain of P4 mice injected with FIV(lacZ) versus periventricular only localization following "adult administration" was shown.

### 5. Immune system development

Specific immunity in vertebrates is dependent on the host's ability to generate a heterogeneous repertoire of antigen-binding structures that are displayed on the surface of lymphocytes. Immunologic competence arises early in mammalian development. Since the expression of β-Hex therapeutic gene in *hexA*^{*-*/*-*}/*hexB*^{*-*/}*⁻* mice may be perceived as presentation of "non-self' antigens, one needs to consider the possibility of an immune response against human HEXA and HEXB following gene therapy. In these terms, perinatal administration can offer a unique opportunity in gene therapy application. Specifically, numerous studies have documented that the human and mouse neonate is unable to mount satisfactory responses to various antigenic challenges, which in many instances is delayed well beyond infancy (Schroeder et al., 1995). Therefore, due to this "immature" immunologic state of mice and humans early in their postnatal life, perinatal gene therapy is consistent with adequate "training" of the immune system to recognize HEXA and HEXB as "self" antigens circumventing any potential immunologic rejection. It is understood that the transtherapy can take place in an infant as well.

Disclosed is a bicistronic lentiviral vector comprising a nucleic acid sequence having at least 70% identity to the sequence set forth in SEQ ID NO:2 and encoding the subunit α of the β -hexosaminidase (HEX-α), a nucleic acid sequence having at least 70% identity to the sequence set forth in SEQ ID NO:4 and encoding the subunit β of the β-hexosaminidase (HEX-β), a promoter, and an integrated ribosomal entry site (IRES), wherein the sequence encoding the HEX-β is orientated 5' to the IRES sequence and the IRES sequence is located 5' to the sequence encoding HEX-α, and wherein said bicistronic lentiviral vector produces a functional HEX-α/HEX-β heterodimer. Also disclosed are vectors wherein HEX-β has at least 80% identity to the sequence set forth in SEQ ID NO:3 and the HEX-α has at least 80% identity to the sequence set forth in SEQ ID NO:1, wherein the HEX-β has at least 85% identity to the sequence set forth in SEQ ID NO:3 and the HEX-α has at least 80% identity to the sequences set forth in SEQ ID NO:1, wherein the HEX-β has at least 90% identity to the sequence set forth in SEQ ID NO:3 and the HEX-α has at least 80% identity to the sequence set forth in SEQ ID NO:1, wherein the HEX-β has at least 95% identity to the sequence set forth in SEQ ID NO:3 and the HEX-α has at least 80% identity to the sequence set forth in SEQ ID NO:1, wherein the HEX-β has the sequence set forth in SEQ ID NO:3 and the HEX-α has the sequence set forth in SEQ ID NO:1, wherein the promoter is located 5' to the sequence encoding the HEX-β and the sequence encoding the HEX-β is orientated 5' to the IRES sequence and the IRES sequence is located 5' to the sequence encoding HEX-α.

Also disclosed are cells comprising the vectors defined hereinbefore.

Also disclosed are transgenic non-human animal/mammal having the vectors or cells defined hereinbefore.

Also disclosed are in-vitro methods providing HEX-α and HEX-β in a cell comprising transfecting the cell with the vector defined hereinbefore.

Also disclosed is a composition comprising the vector as described hereinbefore, wherein the composition is in pharmaceutically acceptable form, or is in an effective dosage, wherein the effective dosage is determined as a dosage that reduces the effects of Tay Sachs or Sandoff's disease.

Also disclosed is the use of a vector as defined hereinbefore for preparing an agent for making a transgenic non-human organism.

Also disclosed are methods of making the vector defined hereinbefore, the method comprising linking in an operative way a promoter element, an element comprising sequence encoding HEX-β, a IRES element, and an element encoding HEX-α.

Also disclosed is the use of the vector defined hereinbefore for preparing a medicament for treating a subject having Tay Sachs disease and/or Sandoff disease.

### C. Compositions

### 1. β-Hexosaminidase transgene (β-Hex)

The β-Hexosaminidase protein is a protein comprised of two subunits, one subunit is encoded by the HexA gene and a second subunit encoded by the gene HexB. The human HexA Exon I can be found 316 bp upstream of MstII site; chromosome 15qll-15qter. The human HexA gene can be found at human chromosomal region 15q23 ---- q24. The human HexB gene can be found on chromosome 5, map 5q13.

Disclosed are vectors capable of expressing the HEX-α/HEX-β. Disclosed are vectors comprising a cytomegalovirus (CMV) promoter-driven bicistronic gene (β-Hex) that encodes for both human HexA and HexB genes, which can lead to the synthesis of functional β-hexosaminidase isoenzymes.

The vectors comprise four parts: 1) a promoter, 2) the HexA coding sequence, 3) the HexB coding sequence, and 4) an IRES sequence (integrated ribosomal entry site). These four parts can be integrated into any vector delivery system. In preferred embodiments, the orientation of the four parts is 5'-promoter-HexB-IRES-HexA-3'.

The promoter can be any promoter, such as those discussed herein. It is understood as discussed herein that there are functional variants of the HexA and HexB which can be made. Furthermore, it is understood that that there are functional variants of the IRES element, for example as discussed herein. Typically the genes to be expressed are placed on either side of the IRES sequence.

The IRES element is an internal ribosomal entry sequence which can be iosolated from the encephalomyocarditis crius (ECMV). This element allows multiple genes to be expressed and correctly translated when the genes are on the same construct. IRES sequences are discussed in for example in United States Patent No. 4,937,190.

HexA and HexB cDNA can be obtained from the American Tissue Culture Collection. (American Tissue Culture Collection, Manassas, VA 20110-2209; Hex-α: ATCC# 57206; Hex-β ATCC# 57350) The IRES sequence can be obtained from a number of sources including commercial sources, such as the pIRES expressing vector from Clonetech (Clontech, Palo Alto CA 94303-4334).

Also disclosed are tricistronic constructs encoding for both isoforms of human β-hexosaminidase, hHexA & hHexB, as well as the β-galactosidase reporter gene (*lacZ*).

Global delivery of the disclosed constructs is also disclosed. Disclosed is a pseudotyped feline immunodeficiency virus (FIV) for global β-Hex delivery. Stable expression of the therapeutic gene aids prolonged restoration of the genetic anomaly enhancing treatment efficacy and contributing to long-term therapeutic outcomes. The backbone FIV system has been shown to effectively incorporate, due to its lentiviral properties, the transgene of interest into the host's genome, allowing for stable gene expression (Poeschla et al., 1998). Disclosed herein is stable expression of the reporter gene *lacZ* for over 3 months in mice following perinatal systemic FIV(lacZ) administration.

A model system for the study of these vectors is a mouse that is knockout mouse deficient in both HexA and HexB, since the *hexA*^{*-*/*-*}/*hexB*^{*-*/}*⁻* mouse is characterized by global disruption of the *hexA* and *hexB* genes. Gene disruption in this mouse is global, and therefore, can be used as a model for global replacement. The timing of gene therapy is important as it is closely related to the temporal development of the disorder. *HexA*^{*-*/*-*}/*hexB*^{*-*/*-*} mice display mild phenotype aberrations at birth and quickly develop craniofacial dysplasia by 4-5 weeks of age. Similarly, it is not uncommon for patients suffering from this class of genetic disorders to display only mild degree of the disease at infancy, and to progress to severe forms by adolescence.

### 2. Delivery of the compositions to cells

Delivery can be applied, in general, via local or systemic routes of administration. Local administration includes virus injection directly into the region or organ of interest, versus intravenous (*IV*) or intraperitoneal (*IP*) injections (systemic) aiming at viral delivery to multiple sites and organs via the blood circulation. Previous research on the effects of local administration demonstrated gene expression limited to the site/organ of the injection, which did not extend to the rest of the body (Daly et al., 1999a; Kordower et al., 1999). Furthermore, previous studies have demonstrated successful global gene transfer to multiple tissues and organs in rodents and primates following viral *IV* and *IP* injections (Daly et al., 1999b; Tarntal et al., 2001; McCormack et al., 2001; Lipschutz et al., 2001). Disclosed herein *IP* injection of FIV(lacZ) in mice of adult (3 months old) as well as of perinatal age (P4) resulted in global transfer and expression of the reporter gene lacZ in brain, liver, spleen and kidney. Also disclosed, the levels of expression achieved via *IP* injections were superior to those acquired following local administration directly into the liver.

There are a number of compositions and methods which can be used to deliver nucleic acids to cells, either in vitro or in vivo. These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991) Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### a) Nucleic acid based delivery systems

Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)).

As used herein, lenti-viral vectors are agents that transport the disclosed nucleic acids, such as the β-Hex construct into the cell without degradation and include a promoter yielding expression of the HexA and HexB encoding sequences in the cells into which it is delivered.
The lentiviral vectors include, but are not limited to, SIV vectors, HIV vectors or a hybrid construct of these vectors, including viruses with the HIV backbone. These vectors also include first, second and third generation lentiviruses. Third generation lentiviruses have lentiviral packaging genes split into at least 3 independent plasmids or constructs. Also vectors can be any viral family that share the properties of these viruses which make them suitable for use as vectors. Lentiviral vectors are a special type of retroviral vector which are typically characterized by having a long incubation period for infection. Furthermore, lentiviral vectors can infect non-dividing cells. Lentiviral vectors are based on the nucleic acid backbone of a virus from the lentiviral family of viruses. Typically, a lentiviral vector contains the 5' and 3' LTR regions of a lentivirus, such as SIV and HIV. Lentiviral vectors also typically contain the Rev Responsive Element (RRE) of a lentivirus, such as SIV and HIV.

One type of vector that the disclosed constructs can be delivered in is the VSV-G pseudotyped Feline Immunodeficiency Virus system developed by Poeschla *et al*. (1998). This lentivirus has been shown to efficiently infect dividing, growth arrested as well as post-mitotic cells. Furthermore, due to its lentiviral properties, it allows for incorporation of the transgene into the host's genome, leading to stable gene expression. This is a 3-vector system, whereby each confers distinct instructions: the FIV vector carries the transgene of interest and lentiviral apparatus with mutated packaging and envelope genes. A vesicular stomatitis virus G-glycoprotein vector (VSV-G; Bums et al., 1993) contributes to the formation of the viral envelope *in trans*. The third vector confers packaging instructions *in trans* (Poeschla *et al*., 1998). FIV production is accomplished *in vitro* following co-transfection of the aforementioned vectors into 293-T cells. The FIV-rich supernatant is then collected, filtered and can be used directly or following concentration by centrifugation. Titers routinely range between 10⁴ - 10⁷ bfu/ml.

### b) Non-nucleic acid based systems

The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

Thus, the compositions can comprise, in addition to the disclosed constructs or vectors for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham et al. Am. J. Resp. Cell. Mol. Biol. 1:95-100 (1989); Felgner et al. Proc. Natl. Acad. Sci USA 84:7413-7417 (1987): U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the nucleic acid or vector of this invention can be delivered *in vivo* by electroporation, the technology for which is available from Genetronics, Inc. (San Diego, CA) as well as by means of a SONOPORATION machine (ImaRx Pharmaceutical Corp., Tucson, AZ).

The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other speciifc cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo*. The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

Nucleic acids that are delivered to cells which are to be integrated into the host cell genome, typically contain integration sequences. These sequences are often viral related sequences, particularly when viral based systems are used. These viral intergration systems can also be incorporated into nucleic acids which are to be delivered using a non-nucleic acid based system of deliver, such as a liposome, so that the nucleic acid contained in the delivery system can be come integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### c) In vivo/ex vivo

As described herein, the compositions can be administered in a pharmaceutically acceptable carrier and can be delivered to the subjects cells *in vivo* and/or *ex vivo* by a variety of mechanisms well known in the art (e.g., uptake of naked DNA, liposome fusion, intramuscular injection of DNA via a gene gun, endocytosis and the like).

If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

If in vivo delivery methods are performed the methods can be designed to deliver the nucleic acid constructs directly to a particular cell type, via any delivery mechanism, such as intra-peritoneal injection of a vector construct. In this type of delivery situation, the nucleic acid constructs can be delivered to any type of tissue, for example, brain or neural or muscle. The nucleic acid constructs can also be delivered such that they generally deliver the nucleic acid constructs to more than one type of cell. This type of delivery can be accomplished, by for example, injecting the constructs intraperitoneally into the flank of the organism. (See Example 2 and figures 8-10). It in certain delivery methods, the timing of the delivery is monitored. For example, the nucleic acid constructs can be delivered at the perinatal stage of the recipients life or at the adult stage.

The disclosed compositions, can be delivered to any type of cell. For example, they can be delivered to any type of mammalian cell. Exemplary types of cells neuron, glia, fibroblast, chondrocyte, osteocyte, endothelial, and hepatocyte.

### 3. Expression systems

The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### a) Viral Promoters and Enhancers

Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTF.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

In certain embodiments the promoters are constitutive promoters. This can be any promoter that causes transcription regulation in the absence of the addition of other factors. Examples of this type of promoter are the CMV promoter and the beta actin promoter, as well as others dicussed herein. In certain embodiments the promoter can consist of fusions of one or more different types of promoters. For example, the regulatory regions of the CMV promoter and the beta actin promoter are well known and understood, examples, of which are disclosed herein. Parts of these promoters can be fused together to, for example, produce a CMV-beta actin fusion promoter, such as the one shown in SEQ ID NO:23. It is understood that this type of promoter has a CMV component and a beta actin component. These components can function independently as promoters, and thus, are themselves considered beta actin promoters and CMV promoters. A promoter can be any portion of a known promoter that causes promoter activity. It is well understood that many promoters, including the CMV and Beta Actin promoters have functional domains which are understood and that these can be used as a beta actin promoter or CMV promoter. Furthermore, these domains can be determined. For example, SEQ ID NO:s 21-41 display a number of CMV promoters, beta actin promoters, and fusion promoters. These promoters can be compared, and for example, functional regions delineated, as described herein. Furthermore, each of these sequences can function independently or together in any combination to provide a promoter region for the disclosed nucleic acids.

### b) Markers

The viral vectors can include nucleic acid sequence encoding a marker product. This marker product is used to determine if the gene has been delivered to the cell and once delivered is being expressed. Preferred marker genes are the *E. Coli* lacZ gene, which encodes β-galactosidase, and green fluorescent protein.

In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### c) Post transcriptional regulatory elements

The disclosed vectors can also contain post-transcriptional regulatory elements. Post-transcriptional regulatory elements can enhance mRNA stability or enhance translation of the transcribed mRNA. An exemplary post-transcriptional regulatory sequence is the WPRE sequence isolated from the woodchuck hepatitis virus. (Zufferey R, et al., "Woodchuck hepatitis virus post-transcriptional regulatory element enhances expression of transgenes delivered by retroviral vectors," J Virol; 73:2886-92 (1999)). Post-transcriptional regulatory elements can be positioned both 3' and 5' to the exogenous gene, but it is preferred that they are positioned 3' to the exogenous gene.

### d) Transduction efficiency elements

Transduction efficiency elements are sequences that enhance the packaging and transduction of the vector. These elements typically contain polypurine sequences. An example of a transduction efficiency element is the ppt-cts sequence that contains the central polypurine tract (ppt) and central terminal site (cts) from the HIV-1 pSG3 molecular clone (SEQ ID NO:1 bp 4327 to 4483 of HIV-1 pSG3 clone).

### e) 3' untranslated regions

Expression vectors used in eukaryotic host cells (yeast, fungi, insect, plant, animal, human or nucleated cells) may also contain sequences necessary for the termination of transcription which may affect mRNA expression. These 3' untranslated regions are transcribed as polyadenylated segments in the untranslated portion of the mRNA encoding the exogenous gene. The 3' untranslated regions also include transcription termination sites. The transcription unit also can contain a polyadenylation region. One benefit of this region is that it increases the likelihood that the transcribed unit will be processed and transported like mRNA. The identification and use of polyadenylation signals in expression constructs is well established. Homologous polyadenylation signals can be used in the transgene constructs. In an embodiment of the transcription unit, the polyadenylation region is derived from the SV40 early polyadenylation signal and consists of about 400 bases. Transcribed units can contain other standard sequences alone or in combination with the above sequences improve expression from, or stability of, the construct.

### 4. Sequence similarities

It is understood that as discussed herein the use of the terms homology and identity mean the same thing as similarity. Thus, for example, if the use of the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989. It is understood that any of the methods typically can be used and that in certain instances the results of these various methods may differ, but the skilled artisan understands if identity is found with at least one of these methods, the sequences would be said to have the stated identity, and be disclosed herein.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### 5. Hybridization/selective hybridization

The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 . A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the.methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

### 6. Nucleic acids

There are a variety of molecules disclosed herein that are nucleic acid based, including for example the nucleic acids that encode, for example HexA and HexB, or functional nucleic acids. The disclosed nucleic acids can be made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantagous that the antisense molecule be made up of nucleotide analogs that reduce the degradation of the antisense molecule in the cellular environment.

A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate).

A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties.

Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989,86, 6553-6556),

A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### a) Sequences

There are a variety of sequences related to the HexA, HexB, IRES sequences, and promoter sequences. For example, the HexA and hexB genes have the following Genbank Accession Numbers: M 1641 and NM_000520 for HexA and NM_000521 for HexB,. It is understood that there are numerous Genbank accession sequences related to HexA and HexB.

One particular sequence set forth in SEQ ID NO:4 and having Genbank accession number NM_000521, which is a sequence for human HexB cDNA, is used herein, as an example, to exemplify the disclosed compositions and methods. It is understood that the description related to this sequence is applicable to any sequence related to HexA or HexB unless specifically indicated otherwise. Those of skill in the art understand how to resolve sequence discrepancies and differences and to adjust the compositions and methods relating to a particular sequence to other related sequences. Primers and/or probes can be designed for any of the sequences disclosed herein given the information disclosed herein and that known in the art.

It is also understood for example that there are numerous bicistronic vectors that can be used to create the β-Hex construct nucleic acids See for example, Genbank accession no Y11035 and Y11034.

### b) Primers and probes

Disclosed are compositions including primers and probes, which are capable of interacting with, for example, the β-Hex construct nucleic acids, as disclosed herein. In certain embodiments the primers are used to support DNA amplification reactions. Typically the primers will be capable of being extended in a sequence specific manner. Extension of a primer in a sequence specific manner includes any methods wherein the sequence and/or composition of the nucleic acid molecule to which the primer is hybridized or otherwise associated directs or influences the composition or sequence of the product produced by the extension of the primer. Extension of the primer in a sequence specific manner therefore includes, but is not limited to, PCR, DNA sequencing, DNA extension, DNA polymerization, RNA transcription, or reverse transcription. Techniques and conditions that amplify the primer in a sequence specific manner are preferred. In certain embodiments the primers are used for the DNA amplification reactions, such as PCR or direct sequencing. It is understood that in certain embodiments the primers can also be extended using non-enzymatic techniques, where for example, the nucleotides or oligonucleotides used to extend the primer are modified such that they will chemically react to extend the primer in a sequence specific manner. Typically the disclosed primers hybridize with, for example, the β-Hex construct nucleic acid, or region of the β-Hex construct nucleic acids or they hybridize with the complement of the β-Hex construct nucleic acids or complement of a region of the β-Hex construct nucleic acids.

### 7. Peptides

### a) Protein variants

As discussed herein there are numerous variants of the HEX-α and HEX-β proteins that are known and herein contemplated. In addition, to the known functional species and allelic variants of HEX-α and HEX-β there are derivatives of the HEX-α and HEX-β proteins which also function in the disclosed methods and compositions. Protein variants and derivatives are well understood to those of skill in the art and in can involve amino acid sequence modifications. For example, amino acid sequence.modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from I to 10 amino acid residues; and deletions will range about from I to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables I and 2 and are referred to as conservative substitutions.

TABLE 1:Amino Acid Abbreviations

| **Amino Acid** | **Abbreviations** |
|---|---|
| alanine | AlaA |
| allosoleucine | AIle |
| arginine | ArgR |
| asparagine | AsnN |
| aspartic acid | AspD |
| cysteine | CysC |
| glutamic acid | GluE |
| glutamine | GInK |
| glycine | GIyG |
| histidine | HisH |
| isolelucine | IleI |
| leucine | LeuL |
| lysine | LysK |
| phenylalanine | PheF |
| proline | ProP |
| pyroglutamic acidp | Glu |
| serine | SerS |
| threonine | ThrT |
| tyrosine | TyrY |
| tryptophan | TrpW |
| valine | VaIV |

| TABLE 2:Amino Acid Substitutions | |
|---|---|
| Original Residue Exemplary Conservative Substitutions, others are known in the art. | |
| Ala | ser |
| Arg | lys, gln |
| Asn | gln; his |
| Asp | glu |
| Cys | ser |
| Gln | asn, lys |
| Glu | asp |
| Gly | pro |
| His | asn;gln |
| Ile | leu; val |
| Leu | ile; val |
| Lys | arg; gln; |
| Met | Leu; ile |
| Phe | met; leu; tyr |
| Ser | thr |
| Thr | ser |
| Trp | tyr |
| Tyr | trp; phe |
| Val | ile; leu |

Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, He, Leu; Asp, Glu; Asn, GIn; Ser, Thr; Lys, Arg; and Phe, Tyr. Such conservatively substituted variations of each explicitly disclosed sequence are included within the mosaic polypeptides provided herein.

Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

It is understood that one way to define the variants and derivatives of the disclosed proteins herein is through defining the variants and derivatives in terms of homology/identity to specific known sequences. For example, SEQ ID NO: 1 sets forth a particular sequence of HEX-α and SEQ ID NO:3 sets forth a particular sequence of a HEX-β protein. Specifically disclosed are variants of these and other proteins herein disclosed which have at least, 70% or 75% or 80% or 85% or 90% or 95% homology to the stated sequence. Those of skill in the art readily understand how to determine the homology of two proteins. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989.

It is understood that the description of conservative mutations and homology can be combined together in any combination, such as embodiments that have at least 70% homology to a particular sequence wherein the variants are conservative mutations.

As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. For example, one of the many nucleic acid sequences that can encode the protein sequence set forth in SEQ ID NO:3 is set forth in SEQ ID NO:4. Another nucleic acid sequence that encodes the same protein sequence set forth in SEQ ID NO:3 is set forth in SEQ ID NO:11. In addition, for example, a disclosed conservative derivative of SEQ ID NO:3 is shown in SEQ ID NO: 12, where the valine (V) at position 21 is changed to a isoleucine (I). It is understood that for this mutation all of the nucleic acid sequences that encode this particular derivative of the SEQ ID NO:3 polypeptide are also disclosed. It is also understood that while no amino acid sequence indicates what particular DNA sequence encodes that protein within an organism, where particular variants of a disclosed protein are disclosed herein, the known nucleic acid sequence that encodes that protein in the particular organism from which that protein arises is also known and herein disclosed and described.

### 8. Pharmaceutical carriers/Delivery of pharamceutical products

As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et a]., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo*. The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

The compositions, including antibodies, can be used therapeutically in combination with a pharmaceutically acceptable carrier.

Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids or binders may be desirable..

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### 9. Chips and micro arrays

Disclosed are chips where at least one address is the sequences or part of the sequences set forth in any of the nucleic acid sequences disclosed herein. Also disclosed are chips where at least one address is the sequences or portion of sequences set forth in any of the peptide sequences disclosed herein.

Also disclosed are chips where at least one address is a variant of the sequences or part of the sequences set forth in any of the nucleic acid sequences disclosed herein. Also disclosed are chips where at least one address is a variant of the sequences or portion of sequences set forth in any of the peptide sequences disclosed herein.

### 10. Computer readable mediums

It is understood that the disclosed nucleic acids and proteins can be represented as a sequence consisting of the nucleotides of amino acids. There are a variety of ways to display these sequences, for example the nucleotide guanosine can be represented by G or g. Likewise the amino acid valine can be represented by Val or V. Those of skill in the art understand how to display and express any nucleic acid or protein sequence in any of the variety of ways that exist, each of which is considered herein disclosed. Specifically contemplated herein is the display of these sequences on computer readable mediums, such as, commercially available floppy disks, tapes, chips, hard drives, compact disks, and video disks, or other computer readable mediums. Also disclosed are the binary code representations of the disclosed sequences. Those of skill in the art understand what computer readable mediums. Thus, computer readable mediums on which the nucleic acids or protein sequences are recorded, stored, or saved.

Disclosed are computer readable mediums comprising the sequences and information regarding the sequences set forth herein.

### 11. Kits

Disclosed herein are kits that are drawn to reagents that can be used in practicing the methods disclosed herein. The kits can include any reagent or combination of reagent discussed herein or that would be understood to be required or beneficial in the practice of the disclosed methods. For example, the kits could include primers to perform the amplification reactions discussed in certain embodiments of the methods, as well as the buffers and enzymes required to use the primers as intended.

### D. Methods of making the compositions

The compositions disclosed herein and the compositions necessary to perform the disclosed methods can be made using any method known to those of skill in the art for that particular reagent or compound unless otherwise specifically noted.

The disclosed viral vectors can be made using standard recombinant molecular biology techniques. Many of these techniques are illustrated in Maniatis (Maniatis et al., "Molecular Cloning--A Laboratory Manual," (Cold Spring Harbor Laboratory, Latest.edition) and Sambrook et al., Molecular Cloning: A Laboratory Manual, 2nd Ed., Cold Spring Harbor Laboratory, Cold Spring Harbor, New York, 1989.

### 1. Nucleic acid synthesis

For example, the nucleic acids, such as, the oligonucleotides to be used as primers can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System Plus DNA synthesizer (for example, Model 8700 automated synthesizer of Milligen-Biosearch, Burlington, MA or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

### 2. Peptide synthesis

One method of producing the disclosed proteins is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (t*ert* -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY. Alternatively, the peptide or polypeptide is independently synthesized *in vivo* as described herein. Once isolated, these independent peptides or polypeptides may be linked to form a peptide or fragment thereof via similar peptide condensation reactions.

For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

### 3. Processes for making the compositions

Disclosed are processes for making the compositions as well as making the intermediates leading to the compositions. There are a variety of methods that can be used for making these compositions, such as synthetic chemical methods and standard molecular biology methods. It is understood that the methods of making these and the other disclosed compositions are specifically disclosed.

Disclosed are vectors produced by the process comprising linking in an operative way a promoter element, a HexB element, a IRES element, and a HexA element.

Disclosed are vectors produced by the process comprising linking in an operative way nucleic acid molecules comprising sequences set forth in SEQ ID NO: 10 and SEQ ID NO:4.

Also disclosed are vectors produced by the process comprising linking in an operative way nucleic acid mo-lecules comprising sequences having 80% identity to sequences set forth in SEQ ID NO:10 and SEQ ID NO:4.

Also disclosed are vectors produced by the process comprising linking in an operative way nucleic acid molecules comprising sequences that hybridizes under stringent hybridization conditions to sequences set forth in SEQ ID NO:10 and SEQ ID NO:4.

Disclosed are vectors produced by the process comprising linking in an operative way a nucleic acid molecule comprising a sequence encoding HEX-β and HEX-α peptides and a sequence controlling an expression of the sequence encoding HEX-β and HEX-α.

Disclosed are vectors produced by the process comprising linking in an operative way a nucleic acid molecule comprising a sequence encoding HEXβ and HEX-α peptides wherein the HEX-β and HEX-α peptides have 80% identity to the peptides set forth in SEQ ID NO:1 and SEQ ID NO:3 and a sequence controlling expression ' of the sequences encoding the peptides.

Disclosed are vectors produced by the process comprising linking in an operative way a nucleic acid molecule comprising a sequence encoding HEXβ and HEX-α peptides wherein the HEX-β and HEX-α peptides have 80% identity to the peptides set forth in SEQ ID NO:1 and SEQ ID NO:3, wherein any change from the sequences set forth in SEQ ID NO:1 and SEQ ID NO:3 are conservative changes and a sequence controlling expression of the sequences encoding the peptides.

Disclosed are cells produced by the process of transforming the cell with any of the disclosed nucleic acids. Disclosed are cells produced by the process of transforming the cell with any of the non-naturally occurring disclosed nucleic acids.

Disclosed are any of the disclosed peptides produced by the process of expressing any of the disclosed vectors. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.

Disclosed are non-human animals produced by the process of transfecting a cell within the animal with any of the nucleic acid molecules disclosed herein. Disclosed are non-human animals produced by the process of transfecting a cell within the animal any of the nucleic acid molecules disclosed herein, wherein the non-human animal is a non-human mammal. Also disclosed are non-human animals produced by the process of transfecting a cell within the non-human animal any of the nucleic acid molecules disclosed herein, wherein the non-human mammal is mouse, rat, rabbit, cow, sheep, pig, or non-human primate. Also disclosed are non-human mammals wherein non-human mammal is a murine, ungulate, or non-human primate.

Also disclose are non-human animals produced by the process of adding to the animal any of the cells disclosed herein.

### E. Methods of using the compositions

### 1. Methods of using the compositions as research tools

The disclosed compositions can be used in a variety of ways as research tools. For example, the disclosed compositions, the β-Hex constructs, and other nucleic acids, such as SEQ ID NOs:10 and 4 can be used to produce organisms, such as transgenic or knockout mice, which can be used as model systems for the study of Tay Sachs and Sandoffs disease.

### 2. Methods of gene modification and gene disruption

The disclosed compositions and vectors can be used for targeted gene disruption and modification in any animal that can undergo these events. Gene modification and gene disruption refer to the methods, techniques, and compositions that surround the selective removal or alteration of a gene or stretch of chromosome in an animal, such as a mammal, in a way that propagates the modification through the germ line of the mammal. In general, a cell is transformed with a vector which is designed to homologously recombine with a region of a particular chromosome contained within the cell, as for example, described herein. This homologous recombination event can produce a chromosome which has exogenous DNA introduced, for example in frame, with the surrounding DNA. This type of protocol allows for very specific mutations, such as point mutations, to be introduced into the genome contained within the cell. Methods for performing this type of homologous recombination are disclosed herein.

One of the preferred characteristics of performing homologous recombination in mammalian cells is that the cells should be able to be cultured, because the desired recombination event occurs at a low frequency.

Once the cell is produced through the methods described herein, a non-human animal can be produced from this cell through either stem cell technology or cloning technology. For example, if the cell into which the nucleic acid was transfected was a stem cell for the organism, then this cell, after transfection and culturing, can be used to produce an organism which will contain the gene modification or disruption in germ line cells, which can then in turn be used to produce another animal that possesses the gene modification or disruption in all of its cells. In other methods for production of an animal containing the gene modification or disruption in all of its cells, cloning technologies can be used. These technologies generally take the nucleus of the transfected cell and either through fusion or replacement fuse the transfected nucleus with an oocyte which can then be manipulated to produce an animal. The advantage of procedures that use cloning instead of ES technology is that cells other than ES cells can be transfected. For example, a fibroblast cell, which is very easy to culture can be used as the cell which is transfected and has a gene modification or disruption event take place, and then cells derived from this cell can be used to clone a whole non-human animal.

### 3. Therapeutic Uses

Effective dosages and schedules for administering the compositions may be determined empirically, and making such determinations is within the skill in the art. The dosage ranges for the administration of the compositions are those large enough to produce the desired effect in which the symptoms disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products.

Following administration of a disclosed composition, such as the disclosed constructs, for treating, inhibiting, or preventing Tay Sachs or Sandoffs disease, the efficacy of the therapeutic construct can be assessed in various ways well known to the skilled practitioner. For instance, one of ordinary skill in the art will understand that a composition, such as the disclosed constructs, disclosed herein is efficacious in treating Tay Sachs or Sandoffs disease or inhibiting or reducing the effects of Tay Sachs or Sandoffs disease in a subject by observing that the composition reduces the onset of the conditions associated with these diseases. Furthermore, the amount of protein or transcript produced from the constructs can be analyzed using any diagnostic method. For example, it can be measured using polymerase chain reaction assays to detect the presence of construct nucleic acid or antibody assays to detect the presence of protein produced from the construct in a sample (e.g., but not limited to, blood or other cells, such as neural cells) from a subject or patient.

### F. Examples

The following examples are put forth so as to provide those of ordinary skill in the art with a complete disclosure and description of how the compounds, compositions, articles, devices and/or methods claimed herein are made and evaluated, and are intended to be purely exemplary of the invention.

Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1 Making β-Hex constructs

### a) Construction of bicistronic β-Hex construct

A bicistronic construct encoding for both isoforms of human β-hexosaminidase, hHexA and hHexB was made (Figure 1). hHexB cDNA was isolated following *Xho I* digestion of pHexB43 (ATCC, Manassas VA) and cloned into the *Xho* I site of pIRES (Clonetech Laboratories, Palo Alto CA) downstream of the vector's cytomegalovirus (CMV) promoter sequence. The HexA cDNA was isolated from pBHA-5 (ATCC, Manassas VA) by *Xho I* digestion and was subsequently inserted into the *Xba I* site of pIRES(HexB) downstream of the vectors IRES cassette by blunt ligation. In this construct, the cytomegalovirus promoter (CMV) drives transgene expression, and the translation of the second open reading frame, *HexB*, is facilitated by an internal ribosomal entry sequence (IRES).

### b) Results

The HEX/acZ encodes for both isoforms of human β-hexosaminidase, HexA & HexB. (Figure 1) The vector *p*HEX*lacZ* is shown in Figure 1(A). BHK^{HexlacZ} are developed by stable *HexlacZ* transduction. Figure 1(B) shows that the cells transfected with the *p*HEX*lacZ* vector stain positively by X-gal histochemistry. Furthermore, HexA & HexB mRNA was detected by RT-PCR in total RNA extracts (Figure 1(C)). Likewise, not only was transcript of *p*HEX*lacZ* vector identified, human HEXA and human HEXB proteins were detected in the transfected BHK^{HexlacZ} cells by imunocytochemistry. (Figure 1(D₁) and 1(E₁). This data indicates that the disclosed constructs can be expressed in target cells and that sufficient levels of protein are produced within these cells.

The β-Hex therapeutic gene is capable of correcting deficiencies in cells that are not transfected through cross-correction. (Figure 2) An important property of the β-Hex transgene is the products hHEXA & hHEXB have the ability to cross-correct, specifically, to be released extracellularly and then to be absorbed via paracrine pathways by other cells whereby they contribute to β-hexosaminidase activity. BHK^{HexlacZ} cells were cultured and the supernatant was collected (conditioned medium), filtered (.45mm) and applied on normal mouse kidney fibroblasts in culture. Forty-eight hours later, the cells were washed thoroughly with phosphate buffered saline, and briefly treated with a trypsin solution to remove extracellular proteins from the cell surfaces. Following trypsin inactivation with Tris/EDTA buffer, the cells were fixed with 4% paraformaldehyde solution and processed by Fast Garnet histochemistry for β-hexosaminidase activity. Fast Garnet histochemistry of murine fibroblasts exposed to (Figure 2A) conditioned medium collected from BHK^{HexlacZ} cells compared to cells exposed to medium from normal parent BHK-21 cells (Figure 2B). These results demonstrate that hHEXA & hHEXB, products of the β-Hex transgene, are released into the extracellular medium and can be absorbed by other cells via paracrine pathways resulting in induction of the cellular β-hexosaminidase.

### 2. Example 2 Transfecting constructs

### a) Construction of the tricistronic β-Hex construct

A tricistronic construct encoding for both isoforms of human β-hexosaminidase, hHexA & hHexB, as well as the β-galactosidase reporter gene (*lacZ*) was also made. hHexB cDNA was isolated following *Xho I* digestion of HexB43 (ATCC, Manassas VA) and cloned into the *Xho* I site of pIRES (Clonetech Laboratories, Palo Alto CA) downstream of the vector's cytomegalovirus (CMV) promoter sequence. The HexA cDNA was isolated from pBHA-5 (ATCC, Manassas VA) by *Xho I* digestion and was subsequently inserted into the *Xba I* site of pIRES(HexB) downstream of the vector's IRES cassette by blunt ligation. A *IRES-lacZ* cassette was obtained from Dr. Howard J. Federoff, University of Rochester School of Medicine and Dentistry, but can be produced using standard recombinant techniques with known reagents and was inserted downstream to HexA into the Sal I site of pHexB-IRES-HexA by blunt ligation. In this construct, the cytomegalovirus promoter (CMV) drives transgene expression, and the translation of the second and third open reading frames (ORF), *HexB* and *lacZ*, respectively, are facilitated by an internal ribosomal entry sequence (IRES). The FIV(Hex) vector was constructed by isolating the HexB-IRES-HexA (β-Hex) fragment of pHexIacZ with *NheI* - *NotI* digestion is present and it was cloneed into the FIV backbone (Poeschla et al., 1998), derived after excising the lacZ cassette from pFIV(lacZ) with *Bpu1102I*, leading to the successful construction of pFIV(Hex) (See Figures 3 and 4). Restriction fragment analysis indicated that pFIV(Hex) was constructed as designed. (Figure 5).

The viral derived IRES sequence can effectively drive the expression of second genes in bicistronic constructs *in vitro* and *in vivo*, (Gurtu *et al*., 1996; Geschwind *et* al., 1996; Havenga *et al*. 1998). Nevertheless, IRES-mediated transcription in bicistronic constructs has been shown to reduce the levels of expression of the second ORF by about 40-50%. Hence, since HexB is necessary in the synthesis of both HEXA (α/β) and HEXB (α/α), it was cloned first in our tricistronic construct. Confirmation of the construct has been achieved by multiple restriction enzyme digestions as well as direct DNA sequencing.

### b) Results

The FIV backbone vector was isolated from the FIV(lacZ) vector following *Sst* II *& Not* I digestion. The bicistronic transgene *HexB-*IRES*-HexA* was extracted from the pHex*lacZ* vector following *Nhe* I & *Not* I digestion, and was cloned into the FIV backbone by blunt ligation. FIV(Hex) digestion with the restriction enzymes *Xho I* and *Sal I* confirmed the cloning. (Figure 6) FIV(Hex) virus was prepared using established methods and was tested *in vitro* as follows. Cultured murine fibroblasts (CrfK cell line) were exposed to FIV(Hex) for 12 hours, followed fresh media change. After 48 hours, cellular DNA and RNA extracts were collected. The presence of viral DNA was assessed by PCR with primers sets specifically designed for HexB (Figure 6A). HexB expression was assessed by RT-PCR (Figure 6B). These results demonstrate the ability of FIV(Hex) to transduce mouse fibroblasts with β-Hex, resulting in transgene mRNA expression. (Figure 6).

The tricistronic vector pHEXlacZ was stably expressed in embryonic hamster kidney fibroblasts (BHK-21; ATCC) following standard transfection laboratory techniques using the LIPOFECTAMINE ® reagent (Gibco BRL) per manufacturer's instructions. Forty-eight hours post-transfection, the cells were treated with 800µg/mL G418 (Gibco BRL) for 10 days, and cell lines were selected, expanded and analyzed for expression of our tricistronic gene as follows. Analysis of the transfected cells showed that cell lines (Crfk, spleen, brain, liver, and kidney) stained positively for X-gal, indicating expression of and translation of the expressed product from the tricistronic vector. (Figure 6)

### 3. Example 4 In vivo use of FIV HEX vectors

FIV(Hex) was constructed by inserting the bicistronic gene HexB-IRES-HexA in the place of the reporter gene lacZ in the FIV backbone vector using standard mmolecular biology techniques. FIV(Hex) was prepared in vitro by transient co-transfection of the transfer vector along with the packaging and envelop plasmids into 293H cells. The virus-rich supernatant was centrifuged and the viral pellet was reconstituted in normal saline, and was then titered in CrfK cells by the X-Hex histochemical method (10⁷-10⁸ infectious particles/ml). The viral solution was injected intraperitoneally to 2 days old HexB^{-/-} knockout mouse pups, which were allowed to reach the critical age of 16 weeks, when they displayed full signs of the lysosomal storage disease. For control, litermates were injected with the FIV(lacZ) virus, which is identical to FIV(Hex), but instead of carrying the HexB-IPES-HexA gene it carries the reporter gene lacZ. Locomotive performance was evaluated by placing the mice on a wire mesh attached on a clear plexiglass cylinder, and turning the wire mesh up-side-down. The lapse time until the mice fell off the wire mesh was recorded on weekly basis until the mice were terminated. It is important to state that at the critical time point of 16 weeks, the FIV(Hex) injected mice showed statistically better locomotive performance compared to FIV(lacZ) injected mice (controls). Furthermore, the FIV(Hex) mice had an extended life span for at least 2-3 additional weeks, at which point they were also terminated because they were showing signs of the disease.

### 4. Example 3 HIV HEX vectors

The HexB-IRES-HexA therapeutic gene was cloned into the Lenti6/V5D-TOPO vector commercially available by Invitrogen (Carlsbad, CA), whereby the cytomegalovirus promoter CMV drives gene expression [in a manner similar to FIV(Hex)]. A virus was constructed whereby the expression of HexB-IRES-HexA is driven by a promoter, such as that show in SEQ ID NO:23, which consists of a beta-actin portion and a CMV portion. This type of promoter has high expression in mammalian cells.

### G. References

Akli S, Guidotti J-E, Vigne E, Perricaudet M, Sandhoff K, Kahn A, Poenaru L (1996) Restoration of hexosaminidase A activity in human Tay-Sachs fibriblasts via adenoviral vector mediated gene transfer. Gene Therapy 3: 769-774

Bowers WJ, Howard DF, Federoff HJ (2000). Discordance between expression and genome transfer titering of HSV amplicon vectors: recommendation for standardized enumeration. Mol Ther 1:294-9.

Bums JC, Friedmann T, Driever W, Burrascano M, Yee J-K (1993) Vescicular stomatitis virus G-glycoprotein pseudotyped retroviral vectors Concentration to very high titer and efficient gene transfer into mammalian and nonmammalian cells. Proc Natl Acad Scie USA 90: 8033-8037.

Cannon PM, Anderson WF (2000) Retroviral vectors for gene therapy. In Gene Therapy: therapeutic mechanisms and strategies. NS Templeton and DD Lasic, Editors. Marcel Dekker Inc, New York, pp 1-16.

Daly TM, Okuyama T, Vogler C, Haskins ME, Muzyczka N, Sands MS (1999a) Intracranial injection of recombinant adeno-associated virus improves cognitive function in a murine model of mucopolysaccharidosis type VII.Hum Gene Ther 10:85-94.

Daly TM, Vogler C, Levy B, Haskins ME, Sands MS (1999b) Neonatal gene transfer leads to widespread correction of pathology in a murine model of lysosomal disease. Proc Natl Acad Sci USA 96: 2296-2300.

Enlow DH, Hans MG (1996) Essentials of facial growth. W.B.Saunders Co, New York.

Goldstein GW, Betz AL, Bowman PD, Dorovini-Zis K (1986) In vitro studies of the blood-brain barrier using isolated brain capillaries and cultured endothelial cells. Ann NY Acad Sci 481:202-13.

Gorlin RJ, Cohen MM, Levin LS (1990). SYNDROMES OF THE HEAD AND NECK, 3rd Edition. Oxford university Press, New York.

Guidotti JE, M. A., Haase G, Caillaud C, McDonell N, Kahn A, Poenaru L (1999) Adenoviral gene therapy of the Tay-Sachs disease in hexosaminidase A deficient knockout mice. Hum Mol Genet 8: 831-838.

Kjaer I, Keeling JW, Hansen BF (1999) The prenatal human cranium-Normal and pathologic development. Munksgaard, Copenhagen, pp 20-51.

Kordower JH, Bloch J, Ma SY, Chu Y, Palfi S, Roitberg BZ, Emborg M, Hantraye P, Deglon N, Aebischer P (1999) Lentiviral gene transfer to the nonhuman primate brain. Exp Neurol 160: 1-16.

Kyrkanides S, Kjaer I, Fisher-Hansen B (1993) The basilar part of the occipital bone in normal and pathological fetuses. J Cranio Genet Develop Biol 13: 184-192.

Kyrkanides S, Bellohusen R, Subtelny JD (1995) Skeletal asymmetries of the nasomaxillary complex in non-cleft and post-surgical unilateral cleft lip and palate individuals. Cleft Pal Craniofac J 32: 428-32.

Kyrkanides S, Bellohusen R, Subtelny JD (1996) Asymmetries of the upper lip and nose in non-cleft and post-surgical unilateral cleft lip and palate individuals. Cleft Palate-Craniofacial Journal, 33:306-10.

Kyrkanides S, Olschowka JA, Williams JP, Hansen JT, O'Banion MK (1999). TNFα and IL-1β mediate ICAM-1 induction via microglia-astrocyte interaction in CNS radiation injury. J Neuroimmunol 95:95-106.

Kyrkanides S, Klambani M, Subtelny JD (2000). Cranial base and facial skeleton asymmetries in unilateral cleft lip & palate individuals. Cleft Palate-Craniofacial Journal 37: 556-561.

Kyrkanides S, Olschowka JA, Whitley P, O'Banion MK (2001). Enhanced glial activation and expression of specific CNS inflammation-related molecules in aged versus young rats following cortical stab injury. In Press, J Neuroimmunol.

Lipschutz GS, Gruber CA, Cao Y, Hardy J, Contag CH, Gaensler KML (2001). In utero delivery of adeno-associated viral vectors: Intraperitoneal gene transfer produces long term expression. Mol Ther 3: 284-92.

McCormack JE, Edwards W, Sensintaffer J, Lillegren L, Kozloski M, Brumm D, Karavodin L, Jolly DJ, Greengard J (2001) Mol Ther 3: 516-525.

Phaneuf D, Wakamatsu N, Huang J-Q, Borowski A, Peterson AC, Fortunato SR, Ritter G, Igdoura SA, Morales CR, Benoit G, Akerman BR, Leclerc D, Hanai N, Marth JD, Trasler JM, Gravel RA (1996) Dramatically different phenotypes in mouse models of human Tay-Sachs and Sandhoff diseases. Hum Mol Genet 5: 1-14.

Poeschla EM, Wong-Stall F, Looney DL (1998) Efficient transduction of nondividing human cells by feline immunodeficiency virus lentiviral vectors. Nature Medicine 4: 354-357.

Proia RL, d'Azzo A, Neufeld EF (1984). Association of a- and b- subunits during the biosynthesis of b-hexosaminidase in cultured human fibroblasts. J Biol Chem 259: 3350-3354.

Risau W, Hallmann R, Albrecht U (1986) Differentiatiom-dependent expression of proteins in brain endothelium during development of the blood-brain barrier. Devel Biol 117: 537-545.

Risau W, Wolburg H (1990) Development of blood-brain barrier. TINS 13:174-178.

Rosenberg A (1999) Bones, joints and soft tissue tumors. In Robbins Pathologic Basis of Disease, 6th edition; Cotran, Kumar & Collins (Ed), W.B.Saunders Co, New York.

Sakurada H, Itoh K, Kuroki Y, Kase R, Shimmoto M, Utsumi K, Ozawa H, Tai T, Hara A, Uyama E (1997) Immunocytochemical detection of accumulated substrates in cultured fibroblasts from patients with the infantile and adult forms of Sandhoff disease. Clin Chim Acta 265: 263-266.

Sango K, McDonald MP, Crawley JN, Mack ML, Tifft CJ, Skop E, Starr CM, Hoffmann A, Sandhoff K, Suzuki K, Proia RL (1996) Mice lacking both subunits of lysosomal β-hexosaminidase display gangliosidosis and mucopolysaccharidosis. Nature Genet 14: 348-352.

Sango K, Yamanaka S, Hoffman A, Okuda Y, Grinberg A, Westphal H, McDonald MP, Crawley JN, Sandhoff K, Suzuki K, Proia RL (1995) Mouse models of Tay-Sachs and Sandhoff diseases differ in neurologic phenotype and ganglioside metabolism. Nature Genet 11: 170-176.

Schroeder WH, Mortari F, Shiokawa S, Kirkham PM, Elgavish RA, Bertrand FE III (1995) Developmental regulation of the human antibody repertoire. Ann NY Acad Scie 764: 242-260.

Suzuki K, Sango K, Proia RL, Langman C (1997) Mice deficient in all forms of lysosomal β-hexosaminidase show mucopolysaccharosis-like pathology. J Neuropath Exp Neurol 56: 693-703.

Tarantal AF, O'Rourke JP, Case SS, Newbound GC, Li J, Lee CI, Baskin CR, Kohn DB, Bunnell BA (2001) Rhesus monkey model for fetal gene transfer: studies with retroviral-based vector systems. Mol Ther 3: 128-138.

Wolff JA, Harding CO (2000) Principles of gene therapy for inborn errors of metabolism. In Gene Therapy: therapeutic mechanisms and strategies. NS Templeton and DD Lasic, Editors. Marcel Dekker Inc, New York, pp 507-533.

### H. Sequences

1. SEQ ID NO:1 Homo sapiens hexosaminidase A (alpha polypeptide) (HEXA), polypeptide Genbank Accession No. XM_037778
2. SEQ ID NO:2 Homo sapiens hexosaminidase A (alpha polypeptide) (HEXA), polynucleotide Genbank Accession No. XM_037778
3. SEQ ID NO:3 Homo sapiens hexosaminidase B (beta polypeptide) (HEXB), polypeptide protein Genbank Accession No XM_032554
4. SEQ ID NO:4 Homo sapiens hexosaminidase B (beta polypeptide) (HEXB), polynucleotide mRNA Genbank Accession No XM_032554
5. SEQ ID NO:5 IRES sequence United States Patent No. 4,937,190 covers entire Vector, and is cited at least for material relating to the pIRES vector)
6. SEQ ID NO:6 Mus musculus hexosaminidase A (Hexa), protein Genbank Accession No, NM_010421
7. SEQ ID NO:7 Mus musculus hexosaminidase A (Hexa), mRNA Genbank Accession No, NM_010421
8. SEQ ID NO:8 FIV(LacZ) construct 12750 bp
9. SEQ ID NO:9: HEX-α polypeptide Genbank accession number NM_000520 (Proia) beta-hexosaminidase A alpha-subunit to human chromosomal region 15q23---q24
10. SEQ ID NO:10 HexA gene Genbank accession number NM_000520 (Proia)
11. SEQ ID NO:11 HexB degenerate cDNA G to A change at position 6
12. SEQ ID NO:12: HEX-β polypeptide conservative substitution of Val21 to 121
13. SEQ ID NO:13 HEX-α polypeptide Genbank accession number M16411 (Tissue sample from ATCC)
14. SEQ ID NO:14 HexA gene Genbank accession number M16411
15. SEQ ID NO:15: HEX-β polypeptide Genbank accession number NM_000521 (Proia) beta-hexosaminidase A alpha-subunit to human chromosomal region chromosome 5 map="5q13"
16. SEQ ID NO:16 HexB gene Genbank accession number NM_000521 Proia
17. SEQ ID NO:17 Mus musculus hexosaminidase B (Hexb), protein. Genbank Accession No. NM_010422
18. SEQ ID NO:18 Mus musculus hexosaminidase B (Hexb), mRNA. Genbank Accession No. NM_010422
19. SEQ ID NO:19 Bactin Hex sequence
20. SEQ ID NO:20 HIV Hex vector sequence
21. SEQ ID NO:21 E02199 DNA encoding chicken beta actin gene promoter.
22. SEQ ID NO:22 Chicken Beta Actin promoter
23. SEQ ID NO:23 CMV-Beta actin promoter
24. SEQ ID NO:24 Fusion promoter-CMV portion
25. SEQ ID NO:25 Fusion promoter - beta actin portion
26. SEQ ID NO:26 Chicken beta actin promoter
27. SEQ ID NO:27 Accession # BD136067. promoter element for sustained gene expression from CMV promoter.
28. SEQ IDNO:28 BD136066 Accession # promoter element for sustained gene expression from CMV promoter.
29. SQ ID NO:29 BD136065 Accession # promoter element for sustained gene expression from CMV promoter.
30. SEQ ID NO:30 BD136064 Accession # promoter element for sustained gene expression from CMV promoter
31. SED ID NO:31 L77202 Accession # Murine Cytomegalovirus early (E1) gene, promoter region.
32. SEQ ID NO:32 X03922 Accession # Human cytomegalovirus (HCMV) IE1 gene promoter region.
33. SEQ ID NO:33 E06566 Accession # Promoter gene of human beta-actin gene.
34. SEQ ID NO:34 E02198 Accession # Dna encoding 3'end region of beta-actin gene promoter
35. SEQ ID NO:35 E02197 Accession # DNA encoding 3'end region of beta-actin gene promoter.
36. SEQ ID NO:36 E02196 Accession # DNA encoding 3'end region of beta-actin gene promoter.
37. SEQID NO:37 E02195 Accession # DNA encoding 3'end region of beta-actin gene promoter.
38. EQ ID NO:38 E02194 Accession # DNA encoding chicken beta-actin gene promoter.
39. SEQ ID NO:39 E01452 Accession # Genomic DNA of promoter of human beta-actin.
40. SEQ ID NO E03011 Accession # DNA encoding hybrid promoter that is composed of chicken beta-actin gene promoter and rabbit beta-globin gene promoter.
41. SEQ ID NO:41 BD015377 Accession # Baculovirus containing minimum CMV promoter.
42. Other cytomegalovirus promoter regions

Other human cytomegalovirus promoter regions can be found in accession numbers M64940, Human cytomegalovirus IE-1 promoter region, M64944 Human cytomegalovirus IE-1 promoter region, M64943 Human cytomegalovirus IE-1 promoter region, M64942 Human cytomegalovirus IE-1 promoter region, M64941 Human cytomegalovirus IE-1 promoter region.

## Claims

1. A bicistronic lentiviral vector comprising a nucleic acid sequence having at least 70% identity to the sequence set forth in SEQ ID NO:2 and encoding the subunit α of the β-hexosaminidase (HEX-α), a nucleic acid sequence having at least 70% identity to the sequence set forth in SEQ ID NO:4 and encoding the subunit β of the β-hexosaminidase (HEX-β), a promoter, and an integrated ribosomal entry site (IRES), wherein the sequence encoding the HEX-β is orientated 5' to the IRES sequence and the IRES sequence is located 5' to the sequence encoding HEX-α, and wherein said bicistronic lentiviral vector produces a functional HEX-α/HEX-β heterodimer.

2. The vector of claim 1, wherein the promoter is located 5' to the sequence encoding the HEX-β.

3. The vector of claim 1 or 2, wherein the vector comprises a second IRES sequence, preferably the second IRES sequence is located 3' to the other parts.

4. The vector of any one of claims 1 to 3, wherein any change from SEQ ID NO:4 or SEQ ID NO:2 is a conservative change in the encoded protein, preferably the HEX-β has the sequence set forth in SEQ ID NO:3 and the HEX-α has the sequence set forth in SEQ ID NO:1.

5. The vector of any one of claims 1 to 3, wherein the sequence encoding HEX-β hybridizes to SEQ ID NO:2 under stringent conditions and wherein the HEX-α element hybridizes to SEQ ID NO:4 under stringent conditions.

6. The vector of claim 1 or 4, wherein the IRES sequence comprises a sequence having at least 70%, 75%, 80%, 85%, 90%, or 95% identity to the sequence set

7. The vector of claim 6, wherein
(i) the promoter sequence comprises a constitutive promoter, preferably the promoter sequence comprises a CMV promoter, most preferably the CMV promoter comprises the sequence set forth in SEQ ID NO:32; and/or
(ii) the promoter sequence comprises a beta actin promoter, preferably the beta actin promoter sequence comprises an avian beta actin promoter sequence, most preferably the beta actin promoter sequence comprises a mammalian beta actin promoter sequence, or the sequence set forth in SEQ ID NO:26; and/or
(iii) the promoter sequence comprises an inducible promoter.

8. The vector of claim 1, which further comprises
(i) a reporter gene, preferably the reporter gene is a lacZ gene, and/or the reporter gene is flanked by recombinase sites, most preferably the recombinase sites are for the cre recombinase; and/or
(ii) a transcription termination site, preferably the transcription termination site is oriented 5' to the promoter sequence, more preferably the transcription termination site is flanked by recombinase sites, most preferably the recombinase sites are for the cre recombinase.

9. The vector of claim 1, wherein the lentiviral vector
(i) comprises a feline immunodeficiency virus, or a human immunodeficiency virus; and/or
(ii) can be stably integrated for at least three months.

10. The vector of claim 1 which comprises a nucleic acid sequence having the sequence set forth in SEQ ID NO:2 and encoding HEX-α, and a nucleic acid sequence having the sequence set forth in SEQ ID NO:4 and encoding HEX-β.

11. A cell comprising the vector of claim 1 or 9.

12. The cell of claim 11, which comprises a neuron, glia cell, fibroblast, chandrocyte, osteocyte, endothelial cell, or hepatocyte.

13. A composition comprising the vector of claim 1 or 9, wherein the composition is in pharmaceutically acceptable form, or is in an effective dosage, wherein the effective dosage is determined as a dosage that reduces the effects of Tay Sachs or Sandoff's disease.

14. A transgenic non-human animal having the vector of claim 1 or 9 integrated into the genome, or having the cell of claim 11.

15. The non-human animal of claim 14, wherein the non-human animal is a mammal, preferably the mammal is a murine, ungulate, or non-human primate, most preferably the mammal is a mouse, rat, rabbit, cow, sheep, or pig.

16. The non-human animal of claim 15, wherein the mammal is a mouse, preferably
(i) the mouse comprises a HexB knockout; or
(ii) the mouse comprises a HexA knockout, optionally the mouse further comprises a HexB knockout.

17. The non-human animal of claim 15, wherein the mammal is a non-human primate.

18. An *in vitro* method of providing HEX-α/HEX-β heterodimer in a cell comprising transfecting the cell with the vector of claim 1.

19. Use of the vector of claim 1 or 9, or the cell of claim 11 for preparing an agent for making a transgenic non-human organism.

20. The use of claim 19, wherein the agent comprises a lentiviral vector and is suitable for transfecting the organism at during a perinatal stage of the organism's development.

21. Use of the composition of claim 13 for preparing a medicament for treating a subject having Tay Sachs disease and/or Sandoff disease.

22. A method of making vector of claim 1, the method comprising linking in an operative way a promoter element, an element comprising sequence encoding HEX-β, a IRES element, and an element encoding HEX-α.

23. The method of claim 22 wherein
(i) the HEX-β element comprises a sequence having at least 80% identity to SEQ ID NO:2 and the HEX-α element comprises a sequence having at least 80% identity to SEQ ID NO:4, preferably any change in SEQ ID NO:1 or SEQ ID NO:3 is a conservative change; and/or
(ii) the sequence encoding HEX-β hybridizes to SEQ ID NO:2 under stringent conditions and wherein the sequence encoding the HEX-α hybridizes to SEQ ID NO:4 under stringent conditions.

24. A method of producing the cell of claim 11, the method comprising administering the vector of claim 1 to the cell.

25. A method of producing a HEX-α/HEX-β heterodimer, the method comprising expressing the nucleic acid comprised by the vector of claim 1.

26. The method of claim 25, further comprising isolating the peptide.

## Patentansprüche

1. Bicistronischer lentiviraler Vektor, der Folgendes umfasst: eine Nucleinsäuresequenz mit wenigstens 70% Identität zu der in SEQ ID Nr. 2 dargelegten Sequenz, die die Untereinheit α der β-Hexosaminidase (HEX-α) codiert, eine Nucleinsäuresequenz mit wenigstens 70% Identität zu der in SEQ ID Nr. 4 dargelegten Sequenz, die die Untereinheit β der β-Hexosaminidase (HEX-β) codiert, einen Promotor und eine interne ribosomale Eintrittsstelle (IRES), wobei die Sequenz, die das HEX-β codiert, 5' zur IRES-Sequenz orientiert ist und sich die IRES-Sequenz 5' von der Sequenz, die HEX-α codiert, befindet und wobei der bicistronische lentivirale Vektor ein funktionelles HEX-α/HEX-β-Heterodimer produziert.

2. Vektor gemäß Anspruch 1, wobei sich der Promotor 5' von der Sequenz, die das HEX-β codiert, befindet.

3. Vektor gemäß Anspruch 1 oder 2, wobei der Vektor eine zweite IRES-Sequenz umfasst, wobei sich die zweite IRES-Sequenz vorzugsweise 3' von den anderen Teilen befindet.

4. Vektor gemäß einem der Ansprüche 1 bis 3, wobei jede Veränderung gegenüber SEQ ID Nr. 4 oder SEQ ID Nr. 2 eine Veränderung ist, die konservativ bezüglich des codierten Proteins ist, wobei das HEX-β vorzugsweise die in SEQ ID Nr. 3 dargelegte Sequenz hat und das HEX-α vorzugsweise die in SEQ ID Nr. 1 dargelegte Sequenz hat.

5. Vektor gemäß einem der Ansprüche 1 bis 3, wobei die Sequenz, die HEX-β codiert, unter stringenten Bedingungen mit SEQ ID Nr. 2 hybridisiert und wobei das HEX-α-Element unter stringenten Bedingungen mit SEQ ID Nr. 4 hybridisiert.

6. Vektor gemäß Anspruch 1 oder 4, wobei die IRES-Sequenz eine Sequenz umfasst, die wenigstens 70%, 75%, 80%, 85%, 90% oder 95% Identität mit der in SEQ ID Nr. 5 dargelegten Sequenz hat.

7. Vektor gemäß Anspruch 6, wobei
(i) die Promotorsequenz einen konstitutiven Promotor umfasst, wobei die Promotorsequenz vorzugsweise einen CMV-Promotor umfasst, wobei der CMV-Promotor am meisten bevorzugt die in SEQ ID Nr. 32 dargelegte Sequenz umfasst; und/oder
(ii) die Promotorsequenz einen β-Actin-Promotor umfasst, wobei die β-Actin-Promotorsequenz vorzugsweise eine Vogel-β-Actin-Promotorsequenz umfasst, wobei die β-Actin-Promotorsequenz am meisten bevorzugt eine Säuger-β-Actin-Promotorsequenz oder die in SEQ ID Nr. 26 dargelegte Sequenz umfasst; und/oder
(iii) die Promotorsequenz einen induzierbaren Promotor umfasst.

8. Vektor gemäß Anspruch 1, der weiterhin Folgendes umfasst:
(i) ein Reporter-Gen, wobei das Reporter-Gen vorzugsweise ein lacZ-Gen ist und/oder das Reporter-Gen von Rekombinasestellen flankiert ist, wobei die Rekombinasestellen am meisten bevorzugt Stellen für die cre-Rekombinase sind; und/oder
(ii) eine Transcriptionsterminationsstelle, wobei die Transcriptionsterminationsstelle vorzugsweise 5' zur Promotorsequenz orientiert ist, wobei die Transcriptionsterminationsstelle besonders bevorzugt von Rekombinasestellen flankiert ist, wobei die Rekombinasestellen am meisten bevorzugt Stellen für die cre-Rekombinase sind.

9. Vektor gemäß Anspruch 1, wobei der lentivirale Vektor
(i) ein felines Immunschwächevirus oder ein humanes Immunschwächevirus umfasst; und/oder
(ii) wenigstens drei Monate lang stabil integriert werden kann.

10. Vektor gemäß Anspruch 1, der eine Nucleinsäuresequenz mit der in SEQ ID Nr. 2 dargelegten Sequenz, die HEX-α codiert, und eine Nucleinsäuresequenz mit der in SEQ ID Nr. 4 dargelegten Sequenz, die HEX-β codiert, umfasst.

11. Zelle, die den Vektor gemäß Anspruch 1 oder 9 umfasst.

12. Zelle gemäß Anspruch 11, die ein Neuron, eine Gliazelle, einen Fibroblasten, einen Chondrocyten, einen Osteocyten, eine Endothelzelle oder einen Hepatocyten umfasst.

13. Zusammensetzung, die den Vektor gemäß Anspruch 1 oder 9 umfasst, wobei die Zusammensetzung in pharmazeutisch annehmbarer Form oder in einer wirksamen Dosierung vorliegt, wobei die wirksame Dosierung als Dosierung bestimmt ist, die die Wirkungen der Tay-Sachs- oder der Sandhoff-Krankheit reduziert.

14. Transgenes nichthumanes Tier, bei dem der Vektor gemäß Anspruch 1 oder 9 in das Genom integriert ist oder das die Zelle gemäß Anspruch 11 aufweist.

15. Nichthumanes Tier gemäß Anspruch 14, wobei das nichthumane Tier ein Säuger ist, wobei der Säuger vorzugsweise eine Maus, ein Huftier oder ein nichthumaner Primat ist, wobei der Säuger am meisten bevorzugt eine Maus, Ratte, Kaninchen, Rind, Schaf oder Schwein ist.

16. Nichthumanes Tier gemäß Anspruch 15, wobei der Säuger eine Maus ist, wobei vorzugsweise
(i) die Maus einen HexB-Knockout umfasst; oder
(ii) die Maus einen HexA-Knockout umfasst, wobei die Maus gegebenenfalls weiterhin einen HexB-Knockout umfasst.

17. Nichthumanes Tier gemäß Anspruch 15, wobei der Säuger ein nichthumaner Primat ist.

18. In-vitro-Verfahren zum Bereitstellen eines HEX-α/HEX-β-Heterodimers in einer Zelle, umfassend das Transfizieren der Zelle mit dem Vektor gemäß Anspruch 1.

19. Verwendung des Vektors gemäß Anspruch 1 oder 9 oder der Zelle gemäß Anspruch 11 zur Herstellung eines Mittels zur Schaffung eines transgenen nichthumanen Organismus.

20. Verwendung gemäß Anspruch 19, wobei das Mittel einen lentiviralen Vektor umfasst und für das Transfizieren des Organismus während eines perinatalen Stadiums seiner Entwicklung geeignet ist.

21. Verwendung der Zusammensetzung gemäß Anspruch 13 zur Herstellung eines Medikaments zur Behandlung eines Patienten, der an der Tay-Sachs- oder der Sandhoff-Krankheit leidet.

22. Verfahren zur Herstellung des Vektors gemäß Anspruch 1, wobei das Verfahren das funktionelle Verknüpfen eines Promotorelements, eines Elements, das eine HEX-β codierende Sequenz umfasst, ein IRES-Element und ein HEX-α codierendes Element umfasst.

23. Verfahren gemäß Anspruch 22, wobei
(i) das HEX-β-Element eine Sequenz mit wenigstens 80% Identität zu SEQ ID Nr. 2 umfasst und das HEX-α-Element eine Sequenz mit wenigstens 80% Identität zu SEQ ID Nr. 4 umfasst, wobei vorzugsweise jede Veränderung in SEQ ID Nr. 1 oder SEQ ID Nr. 3 eine konservative Veränderung ist; und/oder
(ii) die Sequenz, die HEX-β codiert, unter stringenten Bedingungen mit SEQ ID Nr. 2 hybridisiert und wobei die Sequenz, die das HEX-α codiert, unter stringenten Bedingungen mit SEQ ID Nr. 4 hybridisiert.

24. Verfahren zur Herstellung der Zelle gemäß Anspruch 11, wobei das Verfahren das Verabreichen des Vektors gemäß Anspruch 1 an die Zellen umfasst.

25. Verfahren zur Herstellung eines HEX-α/HEX-β-Heterodimers, wobei das Verfahren das Exprimieren der vom Vektor gemäß Anspruch 1 umfassten Nucleinsäure umfasst.

26. Verfahren gemäß Anspruch 25, das weiterhin das Isolieren des Peptids umfasst.

## Revendications

1. Vecteur lentiviral bicistronique comprenant une séquence d'acide nucléique ayant au moins une identité de 70% avec la séquence présentée dans SEQ ID NO:2 et codant pour la sous-unité α de la β-hexosaminidase (HEX-α), une séquence d'acide nucléique ayant au moins une identité de 70% avec la séquence présentée dans SEQ ID NO:4 et codant pour la sous-unité β de la β-hexosaminidase (HEX-β), un promoteur et une séquence IRES (Integrated Ribosomal Entry Site), où la séquence codant pour la HEX-β est orientée en 5' par rapport à la séquence IRES et la séquence IRES est située en 5' de la séquence codant pour HEX-α, et où ledit vecteur lentiviral bicistronique produit un hétérodimère fonctionnel HEX-α/HEX-β.

2. Vecteur selon la revendication 1, dans lequel le promoteur est situé en 5' de la séquence codant pour la HEX-β.

3. Vecteur selon la revendication 1 ou 2, où le vecteur comprend une deuxième séquence IRES, de préférence, la deuxième séquence IRES est située en 3' des autres parties.

4. Vecteur selon l'une quelconque des revendications 1 à 3, dans lequel tout changement par rapport à SEQ ID NO:4 ou SEQ ID NO:2 est un changement conservatif dans la protéine codée, de préférence, la HEX-β a la même séquence présentée dans SEQ ID NO:3 et la HEX-α a la séquence présentée dans SEQ ID NO:1.

5. Vecteur selon l'une quelconque des revendications 1 à 3, dans lequel la séquence codant pour HEX-β s'hybride avec SEQ ID NO:2 dans des conditions stringentes et dans lequel l'élément HEX-α s'hybride avec SEQ ID NO:4 dans des conditions stringentes.

6. Vecteur selon la revendication 1 ou 4, dans lequel la séquence IRES comprend une séquence ayant une identité d'au moins 70%, 75%, 80%, 85%, 90% ou 95% avec la séquence présentée dans SEQ ID NO:5.

7. Vecteur selon la revendication 6, dans lequel
(i) la séquence promotrice comprend un promoteur constitutif, de préférence, la séquence promotrice comprend un promoteur de CMV, le plus préférablement, le promoteur de CMV comprend la séquence présentée dans SEQ ID NO:32; et/ou
(ii) la séquence promotrice comprend un promoteur de β-actine, de préférence, la séquence du promoteur de β-actine comprend une séquence de promoteur de β-actinie aviaire, le plus préférablement, la séquence de promoteur de β-actine comprend une séquence de promoteur de β-actinie de mammifères, ou la séquence présentée dans SEQ ID NO:26; et/ou
(iii) la séquence promotrice comprend un promoteur inductible.

8. Vecteur selon la revendication 1, qui comprend en outre:
(i) un gène indicateur, de préférence, le gène indicateur est un gène lacZ, et/ou le gène indicateur est flanqué par des sites de recombinase, le plus préférablement, les sites de recombinase sont pour la recombinase cre; et/ou
(ii) un site de terminaison de la transcription, de préférence, le site de terminaison de la transcription est orienté en 5' par rapport à la séquence promotrice, plus préférablement, le site de terminaison de la transcription est flanqué de sites de recombinase, le plus préférablement, les sites de recombinase sont pour la recombinase cre.

9. Vecteur selon la revendication 1, où le vecteur lentiviral
(i) comprend un virus de l'immunodéficience féline, ou un virus de l'immunodéficience humaine; et/ou
(ii) peut s'intégrer de façon stable pendant au moins trois mois.

10. Vecteur selon la revendication 1, qui comprend une séquence d'acide nucléique ayant la séquence présentée dans SEQ ID NO:2 et codant pour HEX-α, et une séquence d'acide nucléique ayant la séquence présentée dans SEQ ID NO:4 et codant pour HEX-β.

11. Cellule comprenant le vecteur selon la revendication 1 ou 9.

12. Cellule selon la revendication 11, qui comprend un neurone, une cellule gliale, un fibroblaste, un chondrocyte, un ostéocyte, une cellule endothéliale ou un hépatocyte.

13. Composition comprenant le vecteur selon la revendication 1 ou 9, où la composition est sous forme acceptable sur le plan pharmaceutique, ou est dans une posologie efficace, ladite posologie efficace étant déterminée sous forme d'une posologie qui réduit les effets de la maladie de Tay-Sachs ou de Sandoff.

14. Animal transgénique non humain ayant le vecteur selon la revendication 1 ou 9, intégré dans le génome, ou ayant la cellule selon la revendication 11.

15. Animal non humain selon la revendication 14, où l'animal non humain est un mammifère, de préférence, le mammifère est un mammifère murin, un ongulé ou un primate non humain, le plus préférablement, le mammifère est une souris, un rat, un lapin, une vache, un mouton ou un porc.

16. Animal non humain selon la revendication 15, où le mammifère est une souris, de préférence
(i) la souris comprend un knock-out de HexB; ou
(ii) la souris comprend un knock-out de HexA, facultativement, la souris comprend en outre un knock-out de HexB.

17. Animal non humain selon la revendication 15, où le mammifère est un primate non humain.

18. Procédé in vitro pour obtenir un hétérodimère HEX-α/HEX-β dans une cellule, comprenant la transfection de la cellule avec le vecteur selon la revendication 1.

19. Utilisation du vecteur selon la revendication 1 ou 9, ou de la cellule de la revendication 11 pour préparer un agent permettant d'obtenir un organisme transgénique non humain.

20. Utilisation selon la revendication 19, où l'agent comprend un vecteur lentiviral et convient pour transfecter l'organisme au cours d'un stade périnatal du développement de l'organisme.

21. Utilisation de la composition selon la revendication 13 pour préparer un médicament destiné au traitément d'un sujet ayant la maladie de Tay-Sachs et/ou la maladie de Sandoff.

22. Procédé de fabrication d'un vecteur selon la revendication 1, ledit procédé comprenant la liaison d'une manière opérante d'un élément promoteur, d'un élément comprenant une séquence codant pour HEX-β, d'un élément IRES et d'un élément codant pour HEX-α.

23. Procédé selon la revendication 22, dans lequel
(i) l'élément HEX-β comprend une séquence ayant une identité d'au moins 80% avec SEQ ID NO:2 et l'élément HEX-α comprend une séquence ayant une identité d'au moins 80% avec SEQ ID NO:4, de préférence, tout changement dans SEQ ID NO:1 ou SEQ ID NO:3 est un changement conservatif; et/ou
(ii) la séquence codant pour HEX-β s'hybride avec SEQ ID NO:2 dans des conditions stringentes et dans lequel la séquence codant pour HEX-α s'hybride avec SEQ ID NO:4 dans des conditions stringentes.

24. Procédé de production de la cellule selon la revendication 11, ledit procédé comprenant l'administration du vecteur selon la revendication 1 à la cellule.

25. Procédé de production d'un hétérodimère HEX-α/HEX-β, ledit procédé comprenant l'expression de l'acide nucléique intégré dans le vecteur de la revendication 1.

26. Procédé selon la revendication 25, comprenant en outre l'isolement du peptide.


### SEQUENCE LISTING

<110> University of Rochester Kyrkanides, Stephanos
<120> VECTORS HAVING BOTH ISOFORMS OF BETA-HEXOSAMINIDASE
<130> 21108.0018P1
<150> 60/377,503
   <151> 2002-05-02
<160> 41
<170> FastSEQ for Windows Version 4.0
<210> 1
   <211> 409
   <212> PRT
   <213> Artificial Sequence
<220>
   <223> Description of Artificial Sequence:/Note = Synthetic Construct
<400> 1
   Met Met Thr Ser Val Tyr Ser Ser Leu Arg Leu Ser Gly Glu Leu Ser
    1               5                  10                  15
   Glu Val Trp Arg Leu Leu Ala Ser Leu Phe Gly Asn Leu Leu Arg Ala
               20                  25                  30
   Gln Phe Phe Ile Asn Lys Thr Glu Ile Glu Asp Phe Pro Arg Phe Pro
           35                  40                  45
   His Arg Gly Leu Leu Leu Asp Thr Ser Arg His Tyr Leu Pro Leu Ser
       50                  55                  60
   Ser Ile Leu Asp Thr Leu Asp Val Met Ala Tyr Asn Lys Leu Asn Val
   65                  70                  75                  80
   Phe His Trp His Leu Val Asp Asp Pro Ser Phe Pro Tyr Glu Ser Phe
                   85                  90                  95
   Thr Phe Pro Glu Leu Met Arg Lys Gly Ser Tyr Asn Pro Val Thr His
               100                 105                 110
   Ile Tyr Thr Ala Gln Asp Val Lys Glu Val Ile Glu Tyr Ala Arg Leu
           115                 120                 125
   Arg Gly Ile Arg Val Leu Ala Glu Phe Asp Thr Pro Gly His Thr Leu
       130                 135                 140
   Ser Trp Gly Pro Gly Ile Pro Gly Leu Leu Thr Pro Cys Tyr Ser Gly
   145                 150                 155                 160
   Ser Glu Pro Ser Gly Thr Phe Gly Pro Val Asn Pro Ser Leu Asn Asn
                   165                 170                 175
   Thr Tyr Glu Phe Met Ser Thr Phe Phe Leu Glu Val Ser Ser Val Phe
               180                 185                 190
   Pro Asp Phe Tyr Leu His Leu Gly Gly Asp Glu Val Asp Phe Thr Cys
           195                 200                 205
   Trp Lys Ser Asn Pro Glu Ile Gln Asp Phe Met Arg Lys Lys Gly Phe
       210                 215                 220
   Gly Glu Asp Phe Lys Gln Leu Glu Ser Phe Tyr Ile Gln Thr Leu Leu
   225                 230                 235                 240
   Asp Ile Val Ser Ser Tyr Gly Lys Gly Tyr Val Val Trp Gln Glu Val
                   245                 250                 255
   Phe Asp Asn Lys Val Lys Ile Gln Pro Asp Thr Ile Ile Gln Val Trp
               260                 265                 270
   Arg Glu Asp Ile Pro Val Asn Tyr Met Lys Glu Leu Glu Leu Val Thr
           275                 280                 285
   Lys Ala Gly Phe Arg Ala Leu Leu Ser Ala Pro Trp Tyr Leu Asn Arg
       290                 295                 300
   Ile Ser Tyr Gly Pro Asp Trp Lys Asp Phe Tyr Ile Val Glu Pro Leu
   305                 310                 315                 320
   Ala Phe Glu Gly Thr Pro Glu Gln Lys Ala Leu Val Ile Gly Gly Glu
                   325                 330                 335
   Ala Cys Met Trp Gly Glu Tyr Val Asp Asn Thr Asn Leu Val Pro Arg
               340                 345                 350
   Leu Trp Pro Arg Ala Gly Ala Val Ala Glu Arg Leu Trp Ser Asn Lys
           355                 360                 365
   Leu Thr Ser Asp Leu Thr Phe Ala Tyr Glu Arg Leu Ser His Phe Arg
       370                 375                 380
   Cys Glu Leu Leu Arg Arg Gly Val Gln Ala Gln Pro Leu Asn Val Gly
   385                 390                 395                 400
   Phe Cys Glu Gln Glu Phe Glu Gln Thr
                   405
<210> 2
   <211> 2256
   <212> DNA
   <213> Artificial Sequence
<220>
   <223> Description of Artificial Sequence:/Note = Synthetic Construct
<400> 2
   cctccgagag gggagaccag cgggccatga caagctccag gctttggttt tcgctgctgc      60
   tggcggcagc gttcgcagga cgggcgacgg ccctctggcc ctggcctcag aacttccaaa     120
   cctccgacca gcgctacgtc ctttacccga acaactttca attccagtac gatgtcagct     180
   cggccgcgca gcccggctgc tcagtcctcg acgaggcctt ccagcgctat cgtgacctgc     240
   ttttcggttc cgggtcttgg ccccgtcctt acctcacagg gaaacggcat acactggaga     300
   agaatgtgtt ggttgtctct gtagtcacac ctggatgtaa ccagcttcct actttggagt     360
   cagtggagaa ttataccctg accataaatg atgaccagtg tttactcctc tctgagactg     420
   tctggggagc tctccgaggt ctggagactt ttagccagct tgtttggaaa tctgctgagg     480
   gcacagttct ttatcaacaa gactgagatt gaggactttc cccgctttcc tcaccggggc     540
   ttgctgttgg atacatctcg ccattacctg ccactctcta gcatcctgga cactctggat     600
   gtcatggcgt acaataaatt gaacgtgttc cactggcatc tggtagatga tccttccttc     660
   ccatatgaga gcttcacttt tccagagctc atgagaaagg ggtcctacaa ccctgtcacc     720
   cacatctaca cagcacagga tgtgaaggag gtcattgaat acgcacggct ccggggtatc     780
   cgtgtgcttg cagagtttga cactcctggc cacactttgt cctggggacc aggtatccct     840
   ggattactga ctccttgcta ctctgggtct gagccctctg gcacctttgg accagtgaat     900
   cccagtctca ataataccta tgagttcatg agcacattct tcttagaagt cagctctgtc     960
   ttcccagatt tttatcttca tcttggagga gatgaggttg atttcacctg ctggaagtcc    1020
   aacccagaga tccaggactt tatgaggaag aaaggcttcg gtgaggactt caagcagctg    1080
   gagtccttct acatccagac gctgctggac atcgtctctt cttatggcaa gggctatgtg    1140
   gtgtggcagg aggtgtttga taataaagta aagattcagc cagacacaat catacaggtg    1200
   tggcgagagg atattccagt gaactatatg aaggagctgg aactggtcac caaggccggc    1260
   ttccgggccc ttctctctgc cccctggtac ctgaaccgta tatcctatgg ccctgactgg    1320
   aaggatttct acatagtgga acccctggca tttgaaggta cccctgagca gaaggctctg    1380
   gtgattggtg gagaggcttg tatgtgggga gaatatgtgg acaacacaaa cctggtcccc    1440
   aggctctggc ccagagcagg ggctgttgcc gaaaggctgt ggagcaacaa gttgacatct    1500
   gacctgacat ttgcctatga acgtttgtca cacttccgct gtgaattgct gaggcgaggt    1560
   gtccaggccc aacccctcaa tgtaggcttc tgtgagcagg agtttgaaca gacctgagcc    1620
   ccaggcaccg aggagggtgc tggctgtagg tgaatggtag tggagccagg cttccactgc    1680
   atcctggcca ggggacggag ccccttgcct tcgtgcccct tgcctgcgtg cccctgtgct    1740
   tggagagaaa ggggccggtg ctggcgctcg cattcaataa agagtaatgt ggcatttttc     1800
   tataataaac atggattacc tgtgtttaaa aaaaaaagtg tgaatggcgt tagggtaagg    1860
   gcacagccag gctggagtca gtgtctgccc ctgaggtctt ttaagttgag ggctgggaat    1920
   gaaacctata gcctttgtgc tgttctgcct tgcctgtgag ctatgtcact cccctcccac    1980
   tcctgaccat attccagaca cctgccctaa tcctcagcct gctcacttca cttctgcatt    2040
   atatctccaa ggcgttggta tatggaaaaa gatgtagggg cttggaggtg ttctggacag    2100
   tggggagggc tccagaccca acctggtcac agaagagcct ctcccccatg catactcatc    2160
   cacctccctc ccctagagct attctccttt gggtttcttg ctgcttcaat tttatacaac    2220
   cattatttaa atattattaa acacatattg ttctct                              2256
<210> 3
   <211> 544
   <212> PRT
   <213> Artificial Sequence
<220>
   <223> Description of Artificial Sequence:/Note = Synthetic Construct
<400> 3
   Met Leu Leu Ala Leu Leu Leu Ala Thr Leu Leu Ala Ala Met Leu Ala
    1               5                  10                  15
   Leu Leu Thr Gln Val Ala Leu Val Val Gln Val Ala Glu Ala Ala Arg
               20                  25                  30
   Ala Pro Ser Val Ser Ala Lys Pro Gly Pro Ala Leu Trp Pro Leu Pro
           35                  40                  45
   Leu Leu Val Lys Met Thr Pro Asn Leu Leu His Leu Ala Pro Glu Asn
       50                  55                  60
   Phe Tyr Ile Ser His Ser Pro Asn Ser Thr Ala Gly Pro Ser Cys Thr
   65                  70                  75                  80
   Leu Leu Glu Glu Ala Phe Arg Arg Tyr His Gly Tyr Ile Phe Gly Phe
                   85                  90                  95
   Tyr Lys Trp His His Glu Pro Ala Glu Phe Gln Ala Lys Thr Gln Val
               100                 105                 110
   Gln Gln Leu Leu Val Ser Ile Thr Leu Gln Ser Glu Cys Asp Ala Phe
           115                 120                 125
   Pro Asn Ile Ser Ser Asp Glu Ser Tyr Thr Leu Leu Val Lys Glu Pro
       130                 135                 140
   Val Ala Val Leu Lys Ala Asn Arg Val Trp Gly Ala Leu Arg Gly Leu
   145                 150                 155                 160
   Glu Thr Phe Ser Gln Leu Val Tyr Gln Asp Ser Tyr Gly Thr Phe Thr
                   165                 170                 175
   Ile Asn Glu Ser Thr Ile Ile Asp Ser Pro Arg Phe Ser His Arg Gly
               180                 185                 190
   Ile Leu Ile Asp Thr Ser Arg His Tyr Leu Pro Val Lys Ile Ile Leu
           195                 200                 205
   Lys Thr Leu Asp Ala Met Ala Phe Asn Lys Phe Asn Val Leu His Trp
       210                 215                 220
   His Ile Val Asp Asp Gln Ser Phe Pro Tyr Gln Ser Ile Thr Phe Pro
   225                 230                 235                 240
   Glu Leu Ser Asn Lys Gly Ser Tyr Ser Leu Ser His Val Tyr Thr Pro
                   245                 250                 255
   Asn Asp Val Arg Met Val Ile Glu Tyr Ala Arg Leu Arg Gly Ile Arg
               260                 265                 270
   Val Leu Pro Glu Phe Asp Thr Pro Gly His Thr Leu Ser Trp Gly Lys
           275                 280                 285
   Gly Gln Lys Asp Leu Leu Thr Pro Cys Tyr Ser Arg Gln Asn Lys Leu
       290                 295                 300
   Asp Ser Phe Gly Pro Ile Asn Pro Thr Leu Asn Thr Thr Tyr Ser Phe
   305                 310                 315                 320
   Leu Thr Thr Phe Phe Lys Glu Ile Ser Glu Val Phe Pro Asp Gln Phe
                   325                 330                 335
   Ile His Leu Gly Gly Asp Glu Val Glu Phe Lys Cys Trp Glu Ser Asn
               340                 345                 350
   Pro Lys Ile Gln Asp Phe Met Arg Gln Lys Gly Phe Gly Thr Asp Phe
           355                 360                 365
   Lys Lys Leu Glu Ser Phe Tyr Ile Gln Lys Val Leu Asp Ile Ile Ala
       370                 375                 380
   Thr Ile Asn Lys Gly Ser Ile Val Trp Gln Glu Val Phe Asp Asp Lys
   385                 390                 395                 400
   Ala Lys Leu Ala Pro Gly Thr Ile Val Glu Val Trp Lys Asp Ser Ala
                   405                 410                 415
   Tyr Pro Glu Glu Leu Ser Arg Val Thr Ala Ser Gly Phe Pro Val Ile
               420                 425                 430
   Leu Ser Ala Pro Trp Tyr Leu Asp Leu Ile Ser Tyr Gly Gln Asp Trp
           435                 440                 445
   Arg Lys Tyr Tyr Lys Val Glu Pro Leu Asp Phe Gly Gly Thr Gln Lys
       450                 455                 460
   Gln Lys Gln Leu Phe Ile Gly Gly Glu Ala Cys Leu Trp Gly Glu Tyr
   465                 470                 475                 480
   Val Asp Ala Thr Asn Leu Thr Pro Arg Leu Trp Pro Arg Ala Ser Ala
                   485                 490                 495
   Val Gly Glu Arg Leu Trp Ser Ser Lys Asp Val Arg Asp Met Asp Asp
               500                 505                 510
   Ala Tyr Asp Arg Leu Thr Arg His Arg Cys Arg Met Val Glu Arg Gly
           515                 520                 525
   Ile Ala Ala Gln Pro Leu Tyr Ala Gly Tyr Cys Asn His Glu Asn Met
       530                 535                 540
<210> 4
   <211> 1635
   <212> DNA
   <213> Artificial Sequence
<220>
   <223> Description of Artificial Sequence:/Note = Synthetic Construct
<400> 4
<210> 5
   <211> 581
   <212> DNA
   <213> Artificial Sequence
<220>
   <223> Description of Artificial Sequence:/Note = Synthetic Construct
<400> 5
   aattccgccc ctctccctcc ccccccccta acgttactgg ccgaagccgc ttggaataag      60
   gccggtgtgc gtttgtctat atgtgatttt ccaccatatt gccgtctttt ggcaatgtga     120
   gggcccggaa acctggccct gtcttcttga cgagcattcc taggggtctt tcccctctcg     180
   ccaaaggaat gcaaggtctg ttgaatgtcg tgaaggaagc agttcctctg gaagcttctt     240
   gaagacaaac aacgtctgta gcgacccttt gcaggcagcg gaacccccca cctggcgaca     300
   ggtgcctctg cggccaaaag ccacgtgtat aagatacacc tgcaaaggcg gcacaacccc     360
   agtgccacgt tgtgagttgg atagttgtgg aaagagtcaa atggctctcc tcaagcgtat     420
   tcaacaaggg gctgaaggat gcccagaagg taccccattg tatgggatct gatctggggc     480
   ctcggtgcac atgctttaca tgtgtttagt cgaggttaaa aaaacgtcta ggccccccga     540
   accacgggga cgtggttttc ctttgaaaaa cacgatgata a                         581
<210> 6
   <211> 528
   <212> PRT
   <213> Artificial Sequence
<220>
   <223> Description of Artificial Sequence:/Note = Synthetic Construct
<400> 6
   Met Ala Gly Cys Arg Leu Trp Val Ser Leu Leu Leu Ala Ala Ala Leu
    1               5                  10                  15
   Ala Cys Leu Ala Thr Ala Leu Trp Pro Trp Pro Gln Tyr Ile Gln Thr
               20                  25                  30
   Tyr His Arg Arg Tyr Thr Leu Tyr Pro Asn Asn Phe Gln Phe Arg Tyr
           35                  40                  45
   His Val Ser Ser Ala Ala Gln Gly Gly Cys Val Val Leu Asp Glu Ala
       50                  55                  60
   Phe Arg Arg Tyr Arg Asn Leu Leu Phe Gly Ser Gly Ser Trp Pro Arg
   65                  70                  75                  80
   Pro Ser Phe Ser Asn Lys Gln Gln Thr Leu Gly Lys Asn Ile Leu Val
                   85                  90                  95
   Val Ser Val Val Thr Ala Glu Cys Asn Glu Phe Pro Asn Leu Glu Ser
               100                 105                 110
   Val Glu Asn Tyr Thr Leu Thr Ile Asn Asp Asp Gln Cys Leu Leu Ala
           115                 120                 125
   Ser Glu Thr Val Trp Gly Ala Leu Arg Gly Leu Glu Thr Phe Ser Gln
       130                 135                 140
   Leu Val Trp Lys Ser Ala Glu Gly Thr Phe Phe Ile Asn Lys Thr Lys
   145                 150                 155                 160
   Ile Lys Asp Phe Pro Arg Phe Pro His Arg Gly Val Leu Leu Asp Thr
                   165                 170                 175
   Ser Arg His Tyr Leu Pro Leu Ser Ser Ile Leu Asp Thr Leu Asp Val
               180                 185                 190
   Met Ala Tyr Asn Lys Phe Asn Val Phe His Trp His Leu Val Asp Asp
           195                 200                 205
   Ser Ser Phe Pro Tyr Glu Ser Phe Thr Phe Pro Glu Leu Thr Arg Lys
       210                 215                 220
   Gly Ser Phe Asn Pro Val Thr His Ile Tyr Thr Ala Gln Asp Val Lys
   225                 230                 235                 240
   Glu Val Ile Glu Tyr Ala Arg Leu Arg Gly Ile Arg Val Leu Ala Glu
                   245                 250                 255
   Phe Asp Thr Pro Gly His Thr Leu Ser Trp Gly Pro Gly Ala Pro Gly
               260                 265                 270
   Leu Leu Thr Pro Cys Tyr Ser Gly Ser His Leu Ser Gly Thr Phe Gly
           275                 280                 285
   Pro Val Asn Pro Ser Leu Asn Ser Thr Tyr Asp Phe Met Ser Thr Leu
       290                 295                 300
   Phe Leu Glu Ile Ser Ser Val Phe Pro Asp Phe Tyr Leu His Leu Gly
   305                 310                 315                 320
   Gly Asp Glu Val Asp Phe Thr Cys Trp Lys Ser Asn Pro Asn Ile Gln
                   325                 330                 335
   Ala Phe Met Lys Lys Lys Gly Phe Thr Asp Phe Lys Gln Leu Glu Ser
               340                 345                 350
   Phe Tyr Ile Gln Thr Leu Leu Asp Ile Val Ser Asp Tyr Asp Lys Gly
           355                 360                 365
   Tyr Val Val Trp Gln Glu Val Phe Asp Asn Lys Val Lys Val Arg Pro
       370                 375                 380
   Asp Thr Ile Ile Gln Val Trp Arg Glu Glu Met Pro Val Glu Tyr Met
   385                 390                 395                 400
   Leu Glu Met Gln Asp Ile Thr Arg Ala Gly Phe Arg Ala Leu Leu Ser
                   405                 410                 415
   Ala Pro Trp Tyr Leu Asn Arg Val Lys Tyr Gly Pro Asp Trp Lys Asp
               420                 425                 430
   Met Tyr Lys Val Glu Pro Leu Ala Phe His Gly Thr Pro Glu Gln Lys
           435                 440                 445
   Ala Leu Val Ile Gly Gly Glu Ala Cys Met Trp Gly Glu Tyr Val Asp
       450                 455                 460
   Ser Thr Asn Leu Val Pro Arg Leu Trp Pro Arg Ala Gly Ala Val Ala
   465                 470                 475                 480
   Glu Arg Leu Trp Ser Ser Asn Leu Thr Thr Asn Ile Asp Phe Ala Phe
                   485                 490                 495
   Lys Arg Leu Ser His Phe Arg Cys Glu Leu Val Arg Arg Gly Ile Gln
               500                 505                 510
   Ala Gln Pro Ile Ser Val Gly Tyr Cys Glu Gln Glu Phe Glu Gln Thr
           515                 520                 525
<210> 7
   <211> 1960
   <212> DNA
   <213> Artificial Sequence
<220>
   <223> Description of Artificial Sequence:/Note = Synthetic Construct
<400> 7
   ctgcagaatc ctttgcttac ggatctctga gatcgagccg ccttgcttcc ctcccgttca      60
   cgtgaccctc cgattgtcac gcgggcgtcc gctcagctga ccggggctca cgtgggctca     120
   gcctgctggc cggggagctg gccggtgggc atggccggct gcaggctctg ggtttcgctg     180
   ctgctggcgg cggcgttggc ttgcttggcc acggcactgt ggccgtggcc ccagtacatc     240
   caaacctacc accggcgcta caccctgtac cccaacaact tccagttccg gtaccatgtc     300
   agttcggccg cgcagggcgg ctgcgtcgtc ctcgacgagg cctttcgacg ctaccgtaac     360
   ctgctcttcg gttccggctc ttggccccga cccagcttct caaataaaca gcaaacgttg     420
   gggaagaaca ttctggtggt ctccgtcgtc acagctgaat gtaatgaatt tcctaatttg     480
   gagtcggtag aaaattacac cctaaccatt aatgatgacc agtgtttact cgcctctgag     540
   actgtctggg gcgctctccg aggtctggag actttcagtc agcttgtttg gaaatcagct     600
   gagggcacgt tctttatcaa caagacaaag attaaagact ttcctcgatt ccctcaccgg     660
   ggcgtactgc tggatacatc tcgccattac ctgccattgt ctagcatcct ggatacactg     720
   gatgtcatgg catacaataa attcaacgtg ttccactggc acttggtgga cgactcttcc     780
   ttcccatatg agagcttcac tttcccagag ctcaccagaa aggggtcctt caaccctgtc     840
   actcacatct acacagcaca ggatgtgaag gaggtcattg aatacgcaag gcttcggggt     900
   atccgtgtgc tggcagaatt tgacactcct ggccacactt tgtcctgggg gccaggtgcc     960
   cctgggttat taacaccttg ctactctggg tctcatctct ctggcacatt tggaccggtg    1020
   aaccccagtc tcaacagcac ctatgacttc atgagcacac tcttcctgga gatcagctca    1080
   gtcttcccgg acttttatct ccacctggga ggggatgaag tcgacttcac ctgctggaag    1140
   tccaacccca acatccaggc cttcatgaag aaaaagggct ttactgactt caagcagctg    1200
   gagtccttct acatccagac gctgctggac atcgtctctg attatgacaa gggctatgtg    1260
   gtgtggcagg aggtatttga taataaagtg aaggttcggc cagatacaat catacaggtg    1320
   tggcgggaag aaatgccagt agagtacatg ttggagatgc aagatatcac cagggctggc    1380
   ttccgggccc tgctgtctgc tccctggtac ctgaaccgtg taaagtatgg ccctgactgg    1440
   aaggacatgt acaaagtgga gcccctggca tttcatggta cgcctgaaca gaaggctctg    1500
   gtcattggag gggaggcctg tatgtgggga gagtatgtgg acagcaccaa cctggtcccc    1560
   agactctggc ccagagcggg tgccgtcgct gagagactgt ggagcagtaa cctgacaact    1620
   aatatagact ttgcctttaa acgtttgtcg catttccgtt gtgagctggt gaggagagga    1680
   atccaggccc agcccatcag tgtaggctac tgtgagcagg agtttgagca gacttgagcc    1740
   accagcgctg aacacccagg aggttgctgt cctttgagtc agctgcgctg agcacccagg    1800
   agggtgctgg ccttaagaga gcaggtcccg gggcagggct aatctttcac tgcctcccgg    1860
   ccaggggaga gcaccccttg cccgtgtgcc cctgtgacta cagagaagga ggctggtgct    1920
   ggcactggtg ttcaataaag atctatgtgg cattttctct                          1960
<210> 8
   <211> 12745
   <212> DNA
   <213> Artificial Sequence
<220>
   <223> Description of Artificial Sequence:/Note = Synthetic Construct
<400> 8
   atgcggtttt ggcagtacat caatgggcgt ggatagcggt ttgactcacg gggatttcca     60
   agtctccacc ccattgacgt caatgggagt ttgttttggc accaaaatca acgggacttt    120
   ccaaaatgtc gtaacaactc cgccccattg acgcaaatgg gcggtaggcg tgtacggtgg    180
   gaggtctata taagcagagc tctgtgaaac ttcgaggagt ctctttgttg aggacttttg    240
   agttctccct tgaggctccc acagatacaa taaatatttg agattgaacc ctgtcgagta    300
   tctgtgtaat cttttttacc tgtgaggtct cggaatccgg gccgagaact tcgcagttgg    360
   cgcccgaaca gggacttgat tgagagtgat tgaggaagtg aagctagagc aatagaaagc    420
   tgttaagcag aactcctgct gacctaaata gggaagcagt agcagacgct gctaacagtg    480
   agtatctcta gtgaagcgga ctcgagctca taatcaagtc attgtttaaa ggcccagata    540
   aattacatct ggtgactctt cgcggacctt caagccagga gattcgccga gggacagtca    600
   acaaggtagg agagattcta cagcaacatg gggaatggac aggggcgaga ttggaaaatg    660
   gccattaaga gatgtagtaa tgttgctgta ggagtagggg ggaagagtaa aaaatttgga    720
   gaagggaatt tcagatgggc cattagaatg gctaatgtat ctacaggacg agaacctggt    780
   gatataccag agactttaga tcaactaagg ttggttattt gcgatttaca agaaagaaga    840
   gaaaaatttg gatctagcaa agaaattgat atggcaattg tgacattaaa agtctttgcg    900
   gtagcaggac ttttaaatat gacgggtgtc tactgctgct gcagctgaaa atatgtattc    960
   tcaaatggga ttagacacta ggccatctat gaaagaagca ggtggaaaag aggaaggccc   1020
   tccacaggca tatcctattc aaacagtaaa tggagtacca caatatgtag cacttgaccc   1080
   aaaaatggtg tccattttta tggaaaaggc aagagaagga ctaggaggtg aggaagttca   1140
   actatggttt actgccttct ctgcaaattt aacacctact gacatggcca cattaataat   1200
   ggccgcacca gggtgcgctg cagataaaga aatattggat gaaagcttaa agcaactgac   1260
   agcagaatat gatcgcacac atccccctga tgctcccaga ccattaccct attttactgc   1320
   agcagaaatt atgggtatag gattaactca agaacaacaa gcagaagcaa gatttgcacc   1380
   agctaggatg cagtgtagag catggtatct cgaggcatta ggaaaattgg ctgccataaa   1440
   agctaagtct cctcgagctg tgcagttaag acaaggagct aaggaagatt attcatcctt   1500
   tatagacaga ttgtttgccc aaatagatca agaacaaaat acagctgaag ttaagttata   1560
   tttaaaacag tcattgagca tagctaatgc taatgcagac tgtaaaaagg caatgagcca   1620
   ccttaagcca gaaagtaccc tagaagaaaa gttgagagct tgtcaagaaa taggctcacc   1680
   aggatataaa atgcaactct tggcagaagc tcttacaaaa gttcaagtag tgcaatcaaa   1740
   aggatcagga ccagtgtgtt ttaattgtaa aaaaccagga catctagcaa gacaatgtag   1800
   agaagtgaaa aaatgtaata aatgtggaaa acctggtcat gtagctgcca aatgttggca   1860
   aggaaataga aagaattgta caagggaaga aagggataca acaattacaa aagtgggaag   1920
   attgggtagg atggatagga aatattccac aatatttaaa gggactattg ggaggtatct   1980
   tgggaatagg attaggagtg ttattattga ttttatgttt acctacattg gttgattgta   2040
   taagaaattg tatccacaag atactaggat acacagtaat tgcaatgcct gaagtagaag   2100
   gagaagaaat acaaccacaa atggaattga ggagaaatgg taggcaatgt ggcatgtctg   2160
   aaaaagagga ggaatgatga agtatctcag acttatttta taagggagat actgtgctga   2220
   gttcttccct ttgaggaagg tatgtcatat gaatccattt cgaatcaaat caaactaata   2280
   aagtatgtat tgtaaggtaa aaggaaaaga caaagaagaa gaagaaagaa gaaagccttc   2340
   agtacattta tattggctca tgtccaatat gaccgccatg ttgacattga ttattgacta   2400
   gttattaata gtaatcaatt acggggtcat tagttcatag cccatatatg gagttccgcg 2460
   ttacataact tacggtaatt ggcccgcctg ctgaccgccc aacgaccccc gcccattgac 2520
   gtcaataatg acgtatgttc ccatagtaac gccaataggg actttccatt gacgtcaatg 2580
   ggtggagtat ttacggtaaa ctgcccactt ggcagtacat caagtgtatc atatgccaag 2640
   tccggccccc tattgacgtc aatgacggta aatggcccgc ctggcattat gcccagtaca 2700
   tgaccttacg ggactttggt acttggcagt acatctacgt attagtcatc gctattacca 2760
   tggtgatgcg gttttggcag tacaccaatg ggcgtggata gcggtttgac tcacggggat 2820
   ttccaagtct ccaccccatt gacgtcaatg ggagtttgtt ttggcaccaa aatcaacggg 2880
   actttccaaa atgtcgtaat aaccccgccc cgttgacgca aatgggcggt aggcgtgtac 2940
   ggtgggaggt ctatataagc agagctcgtt tagtgaaccg tcagatcgcc tggagacgcc 3000
   atccacgctg ttttgacctc catagaagac accgggaccg atccagcctc cgcggccggg 3060
   aacggtgcat tggaacgcgg attccccgtg ccaagagtga cgtaagtacc gcctatagac 3120
   tctataggca cacccctttg gctcttatgc atgctatact gtttttggct tggggcctat 3180
   acacccccgc tccttatgct ataggtgatg gtatagctta gcctataggt gtgggttatt 3240
   gaccattatt gaccactccc ctattggtga cgatactttc cattactaat ccataacatg 3300
   gctctttgcc acaactatct ctattggcta tatgccaata ctctgtcctt cagagactga 3360
   cacggactct gtatttttac aggatggggt cccatttatt atttacaaat tcacatatac 3420
   aacaacgccg tcccccgtgc ccgcagtttt tattaaacat agcgtgggat ctccacgcga 3480
   atctcgggta cgtgttccgg acatgggctc ttctccggta gcggcggagc ttccacatcc 3540
   gagccctggt cccatgcctc cagcggctca tggtcgctcg gcagctcctt gctcctaaca 3600
   gtggaggcca gacttaggca cagcacaatg cccaccacca ccagtgtgcc gcacaaggcc 3660
   gtggcggtag ggtatgtgtc tgaaaatgag ctcggagatt gggctcgcac cgtgacgcag 3720
   atggaagact taaggcagcg gcagaagaag atgcaggcag ctgagttgtt gtattctgat 3780
   aagagtcaga ggtaactccc gttgcggttc tgttaacggt ggagggcagt gtagtctgag 3840
   cagtactcgt tgctgccgcg cgcgccacca gacataatag ctgacagact aacagactgt 3900
   tcctttccat gggtcttttc tgcagtcacc gtcgtcgaag cttatgacca tgattacgga 3960
   ttcactggcc gtcgttttac aacgtcgtga ctgggaaaac cctggcgtta cccaacttaa 4020
   tcgccttgca gcacatcccc ctttcgccag ctggcgtaat agcgaagagg cccgcaccga 4080
   tcgcccttcc caacagttgc gcagcctgaa tggcgaatgg cgctttgcct ggtttccggc 4140
   accagaagcg gtgccggaaa gctggctgga gtgcgatctt cctgaggccg atactgtcgt 4200
   cgtcccctca aactggcaga tgcacggtta cgatgcgccc atctacacca acgtaaccta 4260
   tcccattacg gtcaatccgc cgtttgttcc cacggagaat ccgacgggtt gttactcgct 4320
   cacatttaat gttgatgaaa gctggctaca ggaaggccag acgcgaatta tttttgatgg 4380
   cgttaactcg gcgtttcatc tgtggtgcaa cgggcgctgg gtcggttacg gccaggacag 4440
   tcgtttgccg tctgaatttg acctgagcgc atttttacgc gccggagaaa accgcctcgc 4500
   ggtgatggtg ctgcgttgga gtgacggcag ttatctggaa gatcaggata tgtggcggat 4560
   gagcggcatt ttccgtgacg tctcgttgct gcataaaccg actacacaaa tcagcgattt 4620
   ccatgttgcc actcgcttta atgatgattt cagccgcgct gtactggagg ctgaagttca 4680
   gatgtgcggc gagttgcgtg actacctacg ggtaacagtt tctttatggc agggtgaaac 4740
   gcaggtcgcc agcggcaccg cgcctttcgg cggtgaaatt atcgatgagc gtggtggtta 4800
   tgccgatcgc gtcacactac gtctgaacgt cgaaaacccg aaactgtgga gcgccgaaat 4860
   cccgaatctc tatcgtgcgg tggttgaact gcacaccgcc gacggcacgc tgattgaagc 4920
   agaagcctgc gatgtcggtt tccgcgaggt gcggattgaa aatggtctgc tgctgctgaa 4980
   cggcaagccg ttgctgattc gaggcgttaa ccgtcacgag catcatcctc tgcatggtca 5040
   ggtcatggat gagcagacga tggtgcagga tatcctgctg atgaagcaga acaactttaa 5100
   cgccgtgcgc tgttcgcatt atccgaacca tccgctgtgg tacacgctgt gcgaccgcta 5160
   cggcctgtat gtggtggatg aagccaatat tgaaacccac ggcatggtgc caatgaatcg 5220
   tctgaccgat gatccgcgct ggctaccggc gatgagcgaa cgcgtaacgc gaatggtgca 5280
   gcgcgatcgt aatcacccga gtgtgatcat ctggtcgctg gggaatgaat caggccacgg 5340
   cgctaatcac gacgcgctgt atcgctggat caaatctgtc gatccttccc gcccggtgca 5400
   gtatgaaggc ggcggagccg acaccacggc caccgatatt atttgcccga tgtacgcgcg 5460
   cgtggatgaa gaccagccct tcccggctgt gccgaaatgg tccatcaaaa aatggctttc 5520
   gctacctgga gagacgcgcc cgctgatcct ttgcgaatac gcccacgcga tgggtaacag 5580
   tcttggcggt ttcgctaaat actggcaggc gtttcgtcag tatccccgtt tacagggcgg 5640
   cttcgtctgg gactgggtgg atcagtcgct gattaaatat gatgaaaacg gcaacccgtg 5700
   gtcggcttac ggcggtgatt ttggcgatac gccgaacgat cgccagttct gtatgaacgg 5760
   tctggtcttt gccgaccgca cgccgcatcc agcgctgacg gaagcaaaac accagcagca 5820
   gtttttccag ttccgtttat ccgggcaaac catcgaagtg accagcgaat acctgttccg 5880
   tcatagcgat aacgagctcc tgcactggat ggtggcgctg gatggtaagc cgctggcaag 5940
   cggtgaagtg cctctggatg tcgctccaca aggtaaacag ttgattgaac tgcctgaact 6000
   accgcagccg gagagcgccg ggcaactctg gctcacagta cgcgtagtgc aaccgaacgc 6060
   gaccgcatgg tcagaagccg ggcacatcag cgcctggcag cagtggcgtc tggcggaaaa 6120
   cctcagtgtg acgctccccg ccgcgtccca cgccatcccg catctgacca ccagcgaaat 6180
   ggatttttgc atcgagctgg gtaataagcg ttggcaattt aaccgccagt caggctttct 6240
   ttcacagatg tggattggcg ataaaaaaca actgctgacg ccgctgcgcg atcagttcac 6300
   ccgtgcaccg ctggataacg acattggcgt aagtgaagcg acccgcattg accctaacgc 6360
   ctgggtcgaa cgctggaagg cggcgggcca ttaccaggcc gaagcagcgt tgttgcagtg 6420
   cacggcagat acacttgctg atgcggtgct gattacgacc gctcacgcgt ggcagcatca 6480
   ggggaaaacc ttatttatca gccggaaaac ctaccggatt gatggtagtg gtcaaatggc 6540
   gattaccgtt gatgttgaag tggcgagcga tacaccgcat ccggcgcgga ttggcctgaa 6600
   ctgccagctg gcgcaggtag cagagcgggt aaactggctc ggattagggc cgcaagaaaa 6660
   ctatcccgac cgccttactg ccgcctgttt tgaccgctgg gatctgccat tgtcagacat 6720
   gtataccccg tacgtcttcc cgagcgaaaa cggtctgcgc tgcgggacgc gcgaattgaa 6780
   ttatggccca caccagtggc gcggcgactt ccagttcaac atcagccgct acagtcaaca 6840
   gcaactgatg gaaaccagcc atcgccatct gctgcacgcg gaagaaggca catggctgaa 6900
   tatcgacggt ttccatatgg ggattggtgg cgacgactcc tggagcccgt cagtatcggc 6960
   ggaattccag ctgagcgccg gtcgctacca ttaccagttg gtctggtgtc aaaaataact 7020
   cgatcgacca gagctgagat cctacaggag tccagggctg gagagaaaac ctctgaagag 7080
   gatgatgaca gagttagaag atcgcttcag gaagctattt ggcacgactt ctacaacggg 7140
   agacagcaca gtagattctg aagatgaacc tcctaaaaaa gaaaaaaggg tggactggga 7200
   tgagtattgg aaccctgaag aaatagaaag aatgcttatg gactagggac tgtttacgaa 7260
   caaatgataa aaggaaatag ctgagcatga ctcatagtta aagcgctagc agctgcctaa 7320
   ccgcaaaacc acatcctatg gaaagcttgc taatgacgta taagttgttc cattgtaaga 7380
   gtatataacc agtgctttgt gaaacttcga ggagtctctt tgttgaggac ttttgagttc 7440
   tcccttgagg ctcccacaga tacaataaat atttgagatt gaaccctgtc gagtatctgt 7500
   gtaatctttt ttacctgtga ggtctcggaa tccgggccga gaacttcgca gcggccgctc 7560
   gagcatgcat ctagagggcc ctattctata gtgtcaccta aatgctagag ctcgctgatc 7620
   agcctcgact gtgccttcta gttgccagcc atctgttgtt tgcccctccc ccgtgccttc 7680
   cttgaccctg gaaggtgcca ctcccactgt cctttcctaa taaaatgagg aaattgcatc 7740
   gcattgtctg agtaggtgtc attctattct ggggggtggg gtggggcagg acagcaaggg 7800
   ggaggattgg gaagacaata gcaggcatgc tggggatgcg gtgggctcta tggcttctga 7860
   ggcggaaaga accagctggg gctcgagggg ggatccccac gcgccctgta gcggcgcatt 7920
   aagcgcggcg ggtgtggtgg ttacgcgcag cgtgaccgct acacttgcca gcgccctagc 7980
   gcccgctcct ttcgctttct tcccttcctt tctcgccacg ttcgccggct ttccccgtca 8040
   agctctaaat cggggcatcc ctttagggtt ccgatttagt gctttacggc acctcgaccc 8100
   caaaaaactt gattagggtg atggttcacg tagtgggcca tcgccctgat agacggtttt 8160
   tcgccctttg acgttggagt ccacgttctt taatagtgga ctcttgttcc aaactggaac 8220
   aacactcaac cctatctcgg tctattcttt tgatttataa gggattttgg ggatttcggc 8280
   ctattggtta aaaaatgagc tgatttaaca aaaatttaac gcgaatttta acaaaatatt 8340
   aacgtttaca atttaaatat ttgcttatac aatcttcctg tttttggggc ttttctgatt 8400
   atcaaccggg gtgggtaccg agctcgaatt ctgtggaatg tgtgtcagtt agggtgtgga 8460
   aagtccccag gctccccagg caggcagaag tatgcaaagc atgcatctca attagtcagc 8520
   aaccaggtgt ggaaagtccc caggctcccc agcaggcaga agtatgcaaa gcatgcatct 8580
   caattagtca gcaaccatag tcccgcccct aactccgccc atcccgcccc taactccgcc 8640
   cagttccgcc cattctccgc cccatggctg actaattttt tttatttatg cagaggccga 8700
   ggccgcctcg gcctctgagc tattccagaa gtagtgagga ggcttttttg gaggcctagg 8760
   cttttgcaaa aagctcccgg gagcttggat atccattttc ggatctgatc aagagacagg 8820
   atgaggatcg tttcgcatga ttgaacaaga tggattgcac gcaggttctc cggccgcttg 8880
   ggtggagagg ctattcggct atgactgggc acaacagaca atcggctgct ctgatgccgc 8940
   cgtgttccgg ctgtcagcgc aggggcgccc ggttcttttt gtcaagaccg acctgtccgg 9000
   tgccctgaat gaactgcagg acgaggcagc gcggctatcg tggctggcca cgacgggcgt 9060
   tccttgcgca gctgtgctcg acgttgtcac tgaagcggga agggactggc tgctattggg 9120
   cgaagtgccg gggcaggatc tcctgtcatc tcaccttgct cctgccgaga aagtatccat 9180
   catggctgat gcaatgcggc ggctgcatac gcttgatccg gctacctgcc cattcgacca 9240
   ccaagcgaaa catcgcatcg agcgagcacg tactcggatg gaagccggtc ttgtcgatca 9300
   ggatgatctg gacgaagagc atcaggggct cgcgccagcc gaactgttcg ccaggctcaa 9360
   ggcgcgcatg cccgacggcg aggatctcgt cgtgacccat ggcgatgcct gcttgccgaa 9420
   tatcatggtg gaaaatggcc gcttttctgg attcatcgac tgtggccggc tgggtgtggc 9480
   ggaccgctat caggacatag cgttggctac ccgtgatatt gctgaagagc ttggcggcga 9540
   atgggctgac cgcttcctcg tgctttacgg tatcgccgct cccgattcgc agcgcatcgc 9600
   cttctatcgc cttcttgacg agttcttctg agcgggactc tggggttcga aatgaccgac 9660
   caagcgacgc ccaacctgcc atcacgagat ttcgattcca ccgccgcctt ctatgaaagg 9720
   ttgggcttcg gaatcgtttt ccgggacgcc ggctggatga tcctccagcg cggggatctc 9780
   atgctggagt tcttcgccca ccccaacttg tttattgcag cttataatgg ttacaaataa 9840
   agcaatagca tcacaaattt cacaaataaa gcattttttt cactgcattc tagttgtggt 9900
   ttgtccaaac tcatcaatgt atcttatcat gtctggatcc cgtcgacctc gagagcttgg 9960
   cgtaatcatg gtcatagctg tttcctgtgt gaaattgtta tccgctcaca attccacaca 10020
   acatacgagc cggaagcata aagtgtaaag cctggggtgc ctaatgagtg agctaactca 10080
   cattaattgc gttgcgctca ctgcccgctt tccagtcggg aaacctgtcg tgccagctgc 10140
   attaatgaat cggccaacgc gcggggagag gcggtttgcg tattgggcgc tcttccgctt 10200
   cctcgctcac tgactcgctg cgctcggtcg ttcggctgcg gcgagcggta tcagctcact 10260
   caaaggcggt aatacggtta tccacagaat caggggataa cgcaggaaag aacatgtgag 10320
   caaaaggcca gcaaaaggcc aggaaccgta aaaaggccgc gttgctggcg tttttccata 10380
   ggctccgccc ccctgacgag catcacaaaa atcgacgctc aagtcagagg tggcgaaacc 10440
   cgacaggact ataaagatac caggcgtttc cccctggaag ctccctcgtg cgctctcctg 10500
   ttccgaccct gccgcttacc ggatacctgt ccgcctttct cccttcggga agcgtggcgc 10560
   tttctcaatg ctcacgctgt aggtatctca gttcggtgta ggtcgttcgc tccaagctgg 10620
   gctgtgtgca cgaacccccc gttcagcccg accgctgcgc cttatccggt aactatcgtc 10680
   ttgagtccaa cccggtaaga cacgacttat cgccactggc agcagccact ggtaacagga 10740
   ttagcagagc gaggtatgta ggcggtgcta cagagttctt gaagtggtgg cctaactacg 10800
   gctacactag aaggacagta tttggtatct gcgctctgct gaagccagtt accttcggaa 10860
   aaagagttgg tagctcttga tccggcaaac aaaccaccgc tggtagcggt ggtttttttg 10920
   tttgcaagca gcagattacg cgcagaaaaa aaggatctca agaagatcct ttgatctttt 10980
   ctacggggtc tgacgctcag tggaacgaaa actcacgtta agggattttg gtcatgagat 11040
   tatcaaaaag gatcttcacc tagatccttt taaattaaaa atgaagtttt aaatcaatct 11100
   aaagtatata tgagtaaact tggtctgaca gttaccaatg cttaatcagt gaggcaccta 11160
   tctcagcgat ctgtctattt cgttcatcca tagttgcctg actccccgtc gtgtagataa 11220
   ctacgatacg ggagggctta ccatctggcc ccagtgctgc aatgataccg cgagacccac 11280
   gctcaccggc tccagattta tcagcaataa accagccagc cggaagggcc gagcgcagaa 11340
   gtggtcctgc aactttatcc gcctccatcc agtctattaa ttgttgccgg gaagctagag 11400
   taagtagttc gccagttaat agtttgcgca acgttgttgc cattgctaca ggcatcgtgg 11460
   tgtcacgctc gtcgtttggt atggcttcat tcagctccgg ttcccaacga tcaaggcgag 11520
   ttacatgatc ccccatgttg tgcaaaaaag cggttagctc cttcggtcct ccgatcgttg 11580
   tcagaagtaa gttggccgca gtgttatcac tcatggttat ggcagcactg cataattctc 11640
   ttactgtcat gccatccgta agatgctttt ctgtgactgg tgagtactca accaagtcat 11700
   tctgagaata gtgtatgcgg cgaccgagtt gctcttgccc ggcgtcaata cgggataata 11760
   ccgcgccaca tagcagaact ttaaaagtgc tcatcattgg aaaacgttct tcggggcgaa 11820
   aactctcaag gatcttaccg ctgttgagat ccagttcgat gtaacccact cgtgcaccca 11880
   actgatcttc agcatctttt actttcacca gcgtttctgg gtgagcaaaa acaggaaggc 11940
   aaaatgccgc aaaaaaggga ataagggcga cacggaaatg ttgaatactc atactcttcc 12000
   tttttcaata ttattgaagc atttatcagg gttattgtct catgagcgga tacatatttg 12060
   aatgtattta gaaaaataaa caaatagggg ttccgcgcac atttccccga aaagtgccac 12120
   ctgacgtcga cggatcggga gatctcccga tcccctatgg tcgactctca gtacaatctg 12180
   ctctgatgcc gcatagttaa gccagtatct gctccctgct tgtgtgttgg aggtcgctga 12240
   gtagtgcgcg agcaaaattt aagctacaac aaggcaaggc ttgaccgaca attgcatgaa 12300
   gaatctgctt agggttaggc gttttgcgct gcttcgcgat gtacgggcca gatatacgcg 12360
   ttgacattga ttattgacta gttattaata gtaatcaatt acggggtcat tagttcatag 12420
   cccatatatg gagttccgcg ttacataact tacggtaaat ggcccgcctg gctgaccgcc 12480
   caacgacccc cgcccattga cgtcaataat gacgtatgtt cccatagtaa cgccaatagg 12540
   gactttccat tgacgtcaat gggtggacta tttacggtaa actgcccact tggcagtaca 12600
   tcaagtgtat catatgccaa gtacgccccc tattgacgtc aatgacggta aatggcccgc 12660
   ctggcattat gcccagtaca tgaccttatg ggactttcct acttggcagt acatctacgt 12720
   attagtcatc gctattacca tggtg 12745
<210> 9
   <211> 529
   <212> PRT
   <213> Artificial Sequence
<220>
   <223> Description of Artificial Sequence:/Note = Synthetic Construct
<400> 9
   Met Thr Ser Ser Arg Leu Trp Phe Ser Leu Leu Leu Ala Ala Ala Phe
    1               5                  10                  15
   Ala Gly Arg Ala Thr Ala Leu Trp Pro Trp Pro Gln Asn Phe Gln Thr
               20                  25                  30
   Ser Asp Gln Arg Tyr Val Leu Tyr Pro Asn Asn Phe Gln Phe Gln Tyr
           35                  40                  45
   Asp Val Ser Ser Ala Ala Gln Pro Gly Cys Ser Val Leu Asp Glu Ala
       50                  55                  60
   Phe Gln Arg Tyr Arg Asp Leu Leu Phe Gly Ser Gly Ser Trp Pro Arg
   65                  70                  75                  80
   Pro Tyr Leu Thr Gly Lys Arg His Thr Leu Glu Lys Asn Val Leu Val
                   85                  90                  95
   Val Ser Val Val Thr Pro Gly Cys Asn Gln Leu Pro Thr Leu Glu Ser
               100                 105                 110
   Val Glu Asn Tyr Thr Leu Thr Ile Asn Asp Asp Gln Cys Leu Leu Leu
           115                 120                 125
   Ser Glu Thr Val Trp Gly Ala Leu Arg Gly Leu Glu Thr Phe Ser Gln
       130                 135                 140
   Leu Val Trp Lys Ser Ala Glu Gly Thr Phe Phe Ile Asn Lys Thr Glu
   145                 150                 155                 160
   Ile Glu Asp Phe Pro Arg Phe Pro His Arg Gly Leu Leu Leu Asp Thr
                   165                 170                 175
   Ser Arg His Tyr Leu Pro Leu Ser Ser Ile Leu Asp Thr Leu Asp Val
               180                 185                 190
   Met Ala Tyr Asn Lys Leu Asn Val Phe His Trp His Leu Val Asp Asp
           195                 200                 205
   Pro Ser Phe Pro Tyr Glu Ser Phe Thr Phe Pro Glu Leu Met Arg Lys
       210                 215                 220
   Gly Ser Tyr Asn Pro Val Thr His Ile Tyr Thr Ala Gln Asp Val Lys
   225                 230                 235                 240
   Glu Val Ile Glu Tyr Ala Arg Leu Arg Gly Ile Arg Val Leu Ala Glu
                   245                 250                 255
   Phe Asp Thr Pro Gly His Thr Leu Ser Trp Gly Pro Gly Ile Pro Gly
               260                 265                 270
   Leu Leu Thr Pro Cys Tyr Ser Gly Ser Glu Pro Ser Gly Thr Phe Gly
           275                 280                 285
   Pro Val Asn Pro Ser Leu Asn Asn Thr Tyr Glu Phe Met Ser Thr Phe
       290                 295                 300
   Phe Leu Glu Val Ser Ser Val Phe Pro Asp Phe Tyr Leu His Leu Gly
   305                 310                 315                 320
   Gly Asp Glu Val Asp Phe Thr Cys Trp Lys Ser Asn Pro Glu Ile Gln
                   325                 330                 335
   Asp Phe Met Arg Lys Lys Gly Phe Gly Glu Asp Phe Lys Gln Leu Glu
               340                 345                 350
   Ser Phe Tyr Ile Gln Thr Leu Leu Asp Ile Val Ser Ser Tyr Gly Lys
           355                 360                 365
   Gly Tyr Val Val Trp Gln Glu Val Phe Asp Asn Lys Val Lys Ile Gln
       370                 375                 380
   Pro Asp Thr Ile Ile Gln Val Trp Arg Glu Asp Ile Pro Val Asn Tyr
   385                 390                 395                 400
   Met Lys Glu Leu Glu Leu Val Thr Lys Ala Gly Phe Arg Ala Leu Leu
                   405                 410                 415
   Ser Ala Pro Trp Tyr Leu Asn Arg Ile Ser Tyr Gly Pro Asp Trp Lys
               420                 425                 430
   Asp Phe Tyr Val Val Glu Pro Leu Ala Phe Glu Gly Thr Pro Glu Gln
           435                 440                 445
   Lys Ala Leu Val Ile Gly Gly Glu Ala Cys Met Trp Gly Glu Tyr Val
       450                 455                 460
   Asp Asn Thr Asn Leu Val Pro Arg Leu Trp Pro Arg Ala Gly Ala Val
   465                 470                 475                 480
   Ala Glu Arg Leu Trp Ser Asn Lys Leu Thr Ser Asp Leu Thr Phe Ala
                   485                 490                 495
   Tyr Glu Arg Leu Ser His Phe Arg Cys Glu Leu Leu Arg Arg Gly Val
               500                 505                 510
   Gln Ala Gln Pro Leu Asn Val Gly Phe Cys Glu Gln Glu Phe Glu Gln
           515                 520                 525
   Thr
<210> 10
   <211> 2255
   <212> DNA
   <213> Artificial Sequence
<220>
   <223> Description of Artificial Sequence:/Note = Synthetic Construct
<400> 10
   cctccgagag gggagaccag cgggccatga caagctccag gctttggttt tcgctgctgc      60
   tggcggcagc gttcgcagga cgggcgacgg ccctctggcc ctggcctcag aacttccaaa     120
   cctccgacca gcgctacgtc ctttacccga acaactttca attccagtac gatgtcagct     180
   cggccgcgca gcccggctgc tcagtcctcg acgaggcctt ccagcgctat cgtgacctgc     240
   ttttcggttc cgggtcttgg ccccgtcctt acctcacagg gaaacggcat acactggaga     300
   agaatgtgtt ggttgtctct gtagtcacac ctggatgtaa ccagcttcct actttggagt     360
   cagtggagaa ttataccctg accataaatg atgaccagtg tttactcctc tctgagactg     420
   tctggggagc tctccgaggt ctggagactt ttagccagct tgtttggaaa tctgctgagg     480
   gcacattctt tatcaacaag actgagattg aggactttcc ccgctttcct caccggggct     540
   tgctgttgga tacatctcgc cattacctgc cactctctag catcctggac actctggatg     600
   tcatggcgta caataaattg aacgtgttcc actggcatct ggtagatgat ccttccttcc     660
   catatgagag cttcactttt ccagagctca tgagaaaggg gtcctacaac cctgtcaccc     720
   acatctacac agcacaggat gtgaaggagg tcattgaata cgcacggctc cggggtatcc     780
   gtgtgcttgc agagtttgac actcctggcc acactttgtc ctggggacca ggtatccctg     840
   gattactgac tccttgctac tctgggtctg agccctctgg cacctttgga ccagtgaatc     900
   ccagtctcaa taatacctat gagttcatga gcacattctt cttagaagtc agctctgtct     960
   tcccagattt ttatcttcat cttggaggag atgaggttga tttcacctgc tggaagtcca    1020
   acccagagat ccaggacttt atgaggaaga aaggcttcgg tgaggacttc aagcagctgg    1080
   agtccttcta catccagacg ctgctggaca tcgtctcttc ttatggcaag ggctatgtgg    1140
   tgtggcagga ggtgtttgat aataaagtaa agattcagcc agacacaatc atacaggtgt    1200
   ggcgagagga tattccagtg aactatatga aggagctgga actggtcacc aaggccggct    1260
   tccgggccct tctctctgcc ccctggtacc tgaaccgtat atcctatggc cctgactgga    1320
   aggatttcta cgtagtggaa cccctggcat ttgaaggtac ccctgagcag aaggctctgg    1380
   tgattggtgg agaggcttgt atgtggggag aatatgtgga caacacaaac ctggtcccca    1440
   ggctctggcc cagagcaggg gctgttgccg aaaggctgtg gagcaacaag ttgacatctg    1500
   acctgacatt tgcctatgaa cgtttgtcac acttccgctg tgagttgctg aggcgaggtg    1560
   tccaggccca acccctcaat gtaggcttct gtgagcagga gtttgaacag acctgagccc    1620
   caggcaccga ggagggtgct ggctgtaggt gaatggtagt ggagccaggc ttccactgca    1680
   tcctggccag gggacggagc cccttgcctt cgtgcccctt gcctgcgtgc cctgtgcttg    1740
   ggagagaaag gggccggtgc tggcgctcgc attcaataaa gagtaatgtg gcatttttct   1800
   ataataaaca tggattacct gtgtttaaaa aaaaaagtgt gaatggcgtt agggtaaggg    1860
   cacagccagg ctggagtcag tgtctgcccc tgaggtcttt taagttgagg gctgggaatg    1920
   aaacctatag cctttgtgct gttctgcctt gcctgtgagc tatgtcactc ccctcccact    1980
   cctgaccata ttccagacac ctgccctaat cctcagcctg ctcacttcac ttctgcatta    2040
   tatctccaag gcgttggtat atggaaaaag atgtaggggc ttggaggtgt tctggacagt    2100
   ggggagggct ccagacccaa cctggtcaca aaagagcctc tcccccatgc atactcatcc    2160
   acctccctcc cctagagcta ttctcctttg ggtttcttgc tgctgcaatt ttatacaacc    2220
   attatttaaa tattattaaa cacatattgt tctct                               2255
<210> 11
   <211> 1635
   <212> DNA
   <213> Artificial Sequence
<220>
   <223> Description of Artificial Sequence:/Note = Synthetic Construct
<400> 11
   atgctactgg cgctgctgtt ggcgacactg ctggcggcga tgttggcgct gctgactcag      60
   gtggcgctgg tggtgcaggt ggcggaggcg gctcgggccc cgagcgtctc ggccaagccg     120
   gggccggcgc tgtggcccct gccgctcttg gtgaagatga ccccgaacct gctgcatctc     180
   gccccggaga acttctacat cagccacagc cccaattcca cggcgggccc ctcctgcacc     240
   ctgctggagg aagcgtttcg acgatatcat ggctatattt ttggtttcta caagtggcat     300
   catgaacctg ctgaattcca ggctaaaacc caggttcagc aacttcttgt ctcaatcacc     360
   cttcagtcag agtgtgatgc tttccccaac atatcttcag atgagtctta tactttactt     420
   gtgaaagaac cagtggctgt ccttaaggcc aacagagttt ggggagcatt acgaggttta     480
   gagaccttta gccagttagt ttatcaagat tcttatggaa ctttcaccat caatgaatcc     540
   accattattg attctccaag gttttctcac agaggaattt tgattgatac atccagacat     600
   tatctgccag ttaagattat tcttaaaact ctggatgcca tggcttttaa taagtttaat     660
   gttcttcact ggcacatagt tgatgaccag tctttcccat atcagagcat cacttttcct     720
   gagttaagca ataaaggaag ctattctttg tctcatgttt atacaccaaa tgatgtccgt     780
   atggtgattg aatatgccag attacgagga attcgagtcc tgccagaatt tgatacccct     840
   gggcatacac tatcttgggg aaaaggtcag aaagacctcc tgactccatg ttacagtaga     900
   caaaacaagt tggactcttt tggacctata aaccctactc tgaatacaac atacagcttc     960
   cttactacat ttttcaaaga aattagtgag gtgtttccag atcaattcat tcatttggga     1020
   ggagatgaag tggaatttaa atgttgggaa tcaaatccaa aaattcaaga tttcatgagg    1080
   caaaaaggct ttggcacaga ttttaagaaa ctagaatctt tctacattca aaaggttttg    1140
   gatattattg caaccataaa caagggatcc attgtctggc aggaggtttt tgatgataaa    1200
   gcaaagcttg cgccgggcac aatagttgaa gtatggaaag acagcgcata tcctgaggaa    1260
   ctcagtagag tcacagcatc tggcttccct gtaatccttt ctgctccttg gtacttagat    1320
   ttgattagct atggacaaga ttggaggaaa tactataaag tggaacctct tgattttggc    1380
   ggtactcaga aacagaaaca acttttcatt ggtggagaag cttgtctatg gggagaatat    1440
   gtggatgcaa ctaacctcac tccaagatta tggcctcggg caagtgctgt tggtgagaga    1500
   ctctggagtt ccaaagatgt cagagatatg gatgacgcct atgacagact gacaaggcac    1560
   cgctgcagga tggtcgaacg tggaatagct gcacaacctc tttatgctgg atattgtaac    1620
   catgagaaca tgtaa                                                     1635
<210> 12
   <211> 544
   <212> PRT
   <213> Artificial Sequence
<220>
   <223> Description of Artificial Sequence:/Note = Synthetic Construct
<400> 12
   Met Leu Leu Ala Leu Leu Leu Ala Thr Leu Leu Ala Ala Met Leu Ala
    1               5                   10                  15
   Leu Leu Thr Gln Ile Ala Leu Val Val Gln Val Ala Glu Ala Ala Arg
               20                  25                  30
   Ala Pro Ser Val Ser Ala Lys Pro Gly Pro Ala Leu Trp Pro Leu Pro
           35                  40                  45
   Leu Leu Val Lys Met Thr Pro Asn Leu Leu His Leu Ala Pro Glu Asn
       50                  55                  60
   Phe Tyr Ile Ser His Ser Pro Asn Ser Thr Ala Gly Pro Ser Cys Thr
   65                  70                  75                  80
   Leu Leu Glu Glu Ala Phe Arg Arg Tyr His Gly Tyr Ile Phe Gly Phe
                   85                  90                  95
   Tyr Lys Trp His His Glu Pro Ala Glu Phe Gln Ala Lys Thr Gln Val
               100                 105                 110
   Gln Gln Leu Leu Val Ser Ile Thr Leu Gln Ser Glu Cys Asp Ala Phe
           115                 120                 125
   Pro Asn Ile Ser Ser Asp Glu Ser Tyr Thr Leu Leu Val Lys Glu Pro
       130                 135                 140
   Val Ala Val Leu Lys Ala Asn Arg Val Trp Gly Ala Leu Arg Gly Leu
   145                 150                 155                 160
   Glu Thr Phe Ser Gln Leu Val Tyr Gln Asp Ser Tyr Gly Thr Phe Thr
                   165                 170                 175
   Ile Asn Glu Ser Thr Ile Ile Asp Ser Pro Arg Phe Ser His Arg Gly
               180                 185                 190
   Ile Leu Ile Asp Thr Ser Arg His Tyr Leu Pro Val Lys Ile Ile Leu
           195                 200                 205
   Lys Thr Leu Asp Ala Met Ala Phe Asn Lys Phe Asn Val Leu His Trp
       210                 215                 220
   His Ile Val Asp Asp Gln Ser Phe Pro Tyr Gln Ser Ile Thr Phe Pro
   225                 230                 235                 240
   Glu Leu Ser Asn Lys Gly Ser Tyr Ser Leu Ser His Val Tyr Thr Pro
                   245                 250                 255
   Asn Asp Val Arg Met Val Ile Glu Tyr Ala Arg Leu Arg Gly Ile Arg
               260                 265                 270
   Val Leu Pro Glu Phe Asp Thr Pro Gly His Thr Leu Ser Trp Gly Lys
           275                 280                 285
   Gly Gln Lys Asp Leu Leu Thr Pro Cys Tyr Ser Arg Gln Asn Lys Leu
       290                 295                 300
   Asp Ser Phe Gly Pro Ile Asn Pro Thr Leu Asn Thr Thr Tyr Ser Phe
   305                 310                 315                 320
   Leu Thr Thr Phe Phe Lys Glu Ile Ser Glu Val Phe Pro Asp Gln Phe
                   325                 330                 335
   Ile His Leu Gly Gly Asp Glu Val Glu Phe Lys Cys Trp Glu Ser Asn
               340                 345                 350
   Pro Lys Ile Gln Asp Phe Met Arg Gln Lys Gly Phe Gly Thr Asp Phe
           355                 360                 365
   Lys Lys Leu Glu Ser Phe Tyr Ile Gln Lys Val Leu Asp Ile Ile Ala
       370                 375                 380
   Thr Ile Asn Lys Gly Ser Ile Val Trp Gln Glu Val Phe Asp Asp Lys
   385                 390                 395                 400
   Ala Lys Leu Ala Pro Gly Thr Ile Val Glu Val Trp Lys Asp Ser Ala
                   405                 410                 415
   Tyr Pro Glu Glu Leu Ser Arg Val Thr Ala Ser Gly Phe Pro Val Ile
               420                 425                 430
   Leu Ser Ala Pro Trp Tyr Leu Asp Leu Ile Ser Tyr Gly Gln Asp Trp
           435                 440                 445
   Arg Lys Tyr Tyr Lys Val Glu Pro Leu Asp Phe Gly Gly Thr Gln Lys
       450                 455                 460
   Gln Lys Gln Leu Phe Ile Gly Gly Glu Ala Cys Leu Trp Gly Glu Tyr
   465                 470                 475                 480
   Val Asp Ala Thr Asn Leu Thr Pro Arg Leu Trp Pro Arg Ala Ser Ala
                   485                 490                 495
   Val Gly Glu Arg Leu Trp Ser Ser Lys Asp Val Arg Asp Met Asp Asp
               500                 505                 510
   Ala Tyr Asp Arg Leu Thr Arg His Arg Cys Arg Met Val Glu Arg Gly
           515                 520                 525
   Ile Ala Ala Gln Pro Leu Tyr Ala Gly Tyr Cys Asn His Glu Asn Met
       530                 535                 540
<210> 13
   <211> 529
   <212> PRT
   <213> Artificial Sequence
<220>
   <223> Description of Artificial Sequence:/Note = Synthetic Construct
<400> 13
   Met Thr Ser Ser Arg Leu Trp Phe Ser Leu Leu Leu Ala Ala Ala Phe
    1               5                  10                  15
   Ala Gly Arg Ala Thr Ala Leu Trp Pro Trp Pro Gln Asn Phe Gln Thr
               20                  25                  30
   Ser Asp Gln Arg Tyr Val Leu Tyr Pro Asn Asn Phe Gln Phe Gln Tyr
           35                  40                  45
   Asp Val Ser Ser Ala Ala Gln Pro Gly Cys Ser Val Leu Asp Glu Ala
       50                  55                  60
   Phe Gln Arg Tyr Arg Asp Leu Leu Phe Gly Ser Gly Ser Trp Pro Arg
   65                  70                  75                  80
   Pro Tyr Leu Thr Gly Lys Arg His Thr Leu Glu Lys Asn Val Leu Val
                       85                  90                  95
   Val Ser Val Val Thr Pro Gly Cys Asn Gln Leu Pro Thr Leu Glu Ser
               100                 105                 110
   Val Glu Asn Tyr Thr Leu Thr Ile Asn Asp Asp Gln Cys Leu Leu Leu
           115                 120                 125
   Ser Glu Thr Val Trp Gly Ala Leu Arg Gly Leu Glu Thr Phe Ser Gln
       130                 135                 140
   Leu Val Trp Lys Ser Ala Glu Gly Thr Phe Phe Ile Asn Lys Thr Glu
   145                 150                 155                 160
   Ile Glu Asp Phe Pro Arg Phe Pro His Arg Gly Leu Leu Leu Asp Thr
                   165                 170                 175
   Ser Arg His Tyr Leu Pro Leu Ser Ser Ile Leu Asp Thr Leu Asp Val
               180                 185                 190
   Met Ala Tyr Asn Lys Leu Asn Val Phe His Trp His Leu Val Asp Asp
           195                 200                 205
   Pro Ser Phe Pro Tyr Glu Ser Phe Thr Phe Pro Glu Leu Met Arg Lys
       210                 215                 220
   Gly Ser Tyr Asn Pro Val Thr His Ile Tyr Thr Ala Gln Asp Val Lys
   225                 230                 235                 240
   Glu Val Ile Glu Tyr Ala Arg Leu Arg Gly Ile Arg Val Leu Ala Glu
                   245                 250                 255
   Phe Asp Thr Pro Gly His Thr Leu Ser Trp Gly Pro Gly Ile Pro Gly
               260                 265                 270
   Leu Leu Thr Pro Cys Tyr Ser Gly Ser Glu Pro Ser Gly Thr Phe Gly
           275                 280                 285
   Pro Val Asn Pro Ser Leu Asn Asn Thr Tyr Glu Phe Met Ser Thr Phe
       290                 295                 300
   Phe Leu Glu Val Ser Ser Val Phe Pro Asp Phe Tyr Leu His Leu Gly
   305                 310                 315                 320
   Gly Asp Glu Val Asp Phe Thr Cys Trp Lys Ser Asn Pro Glu Ile Gln
                   325                 330                 335
   Asp Phe Met Arg Lys Lys Gly Phe Gly Glu Asp Phe Lys Gln Leu Glu
               340                 345                 350
   Ser Phe Tyr Ile Gln Thr Leu Leu Asp Ile Val Ser Ser Tyr Gly Lys
           355                 360                 365
   Gly Tyr Val Val Trp Gln Glu Val Phe Asp Asn Lys Val Lys Ile Gln
       370                 375                 380
   Pro Asp Thr Ile Ile Gln Val Trp Arg Glu Asp Ile Pro Val Asn Tyr
   385                 390                 395                 400
   Met Lys Glu Leu Glu Leu Val Thr Lys Ala Gly Phe Arg Ala Leu Leu
                   405                 410                 415
   Ser Ala Pro Trp Tyr Leu Asn Arg Ile Ser Tyr Gly Pro Asp Trp Lys
               420                 425                 430
   Asp Phe Tyr Val Val Glu Pro Leu Ala Phe Glu Gly Thr Pro Glu Gln
           435                 440                 445
   Lys Ala Leu Val Ile Gly Gly Glu Ala Cys Met Trp Gly Glu Tyr Val
       450                 455                 460
   Asp Asn Thr Asn Leu Val Pro Arg Leu Trp Pro Arg Ala Gly Ala Val
   465                 470                 475                 480
   Ala Glu Arg Leu Trp Ser Asn Lys Leu Thr Ser Asp Leu Thr Phe Ala
                   485                 490                 495
   Tyr Glu Arg Leu Ser His Phe Arg Cys Glu Leu Leu Arg Arg Gly Val
               500                 505                 510
   Gln Ala Gln Pro Leu Asn Val Gly Phe Cys Glu Gln Glu Phe Glu Gln
           515                 520                 525
   Thr
<210> 14
   <211> 739
   <212> DNA
   <213> Artificial Sequence
<220>
   <223> Description of Artificial Sequence:/Note = Synthetic Construct
<400> 14
   ttttaatcct ccgtttttct gcttctgaag ttacttcagc ctggcaagtc ctttacctcc      60
   ccgtaggcct ggcgagctgc atcacaacat tcaagattca ccctagagcc atctgggaaa     120
   ctttcttctc caggtcgccc tgcgtcctcg cctccccacc ccgttcttct cgagtcgggt     180
   gagctgtcta gttccatcac ggccggcacg gccgcagggg tggccggtta tttactgctc     240
   tactgggccc gtgagcagtc tggcgagccg agcagttgcc gacgcccggc acaatccgct     300
   gcacgtagca ggagcctcag gtccaggccg gaagtgaaag ggcagggtgt gggtcctcct     360
   ggggtcgcag gcgcagagcc gcctctggtc acgtgattcg ccgataagtc acgggggcgc     420
   cgctcacctg accagggtct cacgtggcca gccccctccg agaggggaga ccagcgggcc     480
   atgacaagct ccaggctttg gttttcgctg ctgctggcgg cagcgttcgc aggacgggcg     540
   acggccctct ggccctggcc tcagaacttc caaacctccg accagcgcta cgtcctttac     600
   ccgaacaact ttcaattcca gtacgatgtc agctcggccg cgcagcccgg ctgctcagtc     660
   ctcgacgagg ccttccagcg ctatcgtgac ctgcttttcg gttccgggtc ttggccccgt     720
   ccttacctca caggtgagt                                                  739
<210> 15
   <211> 556
   <212> PRT
   <213> Artificial Sequence
<220>
   <223> Description of Artificial Sequence:/Note = Synthetic Construct
<400> 15
   Met Glu Leu Cys Gly Leu Gly Leu Pro Arg Pro Pro Met Leu Leu Ala
    1               5                  10                  15
   Leu Leu Leu Ala Thr Leu Leu Ala Ala Met Leu Ala Leu Leu Thr Gln
               20                  25                  30
   Val Ala Leu Val Val Gln Val Ala Glu Ala Ala Arg Ala Pro Ser Val
           35                  40                  45
   Ser Ala Lys Pro Gly Pro Ala Leu Trp Pro Leu Pro Leu Ser Val Lys
       50                  55                  60
   Met Thr Pro Asn Leu Leu His Leu Ala Pro Glu Asn Phe Tyr Ile Ser
   65                  70                  75                  80
   His Ser Pro Asn Ser Thr Ala Gly Pro Ser Cys Thr Leu Leu Glu Glu
                   85                  90                  95
   Ala Phe Arg Arg Tyr His Gly Tyr Ile Phe Gly Phe Tyr Lys Trp His
               100                 105                 110
   His Glu Pro Ala Glu Phe Gln Ala Lys Thr Gln Val Gln Gln Leu Leu
           115                 120                 125
   Val Ser Ile Thr Leu Gln Ser Glu Cys Asp Ala Phe Pro Asn Ile Ser
       130                 135                 140
   Ser Asp Glu Ser Tyr Thr Leu Leu Val Lys Glu Pro Val Ala Val Leu
   145                 150                 155                 160
   Lys Ala Asn Arg Val Trp Gly Ala Leu Arg Gly Leu Glu Thr Phe Ser
                   165                 170                 175
   Gln Leu Val Tyr Gln Asp Ser Tyr Gly Thr Phe Thr Ile Asn Glu Ser
               180                 185                 190
   Thr Ile Ile Asp Ser Pro Arg Phe Ser His Arg Gly Ile Leu Ile Asp
           195                 200                 205
   Thr Ser Arg His Tyr Leu Pro Val Lys Ile Ile Leu Lys Thr Leu Asp
       210                 215                 220
   Ala Met Ala Phe Asn Lys Phe Asn Val Leu His Trp His Ile Val Asp
   225                 230                 235                 240
   Asp Gln Ser Phe Pro Tyr Gln Ser Ile Thr Phe Pro Glu Leu Ser Asn
                   245                 250                 255
   Lys Gly Ser Tyr Ser Leu Ser His Val Tyr Thr Pro Asn Asp Val Arg
               260                 265                 270
   Met Val Ile Glu Tyr Ala Arg Leu Arg Gly Ile Arg Val Leu Pro Glu
           275                 280                 285
   Phe Asp Thr Pro Gly His Thr Leu Ser Trp Gly Lys Gly Gln Lys Asp
       290                 295                 300
   Leu Leu Thr Pro Cys Tyr Ser Arg Gln Asn Lys Leu Asp Ser Phe Gly
   305                 310                 315                 320
   Pro Ile Asn Pro Thr Leu Asn Thr Thr Tyr Ser Phe Leu Thr Thr Phe
                   325                 330                 335
   Phe Lys Glu Ile Ser Glu Val Phe Pro Asp Gln Phe Ile His Leu Gly
               340                 345                 350
   Gly Asp Glu Val Glu Phe Lys Cys Trp Glu Ser Asn Pro Lys Ile Gln
           355                 360                 365
   Asp Phe Met Arg Gln Lys Gly Phe Gly Thr Asp Phe Lys Lys Leu Glu
       370                 375                 380
   Ser Phe Tyr Ile Gln Lys Val Leu Asp Ile Ile Ala Thr Ile Asn Lys
   385                 390                 395                 400
   Gly Ser Ile Val Trp Gln Glu Val Phe Asp Asp Lys Ala Lys Leu Ala
                   405                 410                 415
   Pro Gly Thr Ile Val Glu Val Trp Lys Asp Ser Ala Tyr Pro Glu Glu
               420                 425                 430
   Leu Ser Arg Val Thr Ala Ser Gly Phe Pro Val Ile Leu Ser Ala Pro
           435                 440                 445
   Trp Tyr Leu Asp Leu Ile Ser Tyr Gly Gln Asp Trp Arg Lys Tyr Tyr
       450                 455                 460
   Lys Val Glu Pro Leu Asp Phe Gly Gly Thr Gln Lys Gln Lys Gln Leu
   465                 470                 475                 480
   Phe Ile Gly Gly Glu Ala Cys Leu Trp Gly Glu Tyr Val Asp Ala Thr
                   485                 490                 495
   Asn Leu Thr Pro Arg Leu Trp Pro Arg Ala Ser Ala Val Gly Glu Arg
               500                 505                 510
   Leu Trp Ser Ser Lys Asp Val Arg Asp Met Asp Asp Ala Tyr Asp Arg
           515                 520                 525
   Leu Thr Arg His Arg Cys Arg Met Val Glu Arg Gly Ile Ala Ala Gln
       530                 535                 540
   Pro Leu Tyr Ala Gly Tyr Cys Asn His Glu Asn Met
   545                 550                 555
<210> 16
   <211> 1857
   <212> DNA
   <213> Artificial Sequence
<220>
   <223> Description of Artificial Sequence:/Note = Synthetic Construct
<400> 16
   ctgatccggg ccgggcggga agtcgggtcc cgaggctccg gctcggcaga ccgggcggaa     60
   agcagccgag cggccatgga gctgtgcggg ctggggctgc cccggccgcc catgctgctg    120
   gcgctgctgt tggcgacact gctggcggcg atgttggcgc tgctgactca ggtggcgctg    180
   gtggtgcagg tggcggaggc ggctcgggcc ccgagcgtct cggccaagcc ggggccggcg    240
   ctgtggcccc tgccgctctc ggtgaagatg accccgaacc tgctgcatct cgccccggag    300
   aacttctaca tcagccacag ccccaattcc acggcgggcc cctcctgcac cctgctggag    360
   gaagcgtttc gacgatatca tggctatatt tttggtttct acaagtggca tcatgaacct    420
   gctgaattcc aggctaaaac ccaggttcag caacttcttg tctcaatcac ccttcagtca    480
   gagtgtgatg ctttccccaa catatcttca gatgagtctt atactttact tgtgaaagaa    540
   ccagtggctg tccttaaggc caacagagtt tggggagcat tacgaggttt agagaccttt    600
   agccagttag tttatcaaga ttcttatgga actttcacca tcaatgaatc caccattatt    660
   gattctccaa ggttttctca cagaggaatt ttgattgata catccagaca ttatctgcca    720
   gttaagatta ttcttaaaac tctggatgcc atggctttta ataagtttaa tgttcttcac    780
   tggcacatag ttgatgacca gtctttccca tatcagagca tcacttttcc tgagttaagc    840
   aataaaggaa gctattcttt gtctcatgtt tatacaccaa atgatgtccg tatggtgatt    900
   gaatatgcca gattacgagg aattcgagtc ctgccagaat ttgatacccc tgggcataca    960
   ctatcttggg gaaaaggtca gaaagacctc ctgactccat gttacagtag acaaaacaag   1020
   ttggactctt ttggacctat aaaccctact ctgaatacaa catacagctt ccttactaca   1080
   tttttcaaag aaattagtga ggtgtttcca gatcaattca ttcatttggg aggagatgaa   1140
   gtggaattta aatgttggga atcaaatcca aaaattcaag atttcatgag gcaaaaaggc   1200
   tttggcacag attttaagaa actagaatct ttctacattc aaaaggtttt ggatattatt   1260
   gcaaccataa acaagggatc cattgtctgg caggaggttt ttgatgataa agcaaagctt   1320
   gcgccgggca caatagttga agtatggaaa gacagcgcat atcctgagga actcagtaga   1380
   gtcacagcat ctggcttccc tgtaatcctt tctgctcctt ggtacttaga tttgattagc   1440
   tatggacaag attggaggaa atactataaa gtggaacctc ttgattttgg cggtactcag   1500
   aaacagaaac aacttttcat tggtggagaa gcttgtctat ggggagaata tgtggatgca   1560
   actaacctca ctccaagatt atggcctcgg gcaagtgctg ttggtgagag actctggagt   1620
   tccaaagatg tcagagatat ggatgacgcc tatgacagac tgacaaggca ccgctgcagg   1680
   atggtcgaac gtggaatagc tgcacaacct ctttatgctg gatattgtaa ccatgagaac   1740
   atgtaaaaaa tggaggggaa aaaggccaca gcaatctgta ctacaatcaa ctttattttg   1800
   aaatcatgta aaataagata ttagactttt ttgaataaaa tatttttatt gattgaa      1857
<210> 17
   <211> 536
   <212> PRT
   <213> Artificial Sequence
<220>
   <223> Description of Artificial Sequence:/Note = Synthetic Construct
<400> 17
   Met Pro Gln Ser Pro Arg Ser Ala Pro Gly Leu Leu Leu Leu Gln Ala
    1               5                  10                  15
   Leu Val Ser Leu Val Ser Leu Ala Leu Val Ala Pro Ala Arg Leu Gln
               20                  25                  30
   Pro Ala Leu Trp Pro Phe Pro Arg Ser Val Gln Met Phe Pro Arg Leu
           35                  40                  45
   Leu Tyr Ile Ser Ala Glu Asp Phe Ser Ile Asp His Ser Pro Asn Ser
       50                  55                  60
   Thr Ala Gly Pro Ser Cys Ser Leu Leu Gln Glu Ala Phe Arg Arg Tyr
   65                  70                  75                  80
   Tyr Asn Tyr Val Phe Gly Phe Tyr Lys Arg His His Gly Pro Ala Arg
                   85                  90                  95
   Phe Arg Ala Glu Pro Gln Leu Gln Lys Leu Leu Val Ser Ile Thr Leu
               100                 105                 110
   Glu Ser Glu Cys Glu Ser Phe Pro Ser Leu Ser Ser Asp Glu Thr Tyr
           115                 120                 125
   Ser Leu Leu Val Gln Glu Pro Val Ala Val Leu Lys Ala Asn Ser Val
       130                 135                 140
   Trp Gly Ala Leu Arg Gly Leu Glu Thr Phe Ser Gln Leu Val Tyr Gln
   145                 150                 155                 160
   Asp Ser Phe Gly Thr Phe Thr Ile Asn Glu Ser Ser Ile Ala Asp Ser
                   165                 170                 175
   Pro Arg Phe Pro His Arg Gly Ile Leu Ile Asp Thr Ser Arg His Phe
               180                 185                 190
   Leu Pro Val Lys Thr Ile Leu Lys Thr Leu Asp Ala Met Ala Phe Asn
           195                 200                 205
   Lys Phe Asn Val Leu His Trp His Ile Val Asp Asp Gln Ser Phe Pro
       210                 215                 220
   Tyr Gln Ser Thr Thr Phe Pro Glu Leu Ser Asn Lys Gly Ser Tyr Ser
   225                 230                 235                 240
   Leu Ser His Val Tyr Thr Pro Asn Asp Val Arg Met Val Leu Glu Tyr
                   245                 250                 255
   Ala Arg Leu Arg Gly Ile Arg Val Ile Pro Glu Phe Asp Thr Pro Gly
               260                 265                 270
   His Thr Gln Ser Trp Gly Lys Gly Gln Lys Asn Leu Leu Thr Pro Cys
           275                 280                 285
   Tyr Asn Gln Lys Thr Lys Thr Gln Val Phe Gly Pro Val Asp Pro Thr
           290                 295                 300
   Val Asn Thr Thr Tyr Ala Phe Phe Asn Thr Phe Phe Lys Glu Ile Ser
   305                 310                 315                 320
   Ser Val Phe Pro Asp Gln Phe Ile His Leu Gly Gly Asp Glu Val Glu
                   325                 330                 335
   Phe Gln Cys Trp Ala Ser Asn Pro Asn Ile Gln Gly Phe Met Lys Arg
               340                 345                 350
   Lys Gly Phe Gly Ser Asp Phe Arg Arg Leu Glu Ser Phe Tyr Ile Lys
           355                 360                 365
   Lys Ile Leu Glu Ile Ile Ser Ser Leu Lys Lys Asn Ser Ile Val Trp
       370                 375                 380
   Gln Glu Val Phe Asp Asp Lys Val Glu Leu Gln Pro Gly Thr Val Val
   385                 390                 395                 400
   Glu Val Trp Lys Ser Glu His Tyr Ser Tyr Glu Leu Lys Gln Val Thr
                   405                 410                 415
   Gly Ser Gly Phe Pro Ala Ile Leu Ser Ala Pro Trp Tyr Leu Asp Leu
               420                 425                 430
   Ile Ser Tyr Gly Gln Asp Trp Lys Asn Tyr Tyr Lys Val Glu Pro Leu
           435                 440                 445
   Asn Phe Glu Gly Ser Glu Lys Gln Lys Gln Leu Val Ile Gly Gly Glu
       450                 455                 460
   Ala Cys Leu Trp Gly Glu Phe Val Asp Ala Thr Asn Leu Thr Pro Arg
   465                 470                 475                 480
   Leu Trp Pro Arg Ala Ser Ala Val Gly Glu Arg Leu Trp Ser Pro Lys
                   485                 490                 495
   Thr Val Thr Asp Leu Glu Asn Ala Tyr Lys Arg Leu Ala Val His Arg
               500                 505                 510
   Cys Arg Met Val Ser Arg Gly Ile Ala Ala Gln Pro Leu Tyr Thr Gly
           515                 520                 525
   Tyr Cys Asn Tyr Glu Asn Lys Ile
       530                 535
<210> 18
   <211> 1750
   <212> DNA
   <213> Artificial Sequence
<220>
   <223> Description of Artificial Sequence:/Note = Synthetic Construct
<400> 18
   ggagcagtca tgccgcagtc cccgcgtagc gcccccgggc tgctgctgct gcaggcgctg     60
   gtgtcgctag tgtcgctggc cctagtggcc ccggcccgac tgcaacctgc gctatggccc    120
   ttcccgcgct cggtgcagat gttcccgcgg ctgttgtaca tctccgcgga ggacttcagc    180
   atcgaccaca gtcccaattc cacagcgggc ccttcctgct cgctgctaca ggaggcgttt    240
   cggcgatatt acaactatgt ttttggtttc tacaagagac atcatggccc tgctagattt    300
   cgagctgagc cacagttgca gaagctcctg gtctccatta ccctcgagtc agagtgcgag    360
   tccttcccta gtctgtcttc agatgaaacc tattctctgc ttgtacaaga accagtagcc    420
   gtcctcaagg ccaacagcgt ttggggagcg ttacgaggtt tagagacgtt tagccagtta    480
   gtttaccaag actctttcgg gactttcacc atcaatgaat ccagtatagc tgattctcca    540
   agattccctc atagaggaat tttaattgat acatctagac acttcctgcc tgtgaagaca    600
   attttaaaaa ctctggatgc catggctttt aataagttta atgttcttca ctggcacata    660
   gtggacgacc agtctttccc ttatcagagt accacttttc ctgagctaag caataaggga    720
   agctactctt tgtctcatgt ctatacacca aacgatgtcc ggatggtgct ggagtacgcc    780
   cggctccgag ggattcgagt cataccagaa tttgataccc ctggccatac acagtcttgg    840
   ggcaaaggac agaaaaacct tctaactcca tgttacaatc aaaaaactaa aactcaagtg    900
   tttgggcctg tagacccaac tgtaaacaca acgtatgcat tctttaacac atttttcaaa    960
   gaaatcagca gtgtgtttcc agatcagttc atccacttgg gaggagatga agtagaattt   1020
   caatgttggg catcaaatcc aaacatccaa ggtttcatga agagaaaggg ctttggcagc   1080
   gattttagaa gactagaatc cttttatatt aaaaagattt tggaaattat ttcatcctta   1140
   aagaagaact ccattgtttg gcaagaagtt tttgatgata aggtggagct tcagccgggc   1200
   acagtagtcg aagtgtggaa gagtgagcat tattcatatg agctaaagca agtcacaggc   1260
   tctggcttcc ctgccatcct ttctgctcct tggtacttag acctgatcag ctatgggcaa   1320
   gactggaaaa actactacaa agttgagccc cttaattttg aaggctctga gaagcagaaa   1380
   caacttgtta ttggtggaga agcttgcctg tggggagaat ttgtggatgc aactaacctt   1440
   actccaagat tatggcctcg agcaagcgct gttggtgaga gactctggag ccctaaaact   1500
   gtcactgacc tagaaaatgc ctacaaacga ctggccgtgc accgctgcag aatggtcagc   1560
   cgtggaatag ctgcacaacc tctctatact ggatactgta actatgagaa taaaatatag   1620
   aagtgacaga cgtctacagc attccagcta tgatcatgtt gattctgaaa tcatgtaaat   1680
   taagatttgt taggctgttt tttttttaaa taaaccatct ttttattgat tgaatctttc   1740
   taaaaaaaaa                                                           1750
<210> 19
   <211> 12263
   <212> DNA
   <213> Artificial Sequence
<220>
   <223> Description of Artificial Sequence:/Note = Synthetic Construct
<400> 19
   aatgtagtct tatgcaatac tcttgtagtc ttgcaacatg gtaacgatga gttagcaaca     60
   tgccttacaa ggagagaaaa agcaccgtgc atgccgattg gtggaagtaa ggtggtacga    120
   tcgtgcctta ttaggaaggc aacagacggg tctgacatgg attggacgaa ccactgaatt    180
   gccgcattgc agagatattg tatttaagtg cctagctcga tacataaacg ggtctctctg    240
   gttagaccag atctgagcct gggagctctc tggctaacta gggaacccac tgcttaagcc    300
   tcaataaagc ttgccttgag tgcttcaagt agtgtgtgcc cgtctgttgt gtgactctgg    360
   taactagaga tccctcagac ccttttagtc agtgtggaaa atctctagca gtggcgcccg    420
   aacagggact tgaaagcgaa agggaaacca gaggagctct ctcgacgcag gactcggctt    480
   gctgaagcgc gcacggcaag aggcgagggg cggcgactgg tgagtacgcc aaaaattttg    540
   actagcggag gctagaagga gagagatggg tgcgagagcg tcagtattaa gcgggggaga    600
   attagatcgc gatgggaaaa aattcggtta aggccagggg gaaagaaaaa atataaatta    660
   aaacatatag tatgggcaag cagggagcta gaacgattcg cagttaatcc tggcctgtta    720
   gaaacatcag aaggctgtag acaaatactg ggacagctac aaccatccct tcagacagga    780
   tcagaagaac ttagatcatt atataataca gtagcaaccc tctattgtgt gcatcaaagg    840
   atagagataa aagacaccaa ggaagcttta gacaagatag aggaagagca aaacaaaagt    900
   aagaccaccg cacagcaagc ggccgctgat cttcagacct ggaggaggag atatgaggga    960
   caattggaga agtgaattat ataaatataa agtagtaaaa attgaaccat taggagtagc   1020
   acccaccaag gcaaagagaa gagtggtgca gagagaaaaa agagcagtgg gaataggagc   1080
   tttgttcctt gggttcttgg gagcagcagg aagcactatg ggcgcagcgt caatgacgct   1140
   gacggtacag gccagacaat tattgtctgg tatagtgcag cagcagaaca atttgctgag   1200
   ggctattgag gcgcaacagc atctgttgca actcacagtc tggggcatca agcagctcca   1260
   ggcaagaatc ctggctgtgg aaagatacct aaaggatcaa cagctcctgg ggatttgggg   1320
   ttgctctgga aaactcattt gcaccactgc tgtgccttgg aatgctagtt ggagtaataa   1380
   atctctggaa cagatttgga atcacacgac ctggatggag tgggacagag aaattaacaa   1440
   ttacacaagc ttaatacact ccttaattga agaatcgcaa aaccagcaag aaaagaatga   1500
   acaagaatta ttggaattag ataaatgggc aagtttgtgg aattggttta acataacaaa   1560
   ttggctgtgg tatataaaat tattcataat gatagtagga ggcttggtag gtttaagaat   1620
   agtttttgct gtactttcta tagtgaatag agttaggcag ggatattcac cattatcgtt   1680
   tcagacccac ctcccaaccc cgaggggacc cgacaggccc gaaggaatag aagaagaagg   1740
   tggagagaga gacagagaca gatccattcg attagtgaac ggatctcgac ggtatcgata   1800
   agcttgatat cgaattcggt accctagtta ttaatagtaa tcaattacgg ggtcattagt   1860
   tcatagccca tatatggagt tccgcgttac ataacttacg gtaaatggcc cgcctggctg   1920
   accgcccaac gacccccgcc cattgacgtc aataatgacg tatgttccca tagtaacgcc   1980
   aatagggact ttccattgac gtcaatgggt ggactattta cggtaaactg cccacttggc   2040
   agtacatcaa gtgtatcata tgccaagtac gccccctatt gacgtcaatg acggtaaatg   2100
   gcccgcctgg cattatgccc agtacatgac cttatgggac tttcctactt ggcagtacat   2160
   ctacgtatta gtcatcgcta ttaccatggt cgaggtgagc cccacgttct gcttcactct   2220
   ccccatctcc cccccctccc cacccccaat tttgtattta tttatttttt aattattttg   2280
   tgcagcgatg ggggcggggg gggggggggg gcgcgcgcca ggcggggcgg ggcggggcga   2340
   ggggcggggc ggggcgaggc ggagaggtgc ggcggcagcc aatcagagcg gcgcgctccg   2400
   aaagtttcct tttatggcga ggcggcggcg gcggcggccc tataaaaagc gaagcgcgcg   2460
   gcgggcggga gtcgctgcga cgctgccttc gccccgtgcc ccgctccgcc gccgcctcgc   2520
   gccgcccgcc ccggctctga ctgaccgcgt tactcccaca ggtgagcggg cgggacggcc   2580
   cttctcctcc gggctgtaat tagcgcttgg tttaatgacg gcttgtttct tttctgtggc 2640
   tgcgtgaaag ccttgagggg ctccgggagg gccctttgtg cgggggggag cggctcgggg 2700
   ggtgcgtgcg tgtgtgtgtg cgtggggagc gccgcgtgcg gcccgcgctg cccggcggct 2760
   gtgagcgctg cgggcgcggc gcggggcttt gtgcgctccg cagtgtgcgc gaggggagcg 2820
   cggccggggg cggtgccccg cggtgcgggg ggggctgcga ggggaacaaa ggctgcgtgc 2880
   ggggtgtgtg cgtggggggg tgagcagggg gtgtgggcgc ggcggtcggg ctgtaacccc 2940
   cccctgcacc cccctccccg agttgctgag cacggcccgg cttcgggtgc ggggctccgt 3000
   acggggcgtg gcgcggggct cgccgtgccg ggcggggggt ggcggcaggt gggggtgccg 3060
   ggcggggcgg ggccgcctcg ggccggggag ggctcggggg aggggcgcgg cggcccccgg 3120
   agcgccggcg gctgtcgagg cgcggcgagc cgcagccatt gccttttatg gtaatcgtgc 3180
   gagagggcgc agggacttcc tttgtcccaa atctgtgcgg agccgaaatc tgggaggcgc 3240
   cgccgcaccc cctctagcgg gcgcggggcg aagcggtgcg gcgccggcag gaaggaaatg 3300
   ggcggggagg gccttcgtgc gtcgccgcgc cgccgtcccc ttctccctct ccagcctcgg 3360
   ggctgtccgc ggggggacgg ctgccttcgg gggggacggg gcagggcggg gttcggcttc 3420
   tggcgtgtga ccggcggctc tagagcctct gctaaccatg ttcatgcctt cttctttttc 3480
   ctacagctcc tgggcaacgt gctggttatt gtgctgtctc atcattttgg caaagaattc 3540
   ctgcagcccg ggggatccac tagtccagtg tggtggaatt gatcccttca cctaatacga 3600
   ctcactatag gctagcctcg agctgatccg ggccgggcgg gaagtcgggt cccgaggctc 3660
   cggctcggca gaccgggcgg aaagcagccg agcggccatg gagctgtgcg ggctggggct 3720
   gccccggccg cccatgctgc tggcgctgct gttggcgaca ctgctggcgg cgatgttggc 3780
   gctgctgact caggtggcgc tggtggtgca ggtggcggag gcggctcggg ccccgagcgt 3840
   ctcggccaag ccggggccgg cgctgtggcc cctgccgctc tcggtgaaga tgaccccgaa 3900
   cctgctgcat ctcgccccgg agaacttcta catcagccac agccccaatt ccacggcggg 3960
   cccctcctgc accctgctgg aggaagcgtt tcgacgatat catggctata tttttggttt 4020
   ctacaagtgg catcatgaac ctgctgaatt ccaggctaaa acccaggttc agcaacttct 4080
   tgtctcaatc acccttcagt cagagtgtga tgctttcccc aacatatctt cagatgagtc 4140
   ttatacttta cttgtgaaag aaccagtggc tgtccttaag gccaacagag tttggggagc 4200
   attacgaggt ttagagacct ttagccagtt agtttatcaa gattcttatg gaactttcac 4260
   catcaatgaa tccaccatta ttgattctcc aaggttttct cacagaggaa ttttgattga 4320
   tacatccaga cattatctgc cagttaagat tattcttaaa actctggatg ccatggcttt 4380
   taataagttt aatgttcttc actggcacat agttgatgac cagtctttcc catatcagag 4440
   catcactttt cctgagttaa gcaataaagg aagctattct ttgtctcatg tttatacacc 4500
   aaatgatgtc cgtatggtga ttgaatatgc cagattacga ggaattcgag tcctgccaga 4560
   atttgatacc cctgggcata cactatcttg ggaaaaaggt cagaaagacc tcctgactcc   4620
   atgttacagt agacaaaaca agttggactc ttttggacct ataaacccta ctctgaatac   4680
   aacatacagc ttccttacta catttttcaa agaaattagt gaggtgtttc cagatcaatt 4740
   cattcatttg ggaggagatg aagtggaatt taaatgttgg gaatcaaatc caaaaattca 4800
   agatttcatg aggcaaaaag gctttggcac agattttaag aaactagaat ctttctacat 4860
   tcaaaaggtt ttggatatta ttgcaaccat aaacaaggga tccattgtct ggcaggaggt 4920
   ttttgatgat aaagcaaagc ttgcgccggg cacaatagtt gaagtatgga aagacagcgc 4980
   atatcctgag gaactcagta gagtcacagc atctggcttc cctgtaatcc tttctgctcc 5040
   ttggtactta gatttgatta gctatggaca agattggagg aaatactata aagtggaacc 5100
   tcttgatttt ggcggtactc agaaacagaa acaacttttc attggtggag aagcttgtct 5160
   atggggagaa tatgtggatg caactaacct cactccaaga ttatggcctc gggcaagtgc 5220
   tgttggtgag agactctgga gttccaaaga tgtcagagat atggatgacg cctatgacag 5280
   actgacaagg caccgctgca ggatggtcga acgtggaata gctgcacaac ctctttatgc 5340
   tggatattgt aaccatgaga acatgtaaaa aatggagggg aaaaaggcca cagcaatctg 5400
   tactacaatc aactttattt tgaaatcatg taaaataaga tattagactt ttttgaataa 5460
   actcgagaat tcacgcgtcg agcatgcatc tagggcggcc aattccgccc ctctccctcc 5520
   ccccccccta acgttactgg ccgaagccgc ttggaataag gccggtgtgc gtttgtctat 5580
   atgtgatttt ccaccatatt gccgtctttt ggcaatgtga gggcccggaa acctggccct 5640
   gtcttcttga cgagcattcc taggggtctt tcccctctcg ccaaaggaat gcaaggtctg 5700
   ttgaatgtcg tgaaggaagc agttcctctg gaagcttctt gaagacaaac aacgtctgta 5760
   gcgacccttt gcaggcagcg gaacccccca cctggcgaca ggtgcctctg cggccaaaag 5820
   ccacgtgtat aagatacacc tgcaaaggcg gcacaacccc agtgccacgt tgtgagttgg 5880
   atagttgtgg aaagagtcaa atggctctcc tcaagcgtat tcaacaaggg gctgaaggat 5940
   gcccagaagg taccccattg tatgggatct gatctggggc ctcggtgcac atgctttaca 6000
   tgtgtttagt cgaggttaaa aaaacgtcta ggccccccga accacgggga cgtggttttc 6060
   ctttgaaaaa cacgatgata agcttgccac aacccgggat cctctatgac aagctccagg 6120
   ctttggtttt cgctgctgct ggcggcagcg ttcgcaggac gggcgacggc cctctggccc 6180
   tggcctcaga acttccaaac ctccgaccag cgctacgtcc tttacccgaa caactttcaa 6240
   ttccagtacg atgtcagctc ggccgcgcag cccggctgct cagtcctcga cgaggccttc 6300
   cagcgctatc gtgacctgct tttcggttcc gggtcttggc cccgtcctta cctcacaggg 6360
   aaacggcata cactggagaa gaatgtgttg gttgtctctg tagtcacacc tggatgtaac 6420
   cagcttccta ctttggagtc agtggagaat tataccctga ccataaatga tgaccagtgt 6480
   ttactcctct ctgagactgt ctggggagct ctccgaggtc tggagacttt tagccagctt 6540
   gtttggaaat ctgctgaggg cacattcttt atcaacaaga ctgagattga ggactttccc 6600
   cgctttcctc accggggctt gctgttggat acatctcgcc attacctgcc actctctagc 6660
   atcctggaca ctctggatgt catggcgtac aataaattga acgtgttcca ctggcatctg 6720
   gtagatgatc cttccttccc atatgagagc ttcacttttc cagagctcat gagaaagggg 6780
   tcctacaacc ctgtcaccca catctacaca gcacaggatg tgaaggaggt cattgaatac 6840
   gcacggctcc ggggtatccg tgtgcttgca gagtttgaca ctcctggcca cactttgtcc 6900
   tggggaccag gtatccctgg attactgact ccttgctact ctgggtctga gccctctggc 6960
   acctttggac cagtgaatcc cagtctcaat aatacctatg agttcatgag cacattcttc 7020
   ttagaagtca gctctgtctt cccagatttt tatcttcatc ttggaggaga tgaggttgat 7080
   ttcacctgct ggaagtccaa cccagagatc caggacttta tgaggaagaa aggcttcggt 7140
   gaggacttca agcagctgga gtccttctac atccagacgc tgctggacat cgtctcttct 7200
   tatggcaagg gctatgtggt gtggcaggag gtgtttgata ataaagtaaa gattcagcca 7260
   gacacaatca tacaggtgtg gcgagaggat attccagtga actatatgaa ggagctggaa 7320
   ctggtcacca aggccggctt ccgggccctt ctctctgccc cctggtacct gaaccgtata 7380
   tcctatggcc ctgactggaa ggatttctac gtagtggaac ccctggcatt tgaaggtacc 7440
   cctgagcaga aggctctggt gattggtgga gaggcttgta tgtggggaga atatgtggac 7500
   aacacaaacc tggtccccag gctctggccc agagcagggg ctgttgccga aaggctgtgg 7560
   agcaacaagt tgacatctga cctgacattt gcctatgaac gtttgtcaca cttccgctgt 7620
   gagttgctga ggcgaggtgt ccaggcccaa cccctcaatg taggcttctg tgagcaggag 7680
   tttgaacaga cctgaagagt cgacccgggc ggccgcttcc ctttagtgag ggttaatgaa 7740
   gggctcgagt ctagagggcc cgcggttcga aggtaagcct atccctaacc ctctcctcgg 7800
   tctcgattct acgcgtaccg gttagtaatg agtttggaat taattctgtg gaatgtgtgt 7860
   cagttagggt gtggaaagtc cccaggctcc ccaggcaggc agaagtatgc aaagcatgca 7920
   tctcaattag tcagcaacca ggtgtggaaa gtccccaggc tccccagcag gcagaagtat 7980
   gcaaagcatg catctcaatt agtcagcaac catagtcccg cccctaactc cgcccatccc 8040
   gcccctaact ccgcccagtt ccgcccattc tccgccccat ggctgactaa ttttttttat 8100
   ttatgcagag gccgaggccg cctctgcctc tgagctattc cagaagtagt gaggaggctt 8160
   ttttggaggc ctaggctttt gcaaaaagct cccgggagct tgtatatcca ttttcggatc 8220
   tgatcagcac gtgttgacaa ttaatcatcg gcatagtata tcggcatagt ataatacgac 8280
   aaggtgagga actaaaccat ggccaagcct ttgtctcaag aagaatccac cctcattgaa 8340
   agagcaacgg ctacaatcaa cagcatcccc atctctgaag actacagcgt cgccagcgca 8400
   gctctctcta gcgacggccg catcttcact ggtgtcaatg tatatcattt tactggggga 8460
   ccttgtgcag aactcgtggt gctgggcact gctgctgctg cggcagctgg caacctgact 8520
   tgtatcgtcg cgatcggaaa tgagaacagg ggcatcttga gcccctgcgg acggtgccga 8580
   caggtgcttc tcgatctgca tcctgggatc aaagccatag tgaaggacag tgatggacag 8640
   ccgacggcag ttgggattcg tgaattgctg ccctctggtt atgtgtggga gggctaagca 8700
   caattcgagc tcggtacctt taagaccaat gacttacaag gcagctgtag atcttagcca 8760
   ctttttaaaa gaaaaggggg gactggaagg gctaattcac tcccaacgaa gacaagatct 8820
   gctttttgct tgtactgggt ctctctggtt agaccagatc tgagcctggg agctctctgg 8880
   ctaactaggg aacccactgc ttaagcctca ataaagcttg ccttgagtgc ttcaagtagt 8940
   gtgtgcccgt ctgttgtgtg actctggtaa ctagagatcc ctcagaccct tttagtcagt 9000
   gtggaaaatc tctagcagta gtagttcatg tcatcttatt attcagtatt tataacttgc 9060
   aaagaaatga atatcagaga gtgagaggaa cttgtttatt gcagcttata atggttacaa 9120
   ataaagcaat agcatcacaa atttcacaaa taaagcattt ttttcactgc attctagttg 9180
   tggtttgtcc aaactcatca atgtatctta tcatgtctgg ctctagctat cccgccccta 9240
   actccgccca tcccgcccct aactccgccc agttccgccc attctccgcc ccatggctga 9300
   ctaatttttt ttatttatgc agaggccgag gccgcctcgg cctctgagct attccagaag 9360
   tagtgaggag gcttttttgg aggcctaggg acgtacccaa ttcgccctat agtgagtcgt 9420
   attacgcgcg ctcactggcc gtcgttttac aacgtcgtga ctgggaaaac cctggcgtta 9480
   cccaacttaa tcgccttgca gcacatcccc ctttcgccag ctggcgtaat agcgaagagg 9540
   cccgcaccga tcgcccttcc caacagttgc gcagcctgaa tggcgaatgg gacgcgccct 9600
   gtagcggcgc attaagcgcg gcgggtgtgg tggttacgcg cagcgtgacc gctacacttg 9660
   ccagcgccct agcgcccgct cctttcgctt tcttcccttc ctttctcgcc acgttcgccg 9720
   gctttccccg tcaagctcta aatcgggggc tccctttagg gttccgattt agtgctttac 9780
   ggcacctcga ccccaaaaaa cttgattagg gtgatggttc acgtagtggg ccatcgccct 9840
   gatagacggt ttttcgccct ttgacgttgg agtccacgtt ctttaatagt ggactcttgt 9900
   tccaaactgg aacaacactc aaccctatct cggtctattc ttttgattta taagggattt 9960
   tgccgatttc ggcctattgg ttaaaaaatg agctgattta acaaaaattt aacgcgaatt 10020
   ttaacaaaat attaacgctt acaatttagg tggcactttt cggggaaatg tgcgcggaac 10080
   ccctatttgt ttatttttct aaatacattc aaatatgtat ccgctcatga gacaataacc 10140
   ctgataaatg cttcaataat attgaaaaag gaagagtatg agtattcaac atttccgtgt   10200
   cgcccttatt cccttttttg cggcattttg ccttcctgtt tttgctcacc cagaaacgct   10260
   ggtgaaagta aaagatgctg aagatcagtt gggtgcacga gtgggttaca tcgaactgga   10320
   tctcaacagc ggtaagatcc ttgagagttt tcgccccgaa gaacgttttc caatgatgag   10380
   cacttttaaa gttctgctat gtggcgcggt attatcccgt attgacgccg ggcaagagca   10440
   actcggtcgc cgcatacact attctcagaa tgacttggtt gagtactcac cagtcacaga   10500
   aaagcatctt acggatggca tgacagtaag agaattatgc agtgctgcca taaccatgag   10560
   tgataacact gcggccaact tacttctgac aacgatcgga ggaccgaagg agctaaccgc   10620
   ttttttgcac aacatggggg atcatgtaac tcgccttgat cgttgggaac cggagctgaa   10680
   tgaagccata ccaaacgacg agcgtgacac cacgatgcct gtagcaatgg caacaacgtt   10740
   gcgcaaacta ttaactggcg aactacttac tctagcttcc cggcaacaat taatagactg   10800
   gatggaggcg gataaagttg caggaccact tctgcgctcg gcccttccgg ctggctggtt   10860
   tattgctgat aaatctggag ccggtgagcg tgggtctcgc ggtatcattg cagcactggg   10920
   gccagatggt aagccctccc gtatcgtagt tatctacacg acggggagtc aggcaactat   10980
   ggatgaacga aatagacaga tcgctgagat aggtgcctca ctgattaagc attggtaact   11040
   gtcagaccaa gtttactcat atatacttta gattgattta aaacttcatt tttaatttaa   11100
   aaggatctag gtgaagatcc tttttgataa tctcatgacc aaaatccctt aacgtgagtt   11160
   ttcgttccac tgagcgtcag accccgtaga aaagatcaaa ggatcttctt gagatccttt   11220
   ttttctgcgc gtaatctgct gcttgcaaac aaaaaaacca ccgctaccag cggtggtttg   11280
   tttgccggat caagagctac caactctttt tccgaaggta actggcttca gcagagcgca   11340
   gataccaaat actgttcttc tagtgtagcc gtagttaggc caccacttca agaactctgt   11400
   agcaccgcct acatacctcg ctctgctaat cctgttacca gtggctgctg ccagtggcga   11460
   taagtcgtgt cttaccgggt tggactcaag acgatagtta ccggataagg cgcagcggtc   11520
   gggctgaacg gggggttcgt gcacacagcc cagcttggag cgaacgacct acaccgaact   11580
   gagataccta cagcgtgagc tatgagaaag cgccacgctt cccgaaggga gaaaggcgga   11640
   caggtatccg gtaagcggca gggtcggaac aggagagcgc acgagggagc ttccaggggg   11700
   aaacgcctgg tatctttata gtcctgtcgg gtttcgccac ctctgacttg agcgtcgatt   11760
   tttgtgatgc tcgtcagggg ggcggagcct atggaaaaac gccagcaacg cggccttttt   11820
   acggttcctg gccttttgct ggccttttgc tcacatgttc tttcctgcgt tatcccctga   11880
   ttctgtggat aaccgtatta ccgcctttga gtgagctgat accgctcgcc gcagccgaac   11940
   gaccgagcgc agcgagtcag tgagcgagga agcggaagag cgcccaatac gcaaaccgcc   12000
   tctccccgcg cgttggccga ttcattaatg cagctggcac gacaggtttc ccgactggaa   12060
   agcgggcagt gagcgcaacg caattaatgt gagttagctc actcattagg caccccaggc   12120
   tttacacttt atgcttccgg ctcgtatgtt gtgtggaatt gtgagcggat aacaatttca   12180
   cacaggaaac agctatgacc atgattacgc caagcgcgca attaaccctc actaaaggga   12240
   acaaaagctg gagctgcaag ctt                                           12263
<210> 20
   <211> 11110
   <212> DNA
   <213> Artificial Sequence
<220>
   <223> Description of Artificial Sequence:/Note = Synthetic Construct
<400> 20
   aatgtagtct tatgcaatac tcttgtagtc ttgcaacatg gtaacgatga gttagcaaca      60
   tgccttacaa ggagagaaaa agcaccgtgc atgccgattg gtggaagtaa ggtggtacga     120
   tcgtgcctta ttaggaaggc aacagacggg tctgacatgg attggacgaa ccactgaatt     180
   gccgcattgc agagatattg tatttaagtg cctagctcga tacataaacg ggtctctctg     240
   gttagaccag atctgagcct gggagctctc tggctaacta gggaacccac tgcttaagcc     300
   tcaataaagc ttgccttgag tgcttcaagt agtgtgtgcc cgtctgttgt gtgactctgg     360
   taactagaga tccctcagac ccttttagtc agtgtggaaa atctctagca gtggcgcccg     420
   aacagggact tgaaagcgaa agggaaacca gaggagctct ctcgacgcag gactcggctt     480
   gctgaagcgc gcacggcaag aggcgagggg cggcgactgg tgagtacgcc aaaaattttg     540
   actagcggag gctagaagga gagagatggg tgcgagagcg tcagtattaa gcgggggaga     600
   attagatcgc gatgggaaaa aattcggtta aggccagggg gaaagaaaaa atataaatta     660
   aaacatatag tatgggcaag cagggagcta gaacgattcg cagttaatcc tggcctgtta     720
   gaaacatcag aaggctgtag acaaatactg ggacagctac aaccatccct tcagacagga     780
   tcagaagaac ttagatcatt atataataca gtagcaaccc tctattgtgt gcatcaaagg     840
   atagagataa aagacaccaa ggaagcttta gacaagatag aggaagagca aaacaaaagt     900
   aagaccaccg cacagcaagc ggccgctgat cttcagacct ggaggaggag atatgaggga     960
   caattggaga agtgaattat ataaatataa agtagtaaaa attgaaccat taggagtagc 1020
   acccaccaag gcaaagagaa gagtggtgca gagagaaaaa agagcagtgg gaataggagc 1080
   tttgttcctt gggttcttgg gagcagcagg aagcactatg ggcgcagcgt caatgacgct 1140
   gacggtacag gccagacaat tattgtctgg tatagtgcag cagcagaaca atttgctgag 1200
   ggctattgag gcgcaacagc atctgttgca actcacagtc tggggcatca agcagctcca 1260
   ggcaagaatc ctggctgtgg aaagatacct aaaggatcaa cagctcctgg ggatttgggg 1320
   ttgctctgga aaactcattt gcaccactgc tgtgccttgg aatgctagtt ggagtaataa 1380
   atctctggaa cagatttgga atcacacgac ctggatggag tgggacagag aaattaacaa 1440
   ttacacaagc ttaatacact ccttaattga agaatcgcaa aaccagcaag aaaagaatga 1500
   acaagaatta ttggaattag ataaatgggc aagtttgtgg aattggttta acataacaaa 1560
   ttggctgtgg tatataaaat tattcataat gatagtagga ggcttggtag gtttaagaat 1620
   agtttttgct gtactttcta tagtgaatag agttaggcag ggatattcac cattatcgtt 1680
   tcagacccac ctcccaaccc cgaggggacc cgacaggccc gaaggaatag aagaagaagg 1740
   tggagagaga gacagagaca gatccattcg attagtgaac ggatctcgac ggtatcgata 1800
   agcttgggag ttccgcgtta cataacttac ggtaaatggc ccgcctggct gaccgcccaa 1860
   cgacccccgc ccattgacgt caataatgac gtatgttccc atagtaacgc caatagggac 1920
   tttccattga cgtcaatggg tggagtattt acggtaaact gcccacttgg cagtacatca 1980
   agtgtatcat atgccaagta cgccccctat tgacgtcaat gacggtaaat ggcccgcctg 2040
   gcattatgcc cagtacatga ccttatggga ctttcctact tggcagtaca tctacgtatt 2100
   agtcatcgct attaccatgg tgatgcggtt ttggcagtac atcaatgggc gtggatagcg 2160
   gtttgactca cggggatttc caagtctcca ccccattgac gtcaatggga gtttgttttg 2220
   gcaccaaaat caacgggact ttccaaaatg tcgtaacaac tccgccccat tgacgcaaat 2280
   gggcggtagg cgtgtacggt gggaggtcta tataagcaga gctcgtttag tgaaccgtca 2340
   gatcgcctgg agacgccatc cacgctgttt tgacctccat agaagacacc gactctagag 2400
   gatccactag tccagtgtgg tggaattgat cccttcacct aatacgactc actataggct 2460
   agcctcgagc tgatccgggc cgggcgggaa gtcgggtccc gaggctccgg ctcggcagac 2520
   cgggcggaaa gcagccgagc ggccatggag ctgtgcgggc tggggctgcc ccggccgccc 2580
   atgctgctgg cgctgctgtt ggcgacactg ctggcggcga tgttggcgct gctgactcag 2640
   gtggcgctgg tggtgcaggt ggcggaggcg gctcgggccc cgagcgtctc ggccaagccg 2700
   gggccggcgc tgtggcccct gccgctctcg gtgaagatga ccccgaacct gctgcatctc 2760
   gccccggaga acttctacat cagccacagc cccaattcca cggcgggccc ctcctgcacc 2820
   ctgctggagg aagcgtttcg acgatatcat ggctatattt ttggtttcta caagtggcat 2880
   catgaacctg ctgaattcca ggctaaaacc caggttcagc aacttcttgt ctcaatcacc 2940
   cttcagtcag agtgtgatgc tttccccaac atatcttcag atgagtctta tactttactt 3000
   gtgaaagaac cagtggctgt ccttaaggcc aacagagttt ggggagcatt acgaggttta 3060
   gagaccttta gccagttagt ttatcaagat tcttatggaa ctttcaccat caatgaatcc 3120
   accattattg attctccaag gttttctcac agaggaattt tgattgatac atccagacat 3180
   tatctgccag ttaagattat tcttaaaact ctggatgcca tggcttttaa taagtttaat 3240
   gttcttcact ggcacatagt tgatgaccag tctttcccat atcagagcat cacttttcct 3300
   gagttaagca ataaaggaag ctattctttg tctcatgttt atacaccaaa tgatgtccgt 3360
   atggtgattg aatatgccag attacgagga attcgagtcc tgccagaatt tgatacccct 3420
   gggcatacac tatcttgggg aaaaggtcag aaagacctcc tgactccatg ttacagtaga 3480
   caaaacaagt tggactcttt tggacctata aaccctactc tgaatacaac atacagcttc 3540
   cttactacat ttttcaaaga aattagtgag gtgtttccag atcaattcat tcatttggga 3600
   ggagatgaag tggaatttaa atgttgggaa tcaaatccaa aaattcaaga tttcatgagg 3660
   caaaaaggct ttggcacaga ttttaagaaa ctagaatctt tctacattca aaaggttttg 3720
   gatattattg caaccataaa caagggatcc attgtctggc aggaggtttt tgatgataaa 3780
   gcaaagcttg cgccgggcac aatagttgaa gtatggaaag acagcgcata tcctgaggaa 3840
   ctcagtagag tcacagcatc tggcttccct gtaatccttt ctgctccttg gtacttagat 3900
   ttgattagct atggacaaga ttggaggaaa tactataaag tggaacctct tgattttggc 3960
   ggtactcaga aacagaaaca acttttcatt ggtggagaag cttgtctatg gggagaatat 4020
   gtggatgcaa ctaacctcac tccaagatta tggcctcggg caagtgctgt tggtgagaga 4080
   ctctggagtt ccaaagatgt cagagatatg gatgacgcct atgacagact gacaaggcac 4140
   cgctgcagga tggtcgaacg tggaatagct gcacaacctc tttatgctgg atattgtaac 4200
   catgagaaca tgtaaaaaat ggaggggaaa aaggccacag caatctgtac tacaatcaac 4260
   tttattttga aatcatgtaa aataagatat tagacttttt tgaataaact cgagaattca 4320
   cgcgtcgagc atgcatctag ggcggccaat tccgcccctc tccctccccc ccccctaacg 4380
   ttactggccg aagccgcttg gaataaggcc ggtgtgcgtt tgtctatatg tgattttcca 4440
   ccatattgcc gtcttttggc aatgtgaggg cccggaaacc tggccctgtc ttcttgacga 4500
   gcattcctag gggtctttcc cctctcgcca aaggaatgca aggtctgttg aatgtcgtga 4560
   aggaagcagt tcctctggaa gcttcttgaa gacaaacaac gtctgtagcg accctttgca 4620
   ggcagcggaa ccccccacct ggcgacaggt gcctctgcgg ccaaaagcca cgtgtataag 4680
   atacacctgc aaaggcggca caaccccagt gccacgttgt gagttggata gttgtggaaa 4740
   gagtcaaatg gctctcctca agcgtattca acaaggggct gaaggatgcc cagaaggtac 4800
   cccattgtat gggatctgat ctggggcctc ggtgcacatg ctttacatgt gtttagtcga 4860
   ggttaaaaaa acgtctaggc cccccgaacc acggggacgt ggttttcctt tgaaaaacac 4920
   gatgataagc ttgccacaac ccgggatcct ctatgacaag ctccaggctt tggttttcgc 4980
   tgctgctggc ggcagcgttc gcaggacggg cgacggccct ctggccctgg cctcagaact 5040
   tccaaacctc cgaccagcgc tacgtccttt acccgaacaa ctttcaattc cagtacgatg 5100
   tcagctcggc cgcgcagccc ggctgctcag tcctcgacga ggccttccag cgctatcgtg 5160
   acctgctttt cggttccggg tcttggcccc gtccttacct cacagggaaa cggcatacac 5220
   tggagaagaa tgtgttggtt gtctctgtag tcacacctgg atgtaaccag cttcctactt 5280
   tggagtcagt ggagaattat accctgacca taaatgatga ccagtgttta ctcctctctg 5340
   agactgtctg gggagctctc cgaggtctgg agacttttag ccagcttgtt tggaaatctg 5400
   ctgagggcac attctttatc aacaagactg agattgagga ctttccccgc tttcctcacc 5460
   ggggcttgct gttggataca tctcgccatt acctgccact ctctagcatc ctggacactc 5520
   tggatgtcat ggcgtacaat aaattgaacg tgttccactg gcatctggta gatgatcctt 5580
   ccttcccata tgagagcttc acttttccag agctcatgag aaaggggtcc tacaaccctg 5640
   tcacccacat ctacacagca caggatgtga aggaggtcat tgaatacgca cggctccggg 5700
   gtatccgtgt gcttgcagag tttgacactc ctggccacac tttgtcctgg ggaccaggta 5760
   tccctggatt actgactcct tgctactctg ggtctgagcc ctctggcacc tttggaccag 5820
   tgaatcccag tctcaataat acctatgagt tcatgagcac attcttctta gaagtcagct 5880
   ctgtcttccc agatttttat cttcatcttg gaggagatga ggttgatttc acctgctgga 5940
   agtccaaccc agagatccag gactttatga ggaagaaagg cttcggtgag gacttcaagc 6000
   agctggagtc cttctacatc cagacgctgc tggacatcgt ctcttcttat ggcaagggct 6060
   atgtggtgtg gcaggaggtg tttgataata aagtaaagat tcagccagac acaatcatac 6120
   aggtgtggcg agaggatatt ccagtgaact atatgaagga gctggaactg gtcaccaagg 6180
   ccggcttccg ggcccttctc tctgccccct ggtacctgaa ccgtatatcc tatggccctg 6240
   actggaagga tttctacgta gtggaacccc tggcatttga aggtacccct gagcagaagg 6300
   ctctggtgat tggtggagag gcttgtatgt ggggagaata tgtggacaac acaaacctgg 6360
   tccccaggct ctggcccaga gcaggggctg ttgccgaaag gctgtggagc aacaagttga 6420
   catctgacct gacatttgcc tatgaacgtt tgtcacactt ccgctgtgag ttgctgaggc 6480
   gaggtgtcca ggcccaaccc ctcaatgtag gcttctgtga gcaggagttt gaacagacct 6540
   gaagagtcga cccgggcggc cgcttccctt tagtgagggt taatgaaggg ctcgagtcta 6600
   gagggcccgc ggttcgaagg taagcctatc cctaaccctc tcctcggtct cgattctacg 6660
   cgtaccggtt agtaatgagt ttggaattaa ttctgtggaa tgtgtgtcag ttagggtgtg 6720
   gaaagtcccc aggctcccca ggcaggcaga agtatgcaaa gcatgcatct caattagtca 6780
   gcaaccaggt gtggaaagtc cccaggctcc ccagcaggca gaagtatgca aagcatgcat 6840
   ctcaattagt cagcaaccat agtcccgccc ctaactccgc ccatcccgcc cctaactccg 6900
   cccagttccg cccattctcc gccccatggc tgactaattt tttttattta tgcagaggcc 6960
   gaggccgcct ctgcctctga gctattccag aagtagtgag gaggcttttt tggaggccta 7020
   ggcttttgca aaaagctccc gggagcttgt atatccattt tcggatctga tcagcacgtg 7080
   ttgacaatta atcatcggca tagtatatcg gcatagtata atacgacaag gtgaggaact 7140
   aaaccatggc caagcctttg tctcaagaag aatccaccct cattgaaaga gcaacggcta 7200
   caatcaacag catccccatc tctgaagact acagcgtcgc cagcgcagct ctctctagcg 7260
   acggccgcat cttcactggt gtcaatgtat atcattttac tgggggacct tgtgcagaac 7320
   tcgtggtgct gggcactgct gctgctgcgg cagctggcaa cctgacttgt atcgtcgcga 7380
   tcggaaatga gaacaggggc atcttgagcc cctgcggacg gtgccgacag gtgcttctcg 7440
   atctgcatcc tgggatcaaa gccatagtga aggacagtga tggacagccg acggcagttg 7500
   ggattcgtga attgctgccc tctggttatg tgtgggaggg ctaagcacaa ttcgagctcg 7560
   gtacctttaa gaccaatgac ttacaaggca gctgtagatc ttagccactt tttaaaagaa 7620
   aaggggggac tggaagggct aattcactcc caacgaagac aagatctgct ttttgcttgt 7680
   actgggtctc tctggttaga ccagatctga gcctgggagc tctctggcta actagggaac 7740
   ccactgctta agcctcaata aagcttgcct tgagtgcttc aagtagtgtg tgcccgtctg 7800
   ttgtgtgact ctggtaacta gagatccctc agaccctttt agtcagtgtg gaaaatctct 7860
   agcagtagta gttcatgtca tcttattatt cagtatttat aacttgcaaa gaaatgaata 7920
   tcagagagtg agaggaactt gtttattgca gcttataatg gttacaaata aagcaatagc 7980
   atcacaaatt tcacaaataa agcatttttt tcactgcatt ctagttgtgg tttgtccaaa 8040
   ctcatcaatg tatcttatca tgtctggctc tagctatccc gcccctaact ccgcccatcc 8100
   cgcccctaac tccgcccagt tccgcccatt ctccgcccca tggctgacta atttttttta 8160
   tttatgcaga ggccgaggcc gcctcggcct ctgagctatt ccagaagtag tgaggaggct 8220
   tttttggagg cctagggacg tacccaattc gccctatagt gagtcgtatt acgcgcgctc 8280
   actggccgtc gttttacaac gtcgtgactg ggaaaaccct ggcgttaccc aacttaatcg 8340
   ccttgcagca catccccctt tcgccagctg gcgtaatagc gaagaggccc gcaccgatcg 8400
   cccttcccaa cagttgcgca gcctgaatgg cgaatgggac gcgccctgta gcggcgcatt 8460
   aagcgcggcg ggtgtggtgg ttacgcgcag cgtgaccgct acacttgcca gcgccctagc 8520
   gcccgctcct ttcgctttct tcccttcctt tctcgccacg ttcgccggct ttccccgtca     8580
   agctctaaat cgggggctcc ctttagggtt ccgatttagt gctttacggc acctcgaccc     8640
   caaaaaactt gattagggtg atggttcacg tagtgggcca tcgccctgat agacggtttt     8700
   tcgccctttg acgttggagt ccacgttctt taatagtgga ctcttgttcc aaactggaac     8760
   aacactcaac cctatctcgg tctattcttt tgatttataa gggattttgc cgatttcggc     8820
   ctattggtta aaaaatgagc tgatttaaca aaaatttaac gcgaatttta acaaaatatt     8880
   aacgcttaca atttaggtgg cacttttcgg ggaaatgtgc gcggaacccc tatttgttta     8940
   tttttctaaa tacattcaaa tatgtatccg ctcatgagac aataaccctg ataaatgctt     9000
   caataatatt gaaaaaggaa gagtatgagt attcaacatt tccgtgtcgc ccttattccc     9060
   ttttttgcgg cattttgcct tcctgttttt gctcacccag aaacgctggt gaaagtaaaa     9120
   gatgctgaag atcagttggg tgcacgagtg ggttacatcg aactggatct caacagcggt     9180
   aagatccttg agagttttcg ccccgaagaa cgttttccaa tgatgagcac ttttaaagtt     9240
   ctgctatgtg gcgcggtatt atcccgtatt gacgccgggc aagagcaact cggtcgccgc     9300
   atacactatt ctcagaatga cttggttgag tactcaccag tcacagaaaa gcatcttacg     9360
   gatggcatga cagtaagaga attatgcagt gctgccataa ccatgagtga taacactgcg     9420
   gccaacttac ttctgacaac gatcggagga ccgaaggagc taaccgcttt tttgcacaac     9480
   atgggggatc atgtaactcg ccttgatcgt tgggaaccgg agctgaatga agccatacca     9540
   aacgacgagc gtgacaccac gatgcctgta gcaatggcaa caacgttgcg caaactatta     9600
   actggcgaac tacttactct agcttcccgg caacaattaa tagactggat ggaggcggat     9660
   aaagttgcag gaccacttct gcgctcggcc cttccggctg gctggtttat tgctgataaa     9720
   tctggagccg gtgagcgtgg gtctcgcggt atcattgcag cactggggcc agatggtaag     9780
   ccctcccgta tcgtagttat ctacacgacg gggagtcagg caactatgga tgaacgaaat     9840
   agacagatcg ctgagatagg tgcctcactg attaagcatt ggtaactgtc agaccaagtt     9900
   tactcatata tactttagat tgatttaaaa cttcattttt aatttaaaag gatctaggtg     9960
   aagatccttt ttgataatct catgaccaaa atcccttaac gtgagttttc gttccactga     10020
   gcgtcagacc ccgtagaaaa gatcaaagga tcttcttgag atcctttttt tctgcgcgta     10080
   atctgctgct tgcaaacaaa aaaaccaccg ctaccagcgg tggtttgttt gccggatcaa     10140
   gagctaccaa ctctttttcc gaaggtaact ggcttcagca gagcgcagat accaaatact     10200
   gttcttctag tgtagccgta gttaggccac cacttcaaga actctgtagc accgcctaca     10260
   tacctcgctc tgctaatcct gttaccagtg gctgctgcca gtggcgataa gtcgtgtctt     10320
   accgggttgg actcaagacg atagttaccg gataaggcgc agcggtcggg ctgaacgggg     10380
   ggttcgtgca cacagcccag cttggagcga acgacctaca ccgaactgag atacctacag     10440
   cgtgagctat gagaaagcgc cacgcttccc gaagggagaa aggcggacag gtatccggta     10500
   agcggcaggg tcggaacagg agagcgcacg agggagcttc cagggggaaa cgcctggtat     10560
   ctttatagtc ctgtcgggtt tcgccacctc tgacttgagc gtcgattttt gtgatgctcg     10620
   tcaggggggc ggagcctatg gaaaaacgcc agcaacgcgg cctttttacg gttcctggcc     10680
   ttttgctggc cttttgctca catgttcttt cctgcgttat cccctgattc tgtggataac     10740
   cgtattaccg cctttgagtg agctgatacc gctcgccgca gccgaacgac cgagcgcagc     10800
   gagtcagtga gcgaggaagc ggaagagcgc ccaatacgca aaccgcctct ccccgcgcgt     10860
   tggccgattc attaatgcag ctggcacgac aggtttcccg actggaaagc gggcagtgag     10920
   cgcaacgcaa ttaatgtgag ttagctcact cattaggcac cccaggcttt acactttatg     10980
   cttccggctc gtatgttgtg tggaattgtg agcggataac aatttcacac aggaaacagc     11040
   tatgaccatg attacgccaa gcgcgcaatt aaccctcact aaagggaaca aaagctggag     11100
   ctgcaagctt                                                            11110
<210> 21
   <211> 1278
   <212> DNA
   <213> Artificial Sequence
<220>
   <223> Description of Artificial Sequence:/Note = Synthetic Construct
<400> 21
<210> 22
   <211> 1278
   <212> DNA
   <213> Artificial Sequence
<220>
   <223> Description of Artificial Sequence:/Note = Synthetic Construct
<400> 22
   tcgaggtgag ccccacgttc tgcttcactc tccccatctc ccccccctcc ccacccccaa 60
   ttttgtattt atttattttt taattatttt gtgcagcgat gggggcgggg gggggggggg 120
   cgcgcgccag gcggggcggg gcggggcgag gggcggggcg gggcgaggcg gagaggtgcg 180
   gcggcagcca atcagagcgg cgcgctccga aagtttcctt ttatggcgag gcggcggcgg 240
   cggcggccct ataaaaagcg aagcgcgcgg cgggcgggag tcgctgcgtt gccttcgccc 300
   cgtgccccgc tccgcgccgc ctcgcgccgc ccgccccggc tctgactgac cgcgttactc 360
   ccacaggtga gcgggcggga cggcccttct cctccgggct gtaattagcg cttggtttaa 420
   tgacggctcg tttcttttct gtggctgcgt gaaagcctta aagggctccg ggagggccct 480
   ttgtgcgggg gggagcggct cggggggtgc gtgcgtgtgt gtgtgcgtgg ggagcgccgc 540
   gtgcggcccg cgctgcccgg cggctgtgag cgctgcgggc gcggcgcggg gctttgtgcg 600
   ctccgcgtgt gcgcgagggg agcgcggccg ggggcggtgc cccgcggtgc gggggggctg 660
   cgaggggaac aaaggctgcg tgcggggtgt gtgcgtgggg gggtgagcag ggggtgtggg 720
   cgcggcggtc gggctgtaac ccccccctgc acccccctcc ccgagttgct gcgcacggcc 780
   cggcttcggg tgcggggctc cgtgcggggc gtggcgcggg gctcgccgtg ccgggcgggg 840
   ggtggcggca ggtgggggtg ccgggcgggg cggggccgcc tcgggccggg gagggctcgg 900
   gggaggggcg cggcggcccc ggagcgccgg cggctgtcga ggcgcggcga gccgcagcca 960
   ttgcctttta tggtaatcgt gcgagagggc gcagggactt cctttgtccc aaatctggcg 1020
   gagccgaaat ctgggaggcg ccgccgcacc ccctctagcg ggcgcgggcg aagcggtgcg 1080
   gcgccggcag gaaggaaatg ggcggggagg gccttcgtgc gtcgccgcgc cgccgtcccc 1140
   ttctccatct ccagcctcgg ggctgccgca gggggacggc tgccttcggg ggggacgggg 1200
   cagggcgggg ttcggcttct ggcgttgtac cggcggggtt tatatcttcc cttctctgtt 1260
   cctccgcagc cagccatg 1278
<210> 23
   <211> 1729
   <212> DNA
   <213> Artificial Sequence
<220>
   <223> Description of Artificial Sequence:/Note = Synthetic Construct
<400> 23
   gaattcggta ccctagttat taatagtaat caattacggg gtcattagtt catagcccat 60
   atatggagtt ccgcgttaca taacttacgg taaatggccc gcctggctga ccgcccaacg 120
   acccccgccc attgacgtca ataatgacgt atgttcccat agtaacgcca atagggactt 180
   tccattgacg tcaatgggtg gactatttac ggtaaactgc ccacttggca gtacatcaag 240
   tgtatcatat gccaagtacg ccccctattg acgtcaatga cggtaaatgg cccgcctggc 300
   attatgccca gtacatgacc ttatgggact ttcctacttg gcagtacatc tacgtattag     360
   tcatcgctat taccatggtc gaggtgagcc ccacgttctg cttcactctc cccatctccc     420
   ccccctcccc acccccaatt ttgtatttat ttatttttta attattttgt gcagcgatgg     480
   gggcgggggg gggggggggg cgcgcgccag gcggggcggg gcggggcgag gggcggggcg     540
   gggcgaggcg gagaggtgcg gcggcagcca atcagagcgg cgcgctccga aagtttcctt     600
   ttatggcgag gcggcggcgg cggcggccct ataaaaagcg aagcgcgcgg cgggcgggag     660
   tcgctgcgac gctgccttcg ccccgtgccc cgctccgccg ccgcctcgcg ccgcccgccc     720
   cggctctgac tgaccgcgtt actcccacag gtgagcgggc gggacggccc ttctcctccg     780
   ggctgtaatt agcgcttggt ttaatgacgg cttgtttctt ttctgtggct gcgtgaaagc     840
   cttgaggggc tccgggaggg ccctttgtgc gggggggagc ggctcggggg gtgcgtgcgt     900
   gtgtgtgtgc gtggggagcg ccgcgtgcgg cccgcgctgc ccggcggctg tgagcgctgc     960
   gggcgcggcg cggggctttg tgcgctccgc agtgtgcgcg aggggagcgc ggccgggggc    1020
   ggtgccccgc ggtgcggggg gggctgcgag gggaacaaag gctgcgtgcg gggtgtgtgc     1080
   gtgggggggt gagcaggggg tgtgggcgcg gcggtcgggc tgtaaccccc ccctgcaccc    1140
   ccctccccga gttgctgagc acggcccggc ttcgggtgcg gggctccgta cggggcgtgg    1200
   cgcggggctc gccgtgccgg gcggggggtg gcggcaggtg ggggtgccgg gcggggcggg      1260
   gccgcctcgg gccggggagg gctcggggga ggggcgcggc ggcccccgga gcgccggcgg    1320
   ctgtcgaggc gcggcgagcc gcagccattg ccttttatgg taatcgtgcg agagggcgca    1380
   gggacttcct ttgtcccaaa tctgtgcgga gccgaaatct gggaggcgcc gccgcacccc    1440
   ctctagcggg cgcggggcga agcggtgcgg cgccggcagg aaggaaatgg gcggggaggg    1500
   ccttcgtgcg tcgccgcgcc gccgtcccct tctccctctc cagcctcggg gctgtccgcg    1560
   gggggacggc tgccttcggg ggggacgggg cagggcgggg ttcggcttct ggcgtgtgac    1620
   cggcggctct agagcctctg ctaaccatgt tcatgccttc ttctttttcc tacagctcct    1680
   gggcaacgtg ctggttattg tgctgtctca tcattttggc aaagaattc                 1729
<210> 24
   <211> 366
   <212> DNA
   <213> Artificial Sequence
<220>
   <223> Description of Artificial Sequence:/Note = Synthetic Construct
<400> 24
   tagttattaa tagtaatcaa ttacggggtc attagttcat agcccatata tggagttccg      60
   cgttacataa cttacggtaa atggcccgcc tggctgaccg cccaacgacc cccgcccatt     120
   gacgtcaata atgacgtatg ttcccatagt aacgccaata gggactttcc attgacgtca     180
   atgggtggac tatttacggt aaactgccca cttggcagta catcaagtgt atcatatgcc     240
   aagtacgccc cctattgacg tcaatgacgg taaatggccc gcctggcatt atgcccagta     300
   catgacctta tgggactttc ctacttggca gtacatctac gtattagtca tcgctattac     360
   catggt                                                                366
<210> 25
   <211> 1295
   <212> DNA
   <213> Artificial Sequence
<220>
   <223> Description of Artificial Sequence:/Note = Synthetic Construct
<400> 25
   ccaattttgt atttatttat tttttaatta ttttgtgcag cgatgggggc gggggggggg      60
   ggggggcgcg cgccaggcgg ggcggggcgg ggcgaggggc ggggcggggc gaggcggaga     120
   ggtgcggcgg cagccaatca gagcggcgcg ctccgaaagt ttccttttat ggcgaggcgg     180
   cggcggcggc ggccctataa aaagcgaagc gcgcggcggg cgggagtcgc tgcgacgctg     240
   ccttcgcccc gtgccccgct ccgccgccgc ctcgcgccgc ccgccccggc tctgactgac     300
   cgcgttactc ccacaggtga gcgggcggga cggcccttct cctccgggct gtaattagcg     360
   cttggtttaa tgacggcttg tttcttttct gtggctgcgt gaaagccttg aggggctccg     420
   ggagggccct ttgtgcgggg gggagcggct cggggggtgc gtgcgtgtgt gtgtgcgtgg     480
   ggagcgccgc gtgcggcccg cgctgcccgg cggctgtgag cgctgcgggc gcggcgcggg     540
   gctttgtgcg ctccgcagtg tgcgcgaggg gagcgcggcc gggggcggtg ccccgcggtg     600
   cggggggggc tgcgagggga acaaaggctg cgtgcggggt gtgtgcgtgg gggggtgagc     660
   agggggtgtg ggcgcggcgg tcgggctgta acccccccct gcacccccct ccccgagttg     720
   ctgagcacgg cccggcttcg ggtgcggggc tccgtacggg gcgtggcgcg gggctcgccg     780
   tgccgggcgg ggggtggcgg caggtggggg tgccgggcgg ggcggggccg cctcgggccg     840
   gggagggctc gggggagggg cgcggcggcc cccggagcgc cggcggctgt cgaggcgcgg     900
   cgagccgcag ccattgcctt ttatggtaat cgtgcgagag ggcgcaggga cttcctttgt     960
   cccaaatctg tgcggagccg aaatctggga ggcgccgccg caccccctct agcgggcgcg    1020
   gggcgaagcg gtgcggcgcc ggcaggaagg aaatgggcgg ggagggcctt cgtgcgtcgc    1080
   cgcgccgccg tccccttctc cctctccagc ctcggggctg tccgcggggg gacggctgcc    1140
   ttcggggggg acggggcagg gcggggttcg gcttctggcg tgtgaccggc ggctctagag    1200
   cctctgctaa ccatgttcat gccttcttct ttttcctaca gctcctgggc aacgtgctgg    1260
   ttattgtgct gtctcatcat tttggcaaag aattc                                1295
<210> 26
   <211> 1278
   <212> DNA
   <213> Artificial Sequence
<220>
   <223> Description of Artificial Sequence:/Note = Synthetic Construct
<400> 26
   tcgaggtgag ccccacgttc tgcttcactc tccccatctc ccccccctcc ccacccccaa      60
   ttttgtattt atttattttt taattatttt gtgcagcgat gggggcgggg gggggggggg     120
   cgcgcgccag gcggggcggg gcggggcgag gggcggggcg gggcgaggcg gagaggtgcg     180
   gcggcagcca atcagagcgg cgcgctccga aagtttcctt ttatggcgag gcggcggcgg     240
   cggcggccct ataaaaagcg aagcgcgcgg cgggcgggag tcgctgcgtt gccttcgccc     300
   cgtgccccgc tccgcgccgc ctcgcgccgc ccgccccggc tctgactgac cgcgttactc     360
   ccacaggtga gcgggcggga cggcccttct cctccgggct gtaattagcg cttggtttaa     420
   tgacggctcg tttcttttct gtggctgcgt gaaagcctta aagggctccg ggagggccct     480
   ttgtgcgggg gggagcggct cggggggtgc gtgcgtgtgt gtgtgcgtgg ggagcgccgc     540
   gtgcggcccg cgctgcccgg cggctgtgag cgctgcgggc gcggcgcggg gctttgtgcg     600
   ctccgcgtgt gcgcgagggg agcgcggccg ggggcggtgc cccgcggtgc gggggggctg     660
   cgaggggaac aaaggctgcg tgcggggtgt gtgcgtgggg gggtgagcag ggggtgtggg     720
   cgcggcggtc gggctgtaac ccccccctgc acccccctcc ccgagttgct gcgcacggcc     780
   cggcttcggg tgcggggctc cgtgcggggc gtggcgcggg gctcgccgtg ccgggcgggg     840
   ggtggcggca ggtgggggtg ccgggcgggg cggggccgcc tcgggccggg gagggctcgg     900
   gggaggggcg cggcggcccc ggagcgccgg cggctgtcga ggcgcggcga gccgcagcca     960
   ttgcctttta tggtaatcgt gcgagagggc gcagggactt cctttgtccc aaatctggcg    1020
   gagccgaaat ctgggaggcg ccgccgcacc ccctctagcg ggcgcgggcg aagcggtgcg    1080
   gcgccggcag gaaggaaatg ggcggggagg gccttcgtgc gtcgccgcgc cgccgtcccc    1140
   ttctccatct ccagcctcgg ggctgccgca gggggacggc tgccttcggg ggggacgggg    1200
   cagggcgggg ttcggcttct ggcgttgtac cggcggggtt tatatcttcc cttctctgtt    1260
   cctccgcagc cagccatg                                                  1278
<210> 27
   <211> 229
   <212> DNA
   <213> Artificial Sequence
<220>
   <223> Description of Artificial Sequence:/Note = Synthetic Construct
<400> 27
   gtattagtca tcgctattac catggtgatg cggttttggc agtacatcaa tgggcgtgga      60
   tagcggtttg actcacgggg atttccaagt ctccacccca ttgacgtcaa tgggagtttg     120
   ttttggcacc aaaatcaacg ggactttcca aaatgtcgta acaactccgc cccattgacg     180
   caaatgggcg gtaggcgtgt acggtgggag gtctatataa gcagagctc                 229
<210> 28
   <211> 281
   <212> DNA
   <213> Artificial Sequence
<220>
   <223> Description of Artificial Sequence:/Note = Synthetic Construct
<400> 28
   tggcattatg cccagtacat gaccttatgg gactttccta cttggcagta catctacgta      60
   ttagtcatcg ctattaccat ggtgatgcgg ttttggcagt acatcaatgg gcgtggatag     120
   cggtttgact cacggggatt tccaagtctc caccccattg acgtcaatgg gagtttgttt     180
   tggcaccaaa atcaacggga ctttccaaaa tgtcgtaaca actccgcccc attgacgcaa     240
   atgggcggta ggcgtgtacg gtgggaggtc tatataagca g                         281
<210> 29
   <211> 282
   <212> DNA
   <213> Artificial Sequence
<220>
   <223> Description of Artificial Sequence:/Note = Synthetic Construct
<400> 29
   attatgccca gtacatgacc ttatgggact ttcctacttg gcagtacatc tacgtattag      60
   tcatcgctat taccatggtg atgcggtttt ggcagtacat caatgggcgt ggatagcggt     120
   ttgactcacg gggatttcca agtctccacc ccattgacgt caatgggagt ttgttttggc     180
   accaaaatca acgggacttt ccaaaatgtc gtaacaactc cgccccattg acgcaaatgg     240
   gcggtaggcg tgtacggtgg gaggtctata taagcagagc tc                        282
<210> 30
   <211> 512
   <212> DNA
   <213> Artificial Sequence
<220>
   <223> Description of Artificial Sequence:/Note = Synthetic Construct
<400> 30
   ttgcgttaca taacttacgg taaatggccc gcctggctga ccgcccaacg acccccgccc      60
   attgacgtca ataatgacgt atgttcccat agtaacgcca atagggactt tccattgacg     120
   tcaatgggtg gactatttac ggtaaactgc ccacttggca gtacatcaag tgtatcatat     180
   gccaagtacg ccccctattg acgtcaatga cggtaaatgg cccgcctggc attatgccca     240
   gtacatgacc ttatgggact ttcctacttg gcagtacatc tacgtattag tcatcgctat     300
   taccatggtg atgcggtttt ggcagtacat caatgggcgt ggatagcggt ttgactcacg     360
   gggatttcca agtctccacc ccattgacgt caatgggagt ttgttttggc accaaaatca     420
   acgggacttt ccaaaatgtc gtaacaactc cgccccattg acgcaaatgg gcggtaggcg     480
   tgtacggtgg gaggtctata taagcagagc tc                                   512
<210> 31
   <211> 308
   <212> DNA
   <213> Artificial Sequence
<220>
   <223> Description of Artificial Sequence:/Note = Synthetic Construct
<400> 31
   tcggcgaagc ctcgcgcggc cggccaggac gaggagcgcc actaggttga acatccgcac      60
   gagccgccgg gccaggtctc ggacgggctc tcgagactcg atctcgtgca tgtcggcggt     120
   ccgcggtgag gttatagacc atctgctagg cgggtccggg gagacaggca cattactggc     180
   ctcggcgccc agcctaggcg tgtctagagc tcgaccgcgc gtccggagcg ccattcgacc     240
   ggcgggtagc gagaagaacg ccggagaccg caggttataa caacgtcatg cataaattaa     300
   gaatgggc                                                              308
<210> 32
   <211> 1848
   <212> DNA
   <213> Artificial Sequence
<220>
   <223> Description of Artificial Sequence:/Note = Synthetic Construct
<400> 32
   ctgcagtgaa taataaaatg tgtgtttgtc cgaaatacgc gtttgagatt tctgtcccga      60
   ctaaattcat gtcgcgcgat agtggtgttt atcgccgata gagatggcga tattggaaaa     120
   atcgatattt gaaaatatgg catattgaaa atgtcgccga tgtgagtttc tgtgtaactg     180
   atatcgccat ttttccaaaa gttgattttt gggcatacgc gatatctggc gatacgctta     240
   tatcgtttac gggggatggc gatagacgcc tttggtgact tgggcgattc tgtgtgtcgc     300
   aaatatcgca gtttcgatat aggtgacaga cgatatgagg ctatatcgcc gatagaggcg     360
   acatcaagct ggcacatggc caatgcatat cgatctatac attgaatcaa tattggccat     420
   tagccatatt attcattggt tatatagcat aaatcaatat tggctattgg ccattgcata     480
   cgttgtatcc atatcataat atgtacattt atattggctc atgtccaaca ttaccgccat     540
   gttgacattg attattgact agttattaat agtaatcaat tacggggtca ttagttcata     600
   gcccatatat ggagttccgc gttacataac ttacggtaaa tggcccgcct ggctgaccgc     660
   ccaacgaccc ccgcccattg acgtcaataa tgacgtatgt tcccatagta acgccaatag     720
   ggactttcca ttgacgtcaa tgggtggagt atttacggta aactgcccac ttggcagtac     780
   atcaagtgta tcatatgcca agtacgcccc ctattgacgt caatgacggt aaatggcccg     840
   cctggcatta tgcccagtac atgaccttat gggactttcc tacttggcag tacatctacg     900
   tattagtcat cgctattacc atggtgatgc ggttttggca gtacatcaat gggcgtggat     960
   agcggtttga ctcacgggga tttccaagtc tccaccccat tgacgtcaat gggagtttgt    1020
   tttggcacca aaatcaacgg gactttccaa aatgtcgtaa caactccgcc ccattgacgc    1080
   aaatgggcgg taggcgtgta cggtgggagg tctatataag cagagctcgt ttagtgaacc    1140
   gtcagatcgc ctggagacgc catccacgct gttttgacct ccatagaaga caccgggacc    1200
   gatccagcct ccgcggccgg gaacggtgca ttggaacgcg gattccccgt gccaagagtg    1260
   acgtaagtac cgcctataga gtctataggc ccaccccctt ggcttcttat gcatgctata    1320
   ctgtttttgg cttggggtct atacaccccc gcttcctcat gttataggtg atggtatagc    1380
   ttagcctata ggtgtgggtt attgaccatt attgaccact cccctattgg tgacgatact    1440
   ttccattact aatccataac atggctcttt gcacaactct ctttattggc tatatgccaa    1500
   tacactgtcc ttcagagact gacacggact ctgtattttt acaggatggg gtctcattta    1560
   ttatttacaa attcacatat acaacaccac cgtccccagt gcccgcagtt tttattaaac    1620
   ataacgtggg atctccagcg aatctcgggt acgtgttccg gacatggggc tcttctccgg    1680
   tagcggcgga gcttctacat ccagccctgc tcccatcctc ccactcatgg tcctcggcag    1740
   ctccttgctc ctaacagtgg aggccagact taggcacagc acgatgccca ccaccaccag    1800
   tgtgcccaca aggccgtggc ggtagggtat gtgtctgaaa atgagctc                 1848
<210> 33
   <211> 1176
   <212> DNA
   <213> Artificial Sequence
<220>
   <223> Description of Artificial Sequence:/Note = Synthetic Construct
<400> 33
   cccgggccca gcaccccaag gcggccaacg ccaaaactct ccctcctcct cttcctcaat      60
   ctcgctctcg ctcttttttt ttttcgcaaa aggaggggag agggggtaaa aaaatgctgc     120
   actgtgcggc gaagccggtg agtgagcggc gcggggccaa tcagcgtgcg ccgttccgaa     180
   agttgccttt tatggctcga gcggccgcgg cggcgcccta taaaacccag cggcgcgacg     240
   cgccaccacc gccgagaccg cgtccgcccc gcgagcacag agcctcgcct ttgccgatcc     300
   gccgcccgtc cacacccgcc gccaggtaag cccggccagc cgaccggggc atgcggccgc     360
   ggccccttcg cccgtgcaga gccgccgtct gggccgcagc ggggggcgca tgggggggga      420
   accggaccgc cgtggggggc gcgggagaag cccctgggcc tccggagatg ggggacaccc     480
   cacgccagtt cggaggcgcg aggccgcgct cgggaggcgc gctccggggg tgccgctctc     540
   ggggcggggg caaccggcgg ggtctttgtc tgagccgggc tcttgccaat ggggatcgca     600
   gggtgggcgc ggcgtagccc ccgccaggcc cggtgggggc tggggcgcca ttgccggtgc     660
   gcgctggtcc tttgggcgct aactgcgtgc gcgctgggaa ttggcgctaa ttgcgcgtgc     720
   gcgctgggac tcaaggcgct aattgcgcgt gcgttctggg gcccggggtg ccgcggcctg     780
   ggctggggcg aaggcgggct cggccggaag gggtggggtc gccgcggctc ccgggcgctt      840
   gcgcgcactt cctgcccgag ccgctggccg cccgagggtg tggccgctgc gtgcgcgcgc     900
   gccgacccgg cgctgtttga accgggcgga ggcggggctg gcgcccggtt gggagggggt     960
   tggggcctgg cttcctgccg cgcgccgcgg ggacgcctcc gaccagtgtt tgccttttat    1020
   ggtaataacg cggccggccc ggcttccttt gtccccaatc tgggcgcgcg ccggcgcccc    1080
   ctggcggcct aaggactcgg cgcgccggaa gtggccaggg cgggggcgac ttcggctcac    1140
   agcgcgcccg gctattctcg cagctcacca tggatg                              1176
<210> 34
   <211> 49
   <212> DNA
   <213> Artificial Sequence
<220>
   <223> Description of Artificial Sequence:/Note = Synthetic Construct
<400> 34
   cttctggcgt gtgaccggcg gggtttatat cttcccttcc caagcttgg 49
<210> 35
   <211> 66
   <212> DNA
   <213> Artificial Sequence
<220>
   <223> Description of Artificial Sequence:/Note = Synthetic Construct
<400> 35
   cttctggcgt gtgaccggcg gggtttatat cttcccttct ctgttcctcc gcagccccaa     60
   gcttgg                                                                66
<210> 36
   <211> 68
   <212> DNA
   <213> Artificial Sequence
<220>
   <223> Description of Artificial Sequence:/Note = Synthetic Construct
<400> 36
   cttctggcgt gtgaccggcg gggtttatat cttcccttct ctgttcctcc gcagccagcc     60
   aagcttgg                                                              68
<210> 37
   <211> 69
   <212> DNA
   <213> Artificial Sequence
<220>
   <223> Description of Artificial Sequence:/Note = Synthetic Construct
<400> 37
   cttctggcgt gtgaccggcg gggtttatat cttcccttct ctgttcctcc gcagccagcc      60
   atggatgat                                                              69
<210> 38
   <211> 1278
   <212> DNA
   <213> Artificial Sequence
<220>
   <223> Description of Artificial Sequence:/Note = Synthetic Construct
<400> 38
   tcgaggtgag ccccacgttc tgcttcactc tccccatctc ccccccctcc ccacccccaa      60
   ttttgtattt atttattttt taattatttt gtgcagcgat gggggcgggg gggggggggg     120
   cgcgcgccag gcggggcggg gcggggcgag gggcggggcg gggcgaggcg gagaggtgcg     180
   gcggcagcca atcagagcgg cgcgctccga aagtttcctt ttatggcgag gcggcggcgg     240
   cggcggccct ataaaaagcg aagcgcgcgg cgggcgggag tcgctgcgtt gccttcgccc     300
   cgtgccccgc tccgcgccgc ctcgcgccgc ccgccccggc tctgactgac cgcgttactc     360
   ccacaggtga gcgggcggga cggcccttct cctccgggct gtaattagcg cttggtttaa     420
   tgacggctcg tttcttttct gtggctgcgt gaaagcctta aagggctccg ggagggccct     480
   ttgtgcgggg gggagcggct cggggggtgc gtgcgtgtgt gtgtgcgtgg ggagcgccgc     540
   gtgcggcccg cgctgcccgg cggctgtgag cgctgcgggc gcggcgcggg gctttgtgcg     600
   ctccgcgtgt gcgcgagggg agcgcggccg ggggcggtgc cccgcggtgc gggggggctg     660
   cgaggggaac aaaggctgcg tgcggggtgt gtgcgtgggg gggtgagcag ggggtgtggg     720
   cgcggcggtc gggctgtaac ccccccctgc acccccctcc ccgagttgct gcgcacggcc     780
   cggcttcggg tgcggggctc cgtgcggggc gtggcgcggg gctcgccgtg ccgggcgggg     840
   ggtggcggca ggtgggggtg ccgggcgggg cggggccgcc tcgggccggg gagggctcgg     900
   gggaggggcg cggcggcccc ggagcgccgg cggctgtcga ggcgcggcga gccgcagcca    960
   ttgcctttta tggtaatcgt gcgagagggc gcagggactt cctttgtccc aaatctggcg    1020
   gagccgaaat ctgggaggcg ccgccgcacc ccctctagcg ggcgcgggcg aagcggtgcg    1080
   gcgccggcag gaaggaaatg ggcggggagg gccttcgtgc gtcgccgcgc cgccgtcccc    1140
   ttctccatct ccagcctcgg ggctgccgca gggggacggc tgccttcggg ggggacgggg    1200
   cagggcgggg ttcggcttct ggcgttgtac cggcgggctt tatatcttcc cttctctgtt    1260
   cctccgcagc cagccatg                                                  1278
<210> 39
   <211> 1176
   <212> DNA
   <213> Artificial Sequence
<220>
   <223> Description of Artificial Sequence:/Note = Synthetic Construct
<400> 39
   cccgggccca gcaccccaag gcggccaacg ccaaaactct ccctcctcct cttcctcaat      60
   ctcgctctcg ctcttttttt ttttcgcaaa aggaggggag agggggtaaa aaaatgctgc     120
   actgtgcggc gaagccggtg agtgagcggc gcggggccaa tcagcgtgcg ccgttccgaa     180
   agttgccttt tatggctcga gcggccgcgg cggcgcccta taaaacccag cggcgcgacg     240
   cgccaccacc gccgagaccg cgtccgcccc gcgagcacag agcctcgcct ttgccgatcc     300
   gccgcccgtc cacacccgcc gccaggtaag cccggccagc cgaccggggc atgcggccgc     360
   ggccccttcg cccgtgcaga gccgccgtct gggccgcagc ggggggcgca tgggggggga       420
   accggaccgc cgtggggggc gcgggagaag cccctgggcc tccggagatg ggggacaccc      480
   cacgccagtt cggaggcgcg aggccgcgct cgggaggcgc gctccggggg tgccgctctc     540
   ggggcggggg caaccggcgg ggtctttgtc tgagccgggc tcttgccaat ggggatcgca     600
   gggtgggcgc ggcgtagccc ccgccaggcc cggtgggggc tggggcgcca ttgccggtgc      660
   gcgctggtcc tttgggcgct aactgcgtgc gcgctgggaa ttggcgctaa ttgcgcgtgc     720
   gcgctgggac tcaaggcgct aattgcgcgt gcgttctggg gcccggggtg ccgcggcctg     780
   ggctggggcg aaggcgggct cggccggaag gggtggggtc gccgcggctc ccgggcgctt      840
   gcgcgcactt cctgcccgag ccgctggccg cccgagggtg tggccgctgc gtgcgcgcgc     900
   gccgacccgg cgctgtttga accgggcgga ggcggggctg gcgcccggtt gggagggggt     960
   tggggcctgg cttcctgccg cgcgccgcgg ggacgcctcc gaccagtgtt tgccttttat    1020
   ggtaataacg cggccggccc ggcttccttt gtccccaatc tgggcgcgcg ccggcgcccc    1080
   ctggcggcct aaggactcgg cgcgccggaa gtggccaggg cgggggcgac ttcggctcac    1140
   agcgcgcccg gctattctcg cagctcacca tggatg                              1176
<210> 40
   <211> 1345
   <212> DNA
   <213> Artificial Sequence
<220>
   <223> Description of Artificial Sequence:/Note = Synthetic Construct
<400> 40
   tcgaggtgag ccccacgttc tgcttcactc tccccatctc ccccccctcc ccacccccaa       60
   ttttgtattt atttattttt taattatttt gtgcagcgat ggggcggggg gggggggggg       120
   cgcgcgccag gcggggcggg gcggggcgag gggcggggcg gggcgaggcg gagaggtgcg     180
   gcggcagcca atcagagcgg cgcgctccga aagtttcctt ttatggcgag gcggcggcgg     240
   cggcggccct ataaaaagcg aagcgcgcgg cgggcgggag tcgctgcgtt gccttcgccc     300
   cgtgccccgc tccgcgccgc ctcgcgccgc ccgccccggc tctgactgac cgcgttactc     360
   ccacaggtga gcgggcggga cggcccttct cctccgggct gtaattagcg cttggtttaa     420
   tgacggctcg tttcttttct gtggctgcgt gaaagcctta aagggctccg ggagggccct     480
   ttgtgcgggg gggagcggct cggggggtgc gtgcgtgtgt gtgtgcgtgg ggagcgccgc     540
   gtgcggcccg cgctgcccgg cggctgtgag cgctgcgggc gcggcgcggg gctttgtgcg     600
   ctccgcgtgt gcgcgagggg agcgcggccg gggcggtgcc cccgcggtgc gggggggctg      660
   cgaggggaac aaaggctgcg tgcggggtgt gtgcgtgggg gggtgagcag ggggtgtggg     720
   cgcggcggtc gggctgtaac ccccccctgc acccccctcc ccgagttgct gagcacggcc      780
   cggcttcggg tgcggggctc cgtgcggggc gtggcgcggg gctcgccgtg ccgggcgggg     840
   ggtggcggca ggtgggggtg ccgggcgggg cggggccgcc tcgggccggg gagggctcgg      900
   gggaggggcg cggcggcccc ggagcgccgg cggctgtcga ggcgcggcga gccgcagcca     960
   ttgcctttta tggtaatcgt gcgagagggc gcagggactt cctttgtccc aaatctggcg    1020
   gagccgaaat ctgggaggcg ccgccgcacc ccctctagcg ggcgcgggcg aagcggtgcg    1080
   gcgccggcag gaaggaaatg ggcggggagg gccttcgtgc gtcgccgcgc cgccgtcccc    1140
   ttctccatct ccagcctcgg ggctgccgca gggggacggc tgccttcggg ggggacgggg    1200
   cagggcgggg ttcggcttct ggcgtgtgac cggcggctct agagcctctg ctaaccatgt    1260
   tcatgccttc ttctttttcc tacagctcct gggcaacgtg ctggttgttg tgctgtctca     1320
   tcattttggc aaagaattca agctt                                          1345
<210> 41
   <211> 684
   <212> DNA
   <213> Artificial Sequence
<220>
   <223> Description of Artificial Sequence:/Note = Synthetic Construct
<400> 41
   tcaatattgg ccattagcca tattattcat tggttatata gcataaatca atattggcta      60
   ttggccattg catacgttgt atctatatca taatatgtac atttatattg gctcatgtcc     120
   aatatgaccg ccatgttggc attgattatt gactagttat taatagtaat caattacggg     180
   gtcattagtt catagcccat atatggagtt ccgcgttaca taacttacgg taaatggccc     240
   gcctggctga ccgcccaacg acccccgccc attgacgtca ataatgacgt atgttcccat     300
   agtaacgcca atagggactt tccattgacg tcaatgggtg gagtatttac ggtaaactgc     360
   ccacttggca gtacatcaag tgtatcatat gccaagtccg ccccctattg acgtcaatga     420
   cggtaaatgg cccgcctggc attatgccca gtacatgacc ttacgggact ttcctacttg     480
   gcagtacatc tacgtattag tcatcgctat taccatggtg atgcggtttt ggcagtacac     540
   caatgggcgt ggatagcggt ttgactcacg gggatttcca agtctccacc ccattgacgt     600
   caatgggagt ttgttttggc accaaaatca acgggacttt ccaaaatgtc gtaataaccc     660
   cgccccgttg acgcaaatgg gcgg                                            684